(19) Europäisches Patentamt
European Patent Office
Office européen des brevets

(11) **EP 4 498 376 A1**

(12) **EUROPEAN PATENT APPLICATION**
published in accordance with Art. 153(4) EPC

(43) Date of publication:
**29.01.2025 Bulletin 2025/05**

(21) Application number: **23774758.9**

(22) Date of filing: **16.03.2023**

(51) International Patent Classification (IPC):
**G16H 10/00** (2018.01)   **G16H 50/30** (2018.01)

(52) Cooperative Patent Classification (CPC):
**G16H 10/00; G16H 50/30**

(86) International application number:
**PCT/JP2023/010383**

(87) International publication number:
**WO 2023/182162 (28.09.2023 Gazette 2023/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.03.2022   JP 2022046894
23.03.2022   JP 2022046895**

(71) Applicant: **Otsuka Pharmaceutical Co., Ltd.
Tokyo 101-8535 (JP)**

(72) Inventors:
• **NAKAGAWA, Yasushi**
**Osaka-shi, Osaka 540-0021 (JP)**
• **DEGUCHI, Naoyuki**
**Osaka-shi, Osaka 540-0021 (JP)**

• **HAMAMOTO, Keisuke**
**Osaka-shi, Osaka 540-0021 (JP)**
• **TOBA, Masamichi**
**Osaka-shi, Osaka 540-0021 (JP)**
• **IWASHITA, Soh**
**Osaka-shi, Osaka 540-0021 (JP)**
• **TSUBOUCHI, Mina**
**Osaka-shi, Osaka 540-0021 (JP)**
• **KWAK, Kyungtak**
**Osaka-shi, Osaka 540-0021 (JP)**
• **YOSHIE, Miho**
**Osaka-shi, Osaka 540-0021 (JP)**

(74) Representative: **Hoffmann Eitle
Patent- und Rechtsanwälte PartmbB
Arabellastraße 30
81925 München (DE)**

(54) **COMPUTER PROGRAM, AND INFORMATION PROCESSING DEVICE AND METHOD**

(57)   Provided is a computer program, an information processing device, and a method that are used to estimate a health state of a target user based on a history of behaviors executed by the target user, and have at least partially improved performance.

According to one embodiment, a computer program can cause, by being executed at least one processor, the at least one processor to function to: acquire target behavior data for identifying a history of behavior executed by a target user; and output, from an estimation model generated by executing supervised learning, target sleep state data for identifying a sleep state of the target user, the target sleep state data including at least one of target property data for identifying a sleep property of the target user, target rhythm data for identifying a sleep rhythm of the target user, target time data for identifying a sleep time of the target user, and target category data for identifying a sleep category to which the target user belongs, by inputting the target behavior data to the estimation model.

Processed by Luminess, 75001 PARIS (FR)

**EP 4 498 376 A1**

**(Cont. next page)**

# FIG. 3

Start

1000

Information processing device acquires teacher data

1002

Information processing device generates estimation model by using teacher data

1004

Information processing device acquires target behavior data for identifying history of behavior executed by target user

1006

Information processing device acquires target sleep state data for identifying sleep state of target user by using target behavior data and estimation model

1008

Information processing device determines target product/target service corresponding to target sleep state data

1010

Information processing device provides proposal data for identifying target product/target service

End

FIG. 3

**Description**

Technical Field

The technology disclosed in the present application relates to a computer program, an information processing device, and a method for inputting, to a learning model, data for identifying a history of behaviors executed by a user (target user) to be estimated, thereby outputting, from the learning model, data for identifying a health state of the target user.

Background Art

Japanese Patent No. 6916367 (Patent Literature 1) discloses a method for constructing an estimation model for estimating a health state of a target user based on data describing a purchase history of the target user. It should be noted that this patent literature is incorporated herein by reference in its entirety.

Citation List

Patent Literature

Patent Literature 1: Japanese Patent No. 6916367

Summary of Invention

Technical Problem

Recently, as a method for estimating a health state of a target user based on a history of a behavior executed by the target user, there has been a demand for providing a method having further improved performance.

Therefore, the technology disclosed in the present application provides a computer program, an information processing device, and a method that are used to estimate the health state of the target user based on the history of behaviors executed by the target user, and have at least partially improved performance.

Solution to Problem

According to one aspect, a computer program can "cause, by being executed by at least one processor, the at least one processor to function to: acquire target behavior data for identifying a history of behavior executed by a target user; and output, from an estimation model generated by executing supervised learning, target sleep state data for identifying a sleep state of the target user, the target sleep state data including at least one of target property data for identifying a sleep property of the target user, target rhythm data for identifying a sleep rhythm of the target user, target time data for identifying a sleep time of the target user, and target category data for identifying a sleep category to which the target user belongs, by inputting the target behavior data to the estimation model".

According to one aspect, an information processing device may include "at least one processor, and the at least one processor is configured to: acquire target behavior data for identifying a history of behavior executed by a target user; and output, from an estimation model generated by executing supervised learning, target sleep state data for identifying a sleep state of the target user, the target sleep state data including at least one of target property data for identifying a sleep property of the target user, target rhythm data for identifying a sleep rhythm of the target user, target time data for identifying a sleep time of the target user, and target category data for identifying a sleep category to which the target user belongs, by inputting the target behavior data to the estimation model".

According to one aspect, a method is executed by at least one processor that executes a computer readable instruction, and the method can include: by the at least one processor executing the instruction, a step of acquiring target behavior data for identifying a history of behavior executed by a target user; and a step of outputting, from an estimation model generated by executing supervised learning, target sleep state data for identifying a sleep state of the target user, the target sleep state data including at least one of target property data for identifying a sleep property of the target user, target rhythm data for identifying a sleep rhythm of the target user, target time data for identifying a sleep time of the target user, and target category data for identifying a sleep category to which the target user belongs, by inputting the target behavior data to the estimation model".

According to another aspect, a method is "executed by at least one processor that executes a computer readable instruction and the method can include: by the at least one processor executing the instruction, an acquisition step of acquiring a plurality of sets of teacher data, each set of teacher data including sample behavior data for identifying a history of behaviors executed by one sample user corresponding to the set and sample sleep state data for identifying a sleep state of one sample user corresponding to the set; a generation step of generating an estimation model configured to output target sleep state data for identifying a sleep state of the target user, the target sleep state data including at least one of target property data for identifying a sleep property of the target user, target rhythm data for identifying a sleep rhythm of the target user, target time data for identifying a sleep time of the target user, and target category data for identifying a sleep category to which the target user belongs, by inputting target behavior data for identifying a history of behavior executed by the target user when inputting the plurality of sets of teacher data into a learning model and learning the plurality of sets of teacher data".

According to another aspect, a computer program can "cause, by being executed by at least one processor, the at least one processor to function to: acquire target behavior data for identifying a history of behavior executed by a target user; and output, from an estimation model generated by executing supervised learning, target menopause state data for identifying a menopause symptom state of the target user, the target menopause state data including at least one of target comprehensive state data for identifying a comprehensive state of a menopause symptom of the target user, target first state data for identifying a first symptom state of the target user, and target second state data for identifying a second symptom state of the target user, by inputting the target behavior data to the estimation model".

According to another aspect, an information processing device may "include at least one processor, and the at least one processor acquires target behavior data for identifying a history of behavior executed by a target user; and outputs, from an estimation model generated by executing supervised learning, target menopause state data for identifying a menopause symptom state of the target user, the target menopause state data including at least one of target comprehensive state data for identifying a comprehensive state of a menopause symptom of the target user, target first state data for identifying a first symptom state of the target user, and target second state data for identifying a second symptom state of the target user, by inputting the target behavior data to the estimation model".

According to another aspect, a method is "executed by at least one processor that executes a computer readable instruction, and the method can include: by the at least one processor executing the instruction, a step of acquiring target behavior data for identifying a history of behavior executed by a target user; and a step of outputting, from an estimation model generated by executing supervised learning, target menopause state data for identifying a menopause symptom state of the target user, the target menopause state data including at least one of target comprehensive state data for identifying a comprehensive state of a menopause symptom of the target user, target first state data for identifying a first symptom state of the target user, and target second state data for identifying a second symptom state of the target user, by inputting the target behavior data to the estimation model".

According to another aspect, a method is executed by at least one processor that executes a computer readable instruction, and the method can include: by the at least one processor executing the instruction, an acquisition step of acquiring a plurality of sets of teacher data, each set of teacher data including sample behavior data for identifying a history of behaviors executed by one sample user corresponding to the set and sample menopause state data for identifying a menopause symptom state of one sample user corresponding to the set; a generation step of generating an estimation model configured to output target menopause state data for identifying a menopause symptom state of the target user, the target menopause state data including at least one of target comprehensive state data for identifying a comprehensive state of a menopause symptom of the target user, target first state data for identifying a first symptom state of the target user, and target second state data for identifying a second symptom state of the target user by inputting target behavior data for identifying a history of behavior executed by the target user when inputting the plurality of sets of teacher data into a learning model and learning the plurality of sets of teacher data".

A computer program according to another aspect "causes an information processing device to execute processing of acquiring first purchase data indicating a purchase history of a target customer, prediction processing of predicting degrees of a plurality of types of health symptoms from the first purchase data by using a health prediction model related to the plurality of types of health symptoms of a first type to an N-th type (N is an integer of 2 or more), and processing of providing health information indicating the predicted degrees of the plurality of types of health symptoms to the target customer, in which the health prediction model related to a k-th type ($1 \leq k \leq N$) of health symptoms can calculate a numerical value indicating the degrees of the k-th type of health symptoms from the first purchase data related to a product category".

Further, the computer program according to another aspect causes "an information processing device to further execute processing of determining a product category to which at least one product belongs from the first purchase data, and the prediction processing can predict the degrees of the plurality of types of health symptoms from the first purchase data related to the product category by using health prediction models related to the plurality of types of health symptoms of a first type to an N-th type (N is an integer of 2 or more)".

Further, in the computer program according to another aspect, "the health prediction model related to the k-th type ($1 \leq k \leq N$) of health symptom can be constructed by machine learning with second purchase data indicating a purchase history of each of a plurality of test subjects as an explanatory variable and health data indicating degrees of health symptoms collected from each of the plurality of test subjects as an objective variable".

Further, in the computer program according to another aspect, "the machine learning can be machine learning by logistic regression".

Further, in the computer program according to another aspect, "the logistic regression uses an L1 normalization method and/or an L2 normalization method".

Further, in the computer program according to another aspect, the k-th type ($1 \leq k \leq N$) of health symptom is classified into a plurality of classes according to the degree of the health symptom, each of the plurality of classes is associated in advance with a numerical range according to the degree of the health symptom, and in the prediction processing, a class corresponding to the numerical value indicating the degree of the health symptom of the target customer is determined based on the numerical range to which the numerical value belongs, thereby predicting the degree of the health symptom of the target customer".

Further, in the computer program according to another aspect, "the plurality of classes can be three or more".

Further, in the computer program according to another aspect, "the program can cause the information processing device to execute proposal processing of referring to a table in which each of the plurality of classes is associated with a service and/or a product suitable for a health symptom of each class to make a proposal related to the service and/or the product suitable for the health symptom of the determined class together with the health information".

Further, in the computer program according to another aspect, "in the proposal processing, an insurance product related to the health information can be proposed by referring to a table in which each of the plurality of classes is associated with a service suitable for a health symptom of each class".

Further, in the computer program according to another aspect, "a medical institution that performs preventive medical care and/or treatment related to the health information can be introduced to the target customer by referring to a table in which each of the plurality of classes is associated with a service suitable for a health symptom of each class in the proposal processing".

Further, in the computer program according to another aspect, "the first purchase data is a purchase history of foods and/or daily necessities purchased in a certain period, and degrees of the k-th type ($1 \leq k \leq N$) of health symptoms can be represented by any one or more of a simplified menopause index (SMI), the Menopause Rating Scale, The Kupperman index, a Greene Climacteric Scale, a PSST (The premenstrual symptoms screening tool), a MRS (Menopause rating Scale), a WHQ (The Women's Health Questionnaire), a VAS (Visual analogue scale), a HFRDI (Hot Flash Related Daily Interference Scale), a HFCS (Hot Flash Composite Score), and a MENQOL (Menopause-Specific Quality of life), and a menopause symptoms assessment table for Japanese women".

Further, in the computer program according to another aspect, "the health symptom of the target customer is one or more selected from a menopause symptom and a premenstrual syndrome (PMS, PMDD) condition, and the health prediction model related to the plurality of types of health symptom can include one or more of a menopause prediction model and a premenstrual syndrome (PMS, PMDD) prediction model".

Further, in the computer program according to another aspect, "the degrees the k-th type ($1 \leq k \leq N$) of health symptoms can be represented by any one or more of the deterioration of the sleep state, the disturbance of the sleep rhythm, and the improper sleep time".

Further, in the computer program according to another aspect, "the health prediction model related to the plurality of types of health symptoms can include a sleep state prediction model".

Further, in the computer program according to another aspect, "the degrees of the k-th type ($1 \leq k \leq N$) of health symptoms can be represented by any one or more of physical inactivity, obesity, and a change in body weight in a certain period of time".

Further, in the computer program according to another aspect, "the machine learning can be performed by including, as explanatory variables, one or more pieces of information on the presence or absence of smoking and the sitting posture of each of the plurality of test subjects".

Further, in the computer program according to another aspect, "the health prediction model related to the plurality of types of health symptoms can include a lifestyle disease prediction model related to an exercise state".

Further, in the computer program according to another aspect, "the degrees of the k-th type ($1 \leq k \leq N$) of health symptoms can be represented by any one or more of serum Na, BUN/creatinine ratio, urine osmotic pressure, urine color, and urine specific gravity".

Further, in the computer program according to another aspect, "the machine learning can be performed by including, as explanatory variables, one or more pieces of information on serum Na, BUN/creatinine ratio, urine osmotic pressure, urine specific gravity, amount of water consumed, amount of alcohol consumed, physical activity amount, and urine color of each of the plurality of test subjects".

Further, in the computer program according to another aspect, "the health prediction model related to the plurality of types of health symptoms can include a hydration status prediction model related to a hydration status".

Further, in the computer program according to another aspect, "the degrees of the k-th type ($1 \leq k \leq N$) of health symptoms can be represented by any one or more of susceptibility to cold, frequency of causing cold, SIgA concentration, an allergic symptom, an oral environment/state of immunity, an exercise state, a stress state, a severe sleep symptom, a moderate sleep symptom, a mild sleep symptom, and a cold-like symptom".

Further, in the computer program according to another aspect, "the health prediction model related to the plurality of types of health symptoms can include an immune state prediction model related to an immune state".

Further, in the computer program according to another aspect, "the degrees of the k-th type ($1 \leq k \leq N$) of health symptoms are represented by any one or more of the presence or absence of subjective symptoms associated with nutritional deterioration, Diversity Score (DVS) of Food Intake, Simplified nutritional appetite questionnaire (SNAQ), and BMI (Body Mass Index)".

Further, in the computer program according to another aspect, "the health prediction model related to the plurality of types of health symptoms can include a nutritional state prediction model related to a nutritional state".

The information processing device according to another aspect "includes an input unit that acquires first purchase data indicating a purchase history of a target customer, a prediction unit that predicts degrees of a plurality of types of health symptoms from the first purchase data by using a health prediction model related to the plurality of types of health symptoms of a first type to an N-th type (N is an integer of 2 or more), and an output unit that provides health information indicating the predicted degrees of the plurality of types of health symptoms to the target customer, in which the health prediction model related to a k-th type ($1 \leq k \leq N$) of health symptoms can calculate a numerical value indicating the degrees of the k-th type of health symptoms from the first purchase data related to the product category".

An information processing method according to another aspect is "a method in which an information processing device performs processing of acquiring first purchase data indicating a purchase history of a target customer, processing of predicting degrees of a plurality of types of health symptoms from the first purchase data by using a health prediction model related to the plurality of types of health symptoms of a first type to an N-th type (N is an integer of 2 or more), and processing of providing health information indicating the predicted degrees of the plurality of types of health symptoms to the target customer, and in the information processing device, the health prediction model related to a k-th type ($1 \leq k \leq N$) of health symptoms can calculate a numerical value indicating the degrees of the k-th type of health symptoms from the first purchase data related to the product category".

Brief Description of Drawings

Fig. 1 is a block diagram illustrating an example of a configuration of a communication system according to an embodiment.

Fig. 2 is a block diagram illustrating an example of a hardware configuration of a server device 10 illustrated in Fig. 1.

Fig. 3 is a flowchart illustrating an example of an operation executed by a communication system 1 illustrated in Fig. 1.

Fig. 4 is a diagram illustrating an example of the Athens Insomnia Scale.

Fig. 5A is a diagram illustrating an example of a Pittsburgh Sleep Quality Index.

Fig. 5B is a diagram illustrating an example of the Pittsburgh Sleep Quality Index.

Fig. 5C is a diagram illustrating an example of the Pittsburgh Sleep Quality Index.

Fig. 6A is a diagram illustrating an example of a three-dimensional sleep scale.

Fig. 6B is a diagram illustrating an example of the three-dimensional sleep scale.

Fig. 7 is a diagram illustrating an example of an insomnia severity index.

Fig. 8A is a diagram illustrating an example of a Munich Chronotype Questionnaire.

Fig. 8B is a diagram illustrating an example of the Munich Chronotype Questionnaire.

Fig. 9 is a diagram illustrating an example of sleep category data used in the communication system 1 illustrated in Fig. 1.

Fig. 10 is a diagram illustrating another example of sleep category data used in the communication system 1 illustrated in Fig. 1.

Fig. 11 is a diagram conceptually illustrating an example of a search table used in the communication system 1 illustrated in Fig. 1.

Fig. 12 is a flowchart illustrating another example of an operation executed by the communication system 1 illustrated in Fig. 1.

Fig. 13 is a diagram illustrating an example of a simplified menopause index (SMI).

Fig. 14 is a diagram conceptually illustrating another example of a search table used in the communication system 1 illustrated in Fig. 1.

Fig. 15 is a diagram schematically illustrating a flow of data for performing health prediction of a customer using a health prediction system according to the embodiment.

Fig. 16 is a diagram illustrating a specific example of a functional configuration of an integrated health prediction program 100 according to the embodiment.

Fig. 17 is a diagram illustrating a specific example of purchase data according to the embodiment.

Fig. 18 is a diagram illustrating a specific example of the purchase data according to the embodiment.

Fig. 19 is a diagram illustrating a specific example of a table for realizing a proposal to a customer according to an output value of a prediction model.

Fig. 20 is a diagram illustrating a specific example of a table for realizing a proposal to a customer according to an output value of a prediction model.

Fig. 21 is a diagram illustrating a flow of processing of an integrated health prediction program according to the embodiment.

Fig. 22 is a diagram illustrating a specific example of a hardware configuration of an information processing device in which the integrated health prediction program according to the embodiment is executed.

Fig. 23 is a diagram illustrating a specific example of a functional configuration of a learning system including a model learning program according to the embodiment.

Fig. 24 is a diagram illustrating a specific example of a product category.

Fig. 25 is a diagram illustrating a specific example of a relationship between a menopause symptom found from a purchased item by a test subject in a H layer related to the menopause symptom and a health problem.

Fig. 26 is a diagram illustrating a specific example of a product category for female having menopause symptoms belonging to the H layer.

Fig. 27 is a diagram illustrating a flow of processing of the model learning program according to the embodiment.

Fig. 28 is a diagram illustrating a specific example of a hardware configuration of the information processing device in which the model learning program according to the embodiment is executed.

Description of Embodiments

Hereinafter, various embodiments of the present invention will be described with reference to the accompanying drawings. Note that components common in the drawings are denoted by the same reference numerals. In addition, it should be noted that the components illustrated in a certain drawing may be omitted in another drawing for convenience of description. Furthermore, it should be noted that the accompanying drawings are not necessarily to scale.

The various systems, methods, and devices described herein are not to be construed as being limited by any method. In practice, the present disclosure is directed to any novel features and aspects of each of the various embodiments disclosed, combinations of the various embodiments with one another, and combinations of portions of the various embodiments with one another. The various systems, methods, and devices described herein are not limited to particular aspects, features, or combinations of such particular aspects and features, nor do the articles and methods described herein require that there be one or more particular effects or problems be solved. Furthermore, various features or aspects of the various embodiments described herein, or portions of such features or aspects, may be used in combination with one another.

Although the operations of some of the various methods disclosed herein have been described along a particular order for convenience, it should be understood that describing such an approach includes reordering the above operations unless a particular order is required by a particular sentence below. For example, the plurality of operations described in sequence are, in some cases, rearranged or performed simultaneously. Furthermore, for purposes of simplicity, the accompanying drawings do not depict various ways in which the various items and methods described herein may be used in conjunction with other items and methods.

The operational theory, scientific principle or other theoretical description presented herein in connection with the device or method of the present disclosure is provided for better understanding and is not intended to limit the scope of the technology. The devices and methods in the appended claims are not limited to the device and method that operate according to the methods described by such operational theory.

Any of the various methods disclosed herein may be implemented using a plurality of computer-executable instructions stored on one or more computer-readable media, and further executed in a computer. The one or more media may be non-transitory computer-readable storage media, such as, for example, at least one optical media disk, a plurality of volatile memory components, or a plurality of nonvolatile memory components. Here, the plurality of volatile memory components includes, for example, a DRAM or an SRAM. The plurality of nonvolatile memory components include, for example, a hard drive and a solid state drive (SSD). Further, the computer includes any computer available on the market, including, for example, a smartphone and other mobile devices having hardware to perform calculations.

Any of such computer-executable instructions for implementing the technology disclosed herein may be stored on one or more computer-readable media (for example, a non-transitory computer-readable storage medium), along with any data generated and used during implementation of the various embodiments disclosed herein. Such computer-executable instructions may, for example, be a part of a separate software application, or may be a part of a software application that is accessed or downloaded via a web browser or other software application (such as a remote computing application). Such software may be executed, for example, on a single local computer (as a process executed on any suitable computer, for example, available on the market) or in a network environment (for example, the Internet, a wide area network, a local area network, a client-server network (such as a cloud computing network), or other such networks) using one or more network computers.

For clarity, only certain selected various aspects of the various software-based implementations are described. Other details well known in the art are omitted. For example, the technology disclosed herein is not limited to a particular computer language or program. For example, the technology disclosed herein may be performed by software written in C, C++, Java (registered trademark), or any other suitable programming language. Similarly, the technology disclosed herein is not limited to a particular computer or a particular type of hardware. The specific details of suitable computers and hardware are well known and need not be described in detail herein.

Furthermore, any of the various embodiments based on such software (including, for example, a plurality of computer-executable instructions for causing a computer to perform any of the various methods disclosed herein) may be uploaded, downloaded, or accessed in a remote manner by a suitable communication means. Such suitable communication means include, for example, the Internet, the World Wide Web, an intranet, a software application, a cable (including a fiber optic cable), magnetic communication, electromagnetic communication (including RF communication, microwave communication, and infrared communication), electronic communication, or other such communication means.

I. Chapter 1

1. Overview

In the technology disclosed in the present application, in brief, at least one information processing device acquires target behavior data for identifying a history of behavior executed by a user (target user) to be estimated, and inputs the target behavior data to an estimation model, so that data (health state data) for identifying a health state of the target user can be output from the estimation model.

Furthermore, at least one information processing device can determine at least one target product and/or at least one target service corresponding to the health state data of the target user among a plurality of products and/or a plurality of services. Furthermore, the at least one information processing device can also output information for identifying the at least one target product and/or the at least one target service determined in this manner.

2. Configuration of communication system

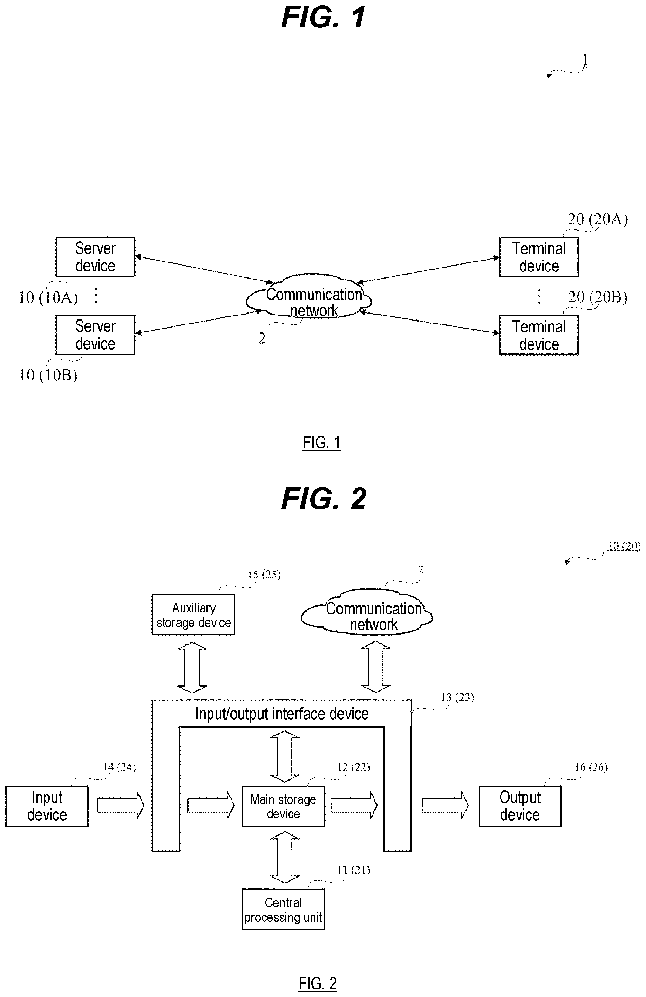

Such technology can be implemented using a communication system as illustrated in Fig. 1. Fig. 1 is a block diagram illustrating an example of a configuration of the communication system according to an embodiment.

As illustrated in Fig. 1, a communication system 1 according to an embodiment can include, for example, at least one server device (information processing device) 10 connectable to a communication network 2 and at least one terminal device (information processing device) 20 connectable to the communication network **2.** Although Fig. 1 illustrates two server devices 10A and 10B as the at least one server device 10, any number of server devices 10 can be used. Similarly, although Fig. 1 illustrates two terminal devices 20A and 20B as the at least one terminal device 20, any number of terminal devices 20 can be used.

Each server device 10 can be connected to at least one other server device 10 and/or at least one terminal device 20 via the communication network **2.** Furthermore, each terminal device 20 can be connected to at least one other terminal device 20 and/or at least one server device 10 via the communication network 2.

Each server device 10 may be an optional information processing device. Such an information processing device can include, for example, a personal computer, a workstation, a supercomputer, a mainframe, and the like without being limited thereto. Each terminal device 20 may be an optional information processing device. Such an information processing device can include, for example, a mobile phone, a smartphone, a tablet, a personal computer, a workstation, a mobile information terminal, and the like without being limited thereto.

In one example, at least one server device 10 can be an information processing device managed by an operating company that operates a service ("estimation service") that estimates a health state of an optional user and/or a service ("proposal service") that proposes at least one target product or the like corresponding to the health state of the optional user. Hereinafter, for convenience, the term "estimation service or the like" may be used as a term indicating the estimation service and/or the proposal service.

In one example, at least one server device 10 and/or at least one terminal device 20 may be an information processing device managed by a company user and/or an individual user who receives provision of an estimation service or the like from the operating company.

The operation of acquiring the target behavior data described above can be executed by at least one server device 10 and/or at least one terminal device 20.

The above-described estimation model can be generated by at least one server device 10 and/or at least one terminal device 20. The estimation model generated in this manner can be held by at least one server device 10 and/or at least one terminal device 20. The estimation model held in this manner can be used by at least one server device 10 and/or at least one terminal device 20.

The above-described operation of outputting the health state data from the estimation model can be executed by at least one server device 10 and/or at least one terminal device 20.

The above-described operation of determining at least one target product and/or at least one target service can be executed by at least one server device 10 and/or at least one terminal device 20. The above-described operation of outputting the data for identifying the at least one target product and/or the at least one target service can be executed by the at least one server device 10 and/or the at least one terminal device 20.

Note that the communication network 2 can include, but is not limited to, a mobile phone network, a wireless network, a fixed-line network, the Internet, an intranet, a local area network (LAN), a wide area network (WAN), and/or an Ethernet (registered trademark) network. The wireless network can include, for example, but is not limited to, RF connectivity via Bluetooth (trademark), WiFi (such as IEEE 802.11a/b/n), WiMax, cellular, satellite, laser, infrared, and the like.

3. Hardware configuration of each information processing device

Next, an example of a hardware configuration of each information processing device (server device 10 and terminal device 20) will be described.

(1) Hardware configuration of server device 10

Fig. 2 is a block diagram illustrating an example of a hardware configuration of the server device 10 illustrated in Fig. 1 (Note that, in Fig. 2, reference numerals in parentheses are described in association with the terminal device 20 as described later).

As illustrated in Fig. 2, the server device 10 can include a central processing unit 11, a main storage device 12, an input/output interface device 13, an input device 14, an auxiliary storage device 15, and an output device 16. These devices are connected by a data bus and/or a control bus.

The central processing unit 11 is referred to as "CPU", and can perform an operation on an instruction and data stored in the main storage device 12 and store a result of the operation in the main storage device 12. Furthermore, the central processing unit 11 can control the input device 14, the auxiliary storage device 15, the output device 16, and the like via the input/output interface device 13. The server device 10 can include one or more such central processing units 11.

The main storage device 12 is referred to as a "memory", and can store an instruction and data received from the input device 14, the auxiliary storage device 15, the communication network 2, and the like (the terminal device 20 and the like) via the input/output interface device 13, and a calculation result of the central processing unit 11. The main storage device 12 can include, but is not limited to, a computer-readable medium such as a volatile memory (for example, register, cache, random access memory (RAM)), a non-volatile memory (for example, read-only memory (ROM), EEPROM, and flash memory), and a storage (for example, a hard disk drive (HDD), a solid state drive (SSD), a magnetic tape, or an optical medium). As will be readily appreciated, the term "computer-readable recording medium" can include media for data storage, such as memory and storage, rather than transmission media, such as modulated data signals or transitory signals.

The auxiliary storage device 15 is a storage device having a larger capacity than the main storage device 12. The auxiliary storage device 15 stores instructions and data (computer program) constituting a specific application or the like, and is controlled by the central processing unit 11, so that the instructions and data (computer program) can be transmitted to the main storage device 12 via the input/output interface device 13. The auxiliary storage device 15 can include, but is not limited to, a magnetic disk device and/or an optical disk device.

The specific application described herein can include, but is not limited to, an operation system, various software applications (including a web browser), a dedicated application executed to provide an estimation service and the like, a dedicated application executed to receive the provision of an estimation service and the like, and the like.

The input device 14 is a device that captures data from the outside, and can include, but is not limited to, a keyboard, a touch panel, a button, a mouse, and/or a sensor (microphone and camera).

The output device 16 can include, but is not limited to, a display device, a touch panel, a printer device, and/or the like.

In such a hardware configuration, the central processing unit 11 can sequentially load the instructions and data (computer program) constituting the specific application stored in the auxiliary storage device 15 into the main storage device 12 and operate the loaded instructions and data. As a result, the central processing unit 11 can control the output device 16 via the input/output interface device 13, or can transmit and receive various types of information (data) to and from other devices (for example, other server devices 10 and/or terminal devices 20) via the input/output interface device 13 and the communication network 2.

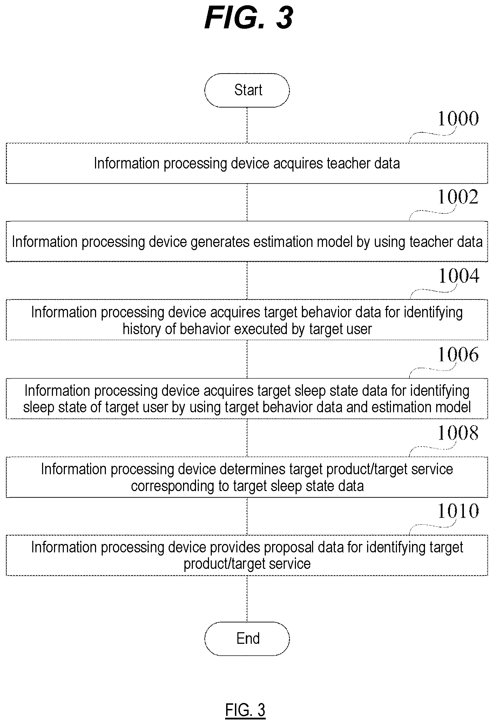

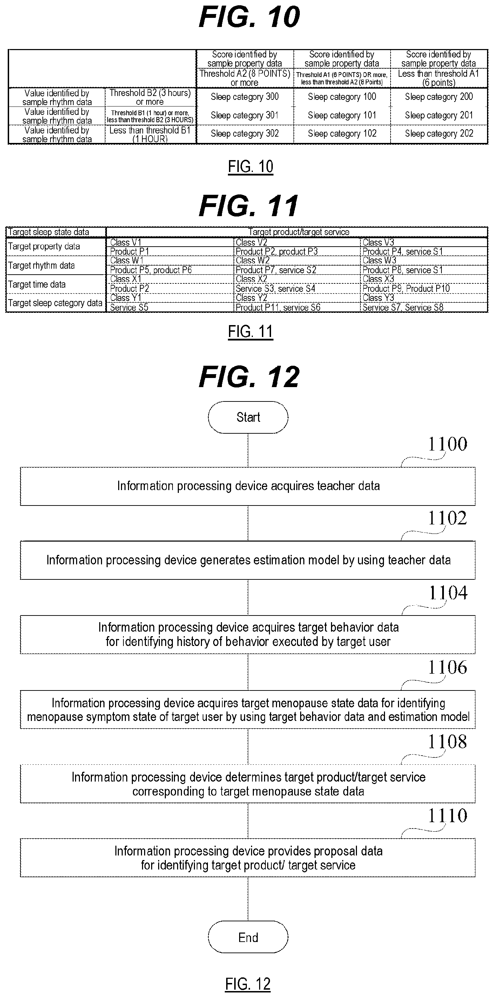

In this manner, the server device 10 can execute operations and the like related to providing the estimation service and the like and/or receiving the provision of the estimation service and the like (various operations and the like described later with reference to Figs. 3 and 12 and the like) by executing the installed specific application. In addition to or instead of this, the server device 10 can execute an operation or the like related to providing the estimation service or the like and/or receiving the provision of the estimation service or the like (various operations or the like described later with reference to Figs. 3 and 12 or the like) by executing the browser and accessing the other server device 10.

Note that the server device 10 can include, instead of the central processing unit 11 or in addition to the central processing unit 11, one or more microprocessors and/or a graphics processing unit (GPU).

(2) Hardware configuration of terminal device 20

As illustrated in parentheses in Fig. 2, the terminal device 20 can have a hardware configuration substantially similar to that of the server device 10. As illustrated in Fig 2, the terminal device 20 can include a central processing unit 21, a main storage device 22, an input/output interface device 23, an input device 24, an auxiliary storage device 25, and an output device 26. These devices are connected by a data bus and/or a control bus. Each of these devices is as described in relation to the server device 10.

The specific application stored in the auxiliary storage device 25 and executed by the central processing unit 21 can include, but is not limited to, an operation system, various software applications (including a web browser), a dedicated application executed to receive the provision of an estimation service and the like, and the like. The specific application can also include a dedicated application executed to provide an estimation service or the like.

In such a hardware configuration, the central processing unit 21 can sequentially load the instructions and data (computer program) constituting the specific application stored in the auxiliary storage device 25 into the main storage device 22 and operate the loaded instructions and data. As a result, the central processing unit 21 can control the output device 26 via the input/output interface device 23, or can transmit and receive various types of information (data) to and from other devices (for example, server devices 10 and/or other terminal devices 20) via the input/output interface device 23 and the communication network 2.

In this manner, the terminal device 20 can execute operations and the like related to receiving the provision of the estimation service and the like and/or providing the estimation service and the like (various operations and the like described later with reference to Figs. 3 and 12 and the like) by executing the installed specific application. In addition to or instead of this, the server device 10 can execute an operation or the like related to providing the estimation service or the like and/or receiving the provision of the estimation service or the like (various operations or the like described later with reference to Figs. 3 and 12 or the like) by executing the browser and accessing the other server device 10.

Note that the terminal device 20 can include, instead of the central processing unit 21 or in addition to the central processing unit 21, one or more microprocessors and/or a graphics processing unit (GPU).

4. Operation executed by communication system 2 to provide sleep state data on sleep state

Next, a specific example of the operation executed by the communication system 2 described above in order to provide the sleep state data regarding the sleep state will be further described with reference to Fig. 3. Fig. 3 is a flowchart illustrating an example of an operation executed by the communication system 1 illustrated in Fig. 1.

(1) Step 1000

First, in step (hereinafter, referred to as "ST") 1000, the server device 10A, which is an information processing device, for example, managed by a company that operates an estimation service or the like, can acquire and store a plurality of sets of teacher data used to generate the estimation model.

Each set of teacher data constituting the plurality of sets of teacher data can include data ("sample behavior data") for identifying a history of behavior executed by one sample user corresponding to the set and data ("sample sleep state data") for identifying a sleep state of one sample user corresponding to the set. For example, the first set of teacher data can include sample behavior data for identifying a history of behaviors executed by one sample user (Mr. A) corresponding to the first set and sample sleep state data for identifying a sleep state of Mr. A, and the second set of teacher data can include sample behavior data for identifying a history of behaviors executed by one sample user (Mr. B) corresponding to the second set and sample sleep state data for identifying a sleep state of Mr. B.

(1A) Sample behavior data

Each sample behavior data may be data for identifying at least one (one or plurality of) behavior of a plurality of predetermined behaviors. Here, each of the plurality of predetermined behaviors may be a behavior that can at least partially positively or negatively affect the sleep state of the human who has executed the behavior. Such a plurality of predetermined behaviors can include, for example, those exemplified below without being limited thereto.

(Example of behavior that can at least partially positively affect human sleep state)

- Aerobic exercise was performed for 1 hour or more at a pace of 2 days or more per week.
- Waking up at a constant time every day.
- Caffeine-containing beverages were kept below 2 servings per day.
- Working hours per day were 8 hours or less.
- More than 10 coffees were purchased (this behavior can be identified, for example, by data including a product category to which coffee belongs and its purchase quantity).

(Example of behavior that can at least partially adversely affect human sleep state)

- Aerobic exercise was performed for 1 hour or more at a pace of 1 day or less per week.
- Waking up at different times each day.
- Caffeine-containing beverages were taken more than 2 servings per day.
- Working hours per day were 13 hours or more.
- Five or more bottles of milk were purchased (this behavior can be identified, for example, by data including a product category to which milk belongs and its purchase quantity).

Each sample behavior data can be data for identifying each of at least one behavior among the plurality of predetermined behaviors exemplified in this manner.

Furthermore, in one example, since the product purchased by the user and/or the service used by the user can tend to have a positive influence or a negative influence on the sleep state of the user, the plurality of predetermined behaviors can include the product purchased by the sample user (or the category to which the product belongs) and the service used by the sample user (or the category to which the service belongs). This type of behavior can be generated using, for example, point of sales (POS) data held by a retail store (drug stores, supermarkets, convenience stores, or the like) or the like.

In one example, unique identification data (alphabetic characters, numbers, combinations thereof, or the like) is assigned to each of the plurality of predetermined behaviors, and each sample behavior data can include such identification data. For example, when POS data is used, each of the plurality of sample behavior data can include, but is not limited to, JICFS classification (classification code), a product category, a purchase amount, a purchase quantity, a purchase count, a sex, an age, and/or a purchase date corresponding to a purchased product.

For example, the server device 10A, which is an information processing device and is managed by a company that operates an estimation service or the like, can acquire such sample behavior data by, for example, at least one of the following methods.

- The information processing device receives the POS data from at least one other server device 10 (for example, another server device 10 that stores POS data installed in a drug store or the like, or another server device 10 that can access the POS data) via the communication network 2.
- The information processing device receives, from at least one terminal device 20 and/or at least one server device 10 via the communication network 2, a mail or a web page describing an answer to a predetermined questionnaire (questionnaire regarding behavior executed by sample users).
- The information processing device receives content of answers from at least one server device 10 that has collected answers from a plurality of users to the predetermined questionnaire via the communication network 2.
- The information processing device receives content of answers from a plurality of users to the predetermined questionnaire via a recording medium (USB memory, DVD-ROM, or the like) that has collected the answers.

(1B) Sample sleep state data

The sample sleep state data included in each set of teacher data can include, but is not limited to, at least one of the data exemplified below.

- Data ("sample property data") for identifying a sleep property of one sample user corresponding to the set
- Data ("sample rhythm data") for identifying the sleep rhythm of one sample user corresponding to the set
- Data ("sample time data") for identifying a sleep time of one sample user corresponding to the set
- Data ("sample sleep category data") identifying a sleep category to which (the sleep of) one sample user corresponding to the set belongs

First of all, the sample property data may be data for identifying whether the quality of sleep of the sample user is good (for example, how good) or bad (for example, how bad). In one example, the sample property data may be data for identifying a score that has comprehensively evaluated the quality of sleep of the sample user. In this case, the sleep quality of the sample user can be, for example, data for identifying a score calculated by any of the methods exemplified below.

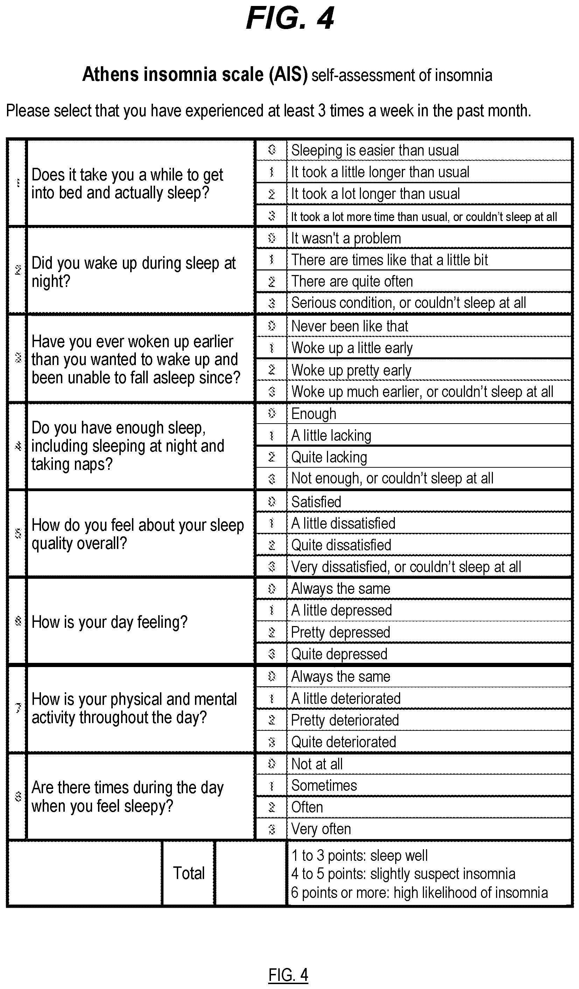

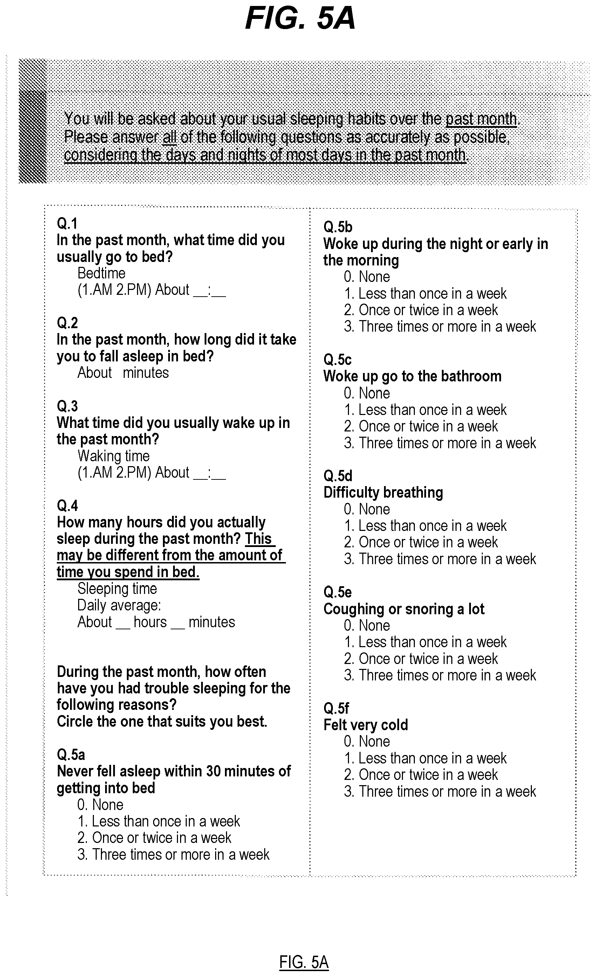

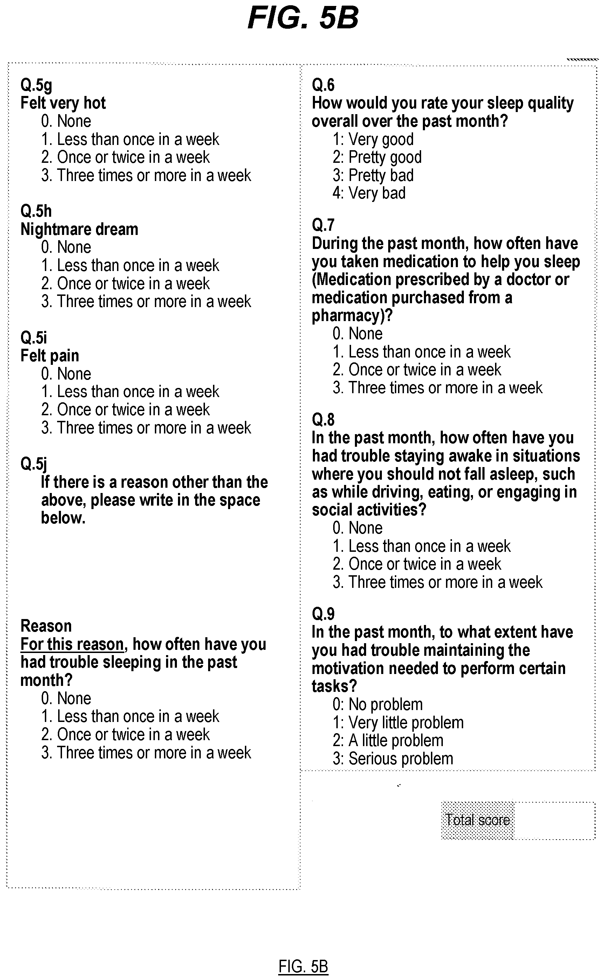

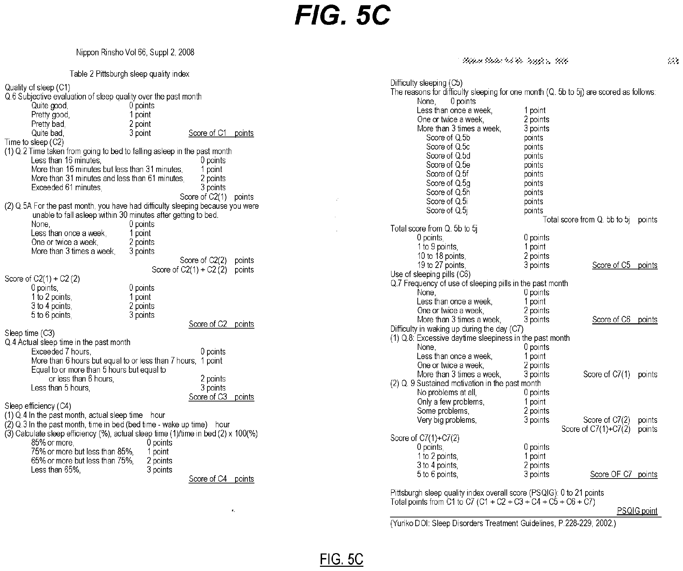

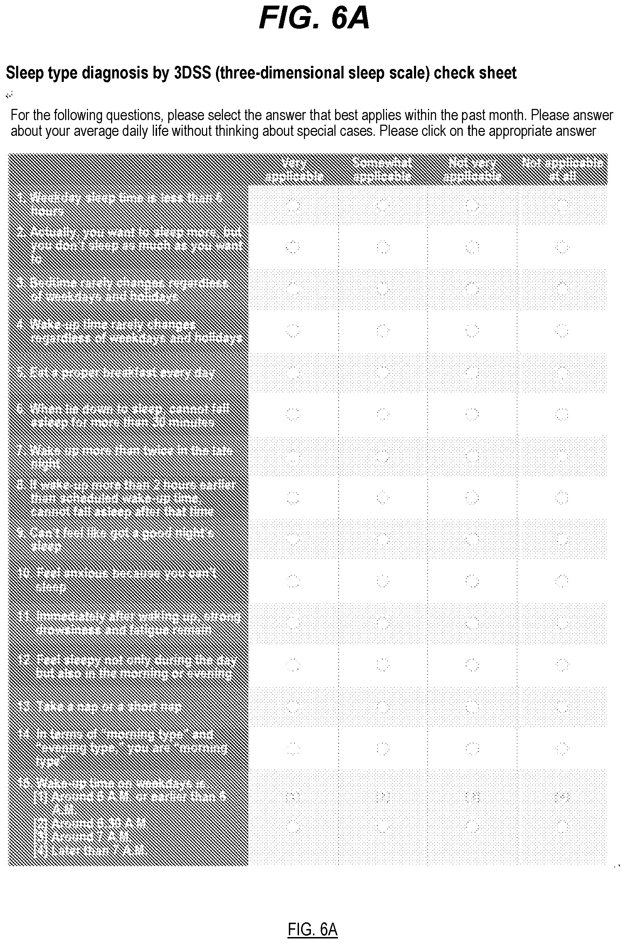

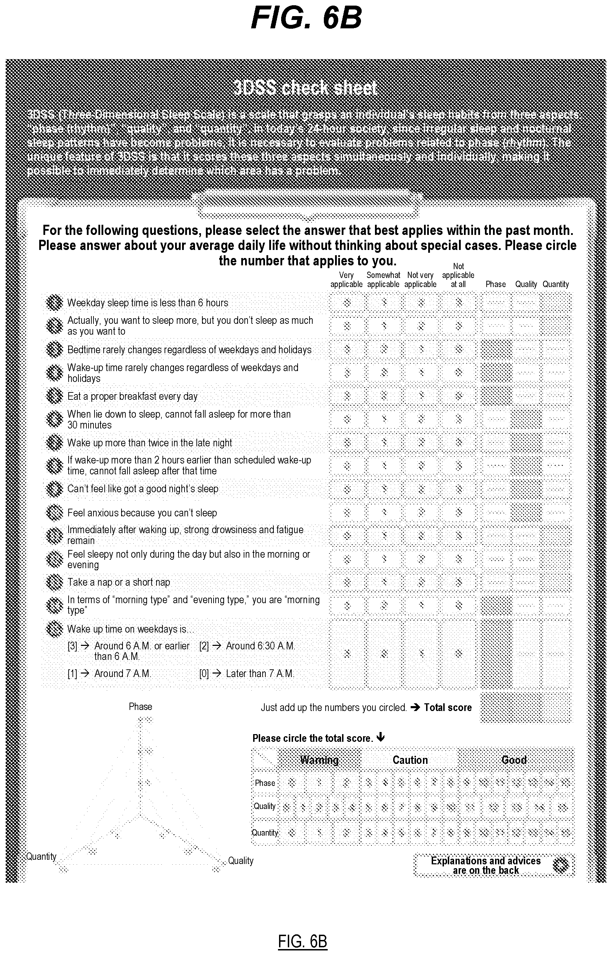

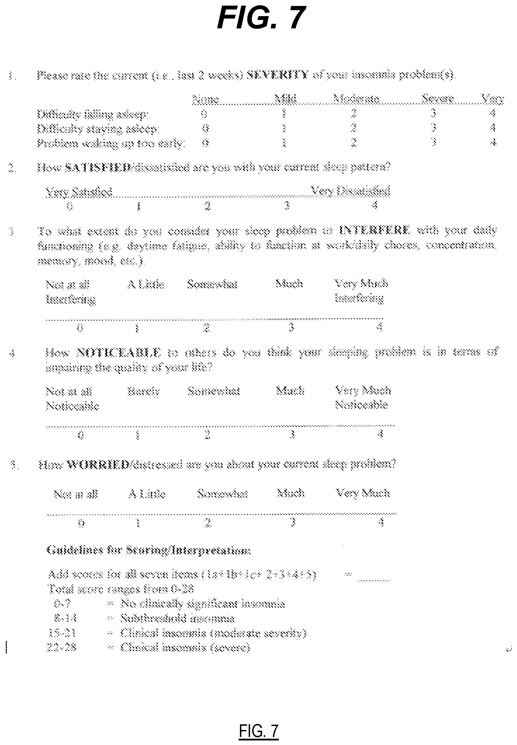

- A score calculated by a method described in an Athens Insomnia Scale (AIS) (refer to Fig. 4) based on the answer content of the sample user to the question items described in the Athens Insomnia Scale, or a corrected score obtained by calculating (adding, subtracting, multiplying, dividing, or the like) this score by an optional method.
- A score calculated by a method described in a Pittsburgh Sleep Quality Index (PSQI) (refer to Figs. 5A to 5C) based on the answer content of the sample user to the question items described in the Pittsburgh Sleep Quality Index, or a corrected score obtained by calculating (adding, subtracting, multiplying, dividing, or the like) this score by an optional method.
- A score calculated by a method described in a three-dimensional sleep scale (3DSS) (refer to Figs. 6A and 6B) based on the answer content of the sample user to the question items described in the three-dimensional sleep scale, or a corrected score obtained by calculating (adding, subtracting, multiplying,dividing, or the like) this score by an optional method.
- A score calculated by a method described in an insomnia severity index (ISI) (refer to Fig. 7) based on the answer content of the sample user to the question items described in the insomnia severity index, or a corrected score obtained by calculating (adding, subtracting, multiplying, dividing, or the like) this score by an optional method.
- A score calculated by a method described in a questionnaire or scale based on the answer content of the sample user to the question items described in the any other questionnaire or scale, or a corrected score obtained by calculating this score by an optional method.

Second, the sample rhythm data may be data for identifying whether the quality of rhythm of the sample user is good (for example, how good) or bad (for example, how bad). In one example, the sample rhythm data may be data for identifying a score that has evaluated the sleep rhythm of the sample user.

In this case, the sleep rhythm of the sample user may be, for example, data for identifying a social jet lag (or a value obtained by calculating this social jet lag by an optional method) that is an absolute value of a difference between a median time of the sleep time zone on weekdays and a median time of the sleep time zone on holidays of the sample user. Here, for example, in a case where the sleep time zone of a certain sample user on weekdays is from 0:00 AM to 6:00 AM, the median time is 3:00 AM, and in a case where the sleep time zone of the sample user on holidays is from 3:00 AM to 11:00 AM, the median time is 7:00 AM, and thus the social jet lag is four hours. In general, it can be said that the smaller (or larger) the social jet lag, the better (or worse) the sleep rhythm of the sample user.

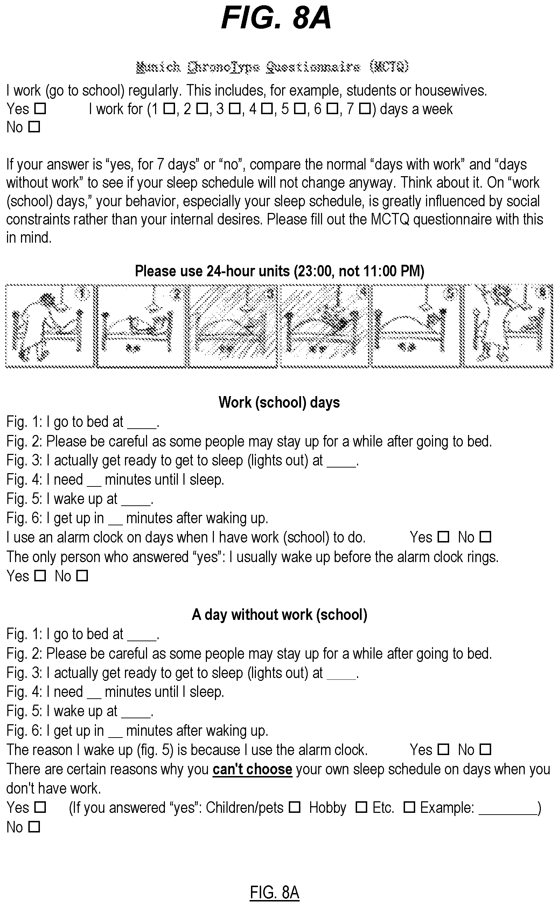

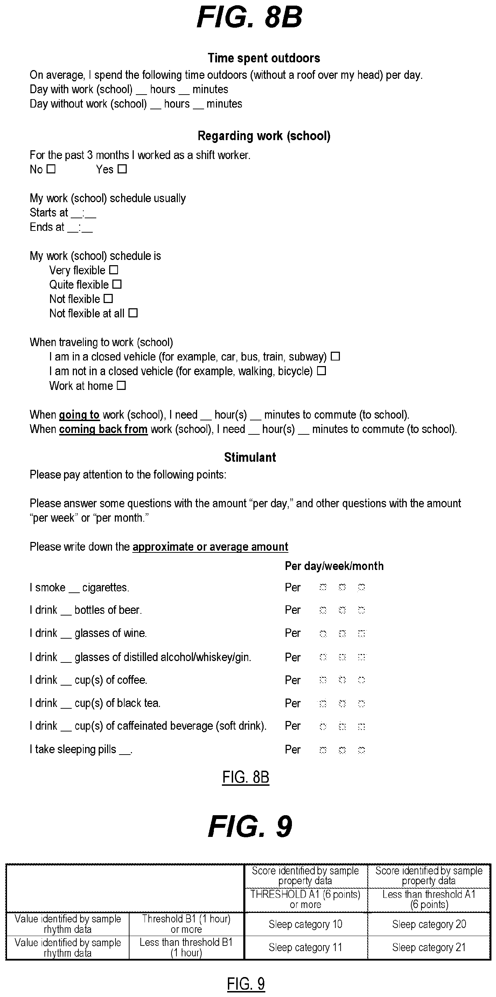

The sleep time zones of the sample user on weekdays and holidays can be extracted, in one example, from answer contents by the sample user to question items described in the Munich Chronotype Questionnaire (MCTQ) (refer to Figs. 8A and 8B), or can be obtained, in another example, from the sample user via an email, a web page, or the like.

Third, the sample time data may be data for identifying the sleep time (daily sleep time) of the sample user. The sleep time of the sample user can be extracted, in one example, from answer contents by the sample user to question items described in the Munich Chronotype Questionnaire (MCTQ) (refer to Figs. 8A and 8B), or can be obtained, in another example, from the sample user via an email, a web page, or the like.

Fourth, the sample sleep category data may be data for identifying a sleep category to which (the sleep of) the sample user belongs. As an example, the sample sleep category data may be data for identifying a category to which (the sleep of) the sample user belongs, determined based on the sample property data of the sample user and the sample rhythm data of the sample user. In this case, the sleep category can be determined, for example, by the following method.

Fig. 9 is a diagram illustrating an example of sleep category data used in the communication system 1 illustrated in Fig. 1. As illustrated in Fig. 9, a score identified by the sample property data may be arranged on one (here, the horizontal axis) of the vertical axes and the horizontal axis, and a value identified by the sample rhythm data may be arranged on the other (here, the vertical axis) of the vertical axis and the horizontal axis. In the one axis, at least one (here, one) threshold, that is, a threshold A1 (six points) can be set for the score identified by the sample property data. In the other axis, at least one (here, one) threshold, that is, a threshold B1 (one hour) can be set for the value identified by the sample rhythm data. As a result, four mutually different sleep categories (here, sleep categories 10, 11, 20, and 21) divided from the viewpoint of the sleep property and the sleep rhythm can be formed by the threshold A1 and the threshold B1. In this example, it can be said that the sleep category 10 is a category in which the disorder occurring in sleep is the most likely to be prominent, the sleep category 21 is a category in which the disorder occurring in sleep is the least likely to be occurring, and the sleep categories 11 and 20 are categories positioned between the sleep categories 10 and 21. The sleep category data may be data for identifying any of the four sleep categories.

Fig. 10 is a diagram illustrating another example of sleep category data used in the communication system 1 illustrated in Fig. 1. In the example illustrated in Fig. 10, for example, two thresholds, that is, the threshold A1 (6 points) and a threshold A2 (8 points) can be set for the score identified by the sample property data in the one axis. In the other axis, for example, two thresholds, that is, the threshold B1 (one hour) and a threshold B2 (three hours) can be set for the value identified by the sample rhythm data. As a result, nine mutually different sleep categories (here, sleep categories 100, 101, 102, 200, 201, 202, 300, 301, and 302) divided from the viewpoint of the sleep property and the sleep rhythm can be formed by the thresholds A1 and A2 and the thresholds B1 and B2. In this example, it can be said that the sleep category 300 is a category in which the disorder occurring in sleep is the most likely to be prominent, the sleep category 202 is a category in which the disorder occurring in sleep is the least likely to be occurring, and the remaining sleep categories are categories positioned between the sleep categories 300 and 202. The sleep category data may be data for identifying any of the nine sleep categories.

The examples illustrated in Figs. 9 and 10 are merely examples illustrated for convenience, and an optional number of thresholds can be set for the score identified by the sample property data, and an optional number of thresholds can be set for the value identified by the sample rhythm data. By setting more thresholds for each of the sample property data and the sample rhythm data, it is possible to generate more mutually different sleep categories divided from the viewpoint of the sleep property and the sleep rhythm.

For example, the server device 10A (and/or the terminal device 20), which is an information processing device managed by a company that operates an estimation service or the like, can generate and store a set of teacher data for each sample user by combining such sample behavior data and sample sleep state data for each sample user. In order to achieve this, for example, the information processing device can extract sample behavior data to which identification data for identifying a certain sample user is attached from a plurality of pieces of acquired sample behavior data, extract sample sleep state data to which identification data for identifying the same sample user is attached from a plurality of pieces of acquired sample sleep state data, and combine these to generate and store a set of teacher data for the sample user. By executing similar processing for each of the plurality of sample users, the information processing device can generate and store a plurality of sets of teacher data.

(2) Step 1002

Returning to Fig. 3, next, in ST1002, the server device 10A (and/or the terminal device 20), which is an information processing device, for example, managed by a company that operates an estimation service or the like, can generate an estimation model by causing a learning model to learn by machine learning using the plurality of sets of teacher data acquired in ST1000. The machine learning herein may include, but is not limited to, any known machine learning, for example, a gradient boosting tree (for example, LightGBM), a neural network, a linear regression, or the like.

The information processing device can execute learning by sequentially inputting the plurality of sets of teacher datadata to the learning model. Here, as described above, each set of teacher data can include sample behavior data of the sample user corresponding to the set and sample sleep state data of the sample user corresponding to the set. The information processing device can use the sample behavior data included in each set of teacher data as an explanatory variable and the sample sleep state data included in the set of teacher data as an objective variable.

(2A) Example of machine learning

For example, in a case where a gradient boosting tree is used, when sample behavior data (explanatory variable) included in a set of teacher data is input to learning data, the information processing device can create a decision tree and add the decision tree to the learning model so as to improve an objective function calculated from sample sleep state data (objective variable) and an estimated value (that is, the value output from the learning model). The information processing device can repeat this processing for the number of decision trees determined by the hyperparameter. For the second and subsequent decision trees, learning can be performed on the difference between the objective variable and the estimated value based on the decision tree created so far. In this way, the information processing device can determine the weight of the branch and the leaf of each decision tree. That is, in the gradient boosting tree, each decision tree uses the explanatory variable in the teacher data as the input data, and sets the value of the leaf node reached by tracing each branch of the decision tree as the output data (objective variable). Furthermore, the method of each branch of the decision tree and the value of each leaf node can be optimized such that the error between the data (objective variable) and the objective variable in the teacher data is reduced.

For example, in a case where the information processing device uses a neural network including an input layer, an intermediate layer, and an output layer, the information processing device can optimize (learn) each parameter (weight) included in the neural network using an error back propagation method so as to reduce an error between data (objective variable) output from the output layer by inputting the explanatory variable of the teacher data to the input layer and the objective variable of the teacher data.

In the case of using the linear regression, the information processing device can learn the parameter of the linear regression model so as to improve the error between the objective variable and the estimated value. This linear regression model is divided into the input layer and the output layer, and parameters included in the linear regression model can be optimized using the least squares method such that an error between data (objective variable) output from the output layer by inputting the explanatory variable of teacher data to the input layer as input data and the objective variable of the teacher data is reduced.

(2B) First plurality of sets of teacher data included in plurality of sets of teacher data

In one example, the information processing device can generate (update) the estimation model by using a first plurality of sets of teacher data included in the plurality of sets of teacher data. Each set of teacher data included in the first plurality of sets of teacher data can include sample behavior data of the sample user corresponding to the set and sample sleep state data of the sample user corresponding to the set. Here, the sample sleep state data in each set of teacher data can include sample property data of the sample user corresponding to the set and sample rhythm data of the sample user.

In this case, the information processing device can execute preprocessing for each set of teacher data included in the first plurality of sets of teacher data. Specifically, the information processing device performs the processing described above with reference to Figs. 9 and 10 using the sample property data and the sample rhythm data included in each set of teacher data, thereby performing preprocessing of generating sample sleep category data for the set. As a result, the information processing device can generate (update) the estimation model by inputting the first plurality of sets of teacher data (each set of teacher data includes sample behavior data of the sample user corresponding to the set and sample sleep category data of the sample user) after the preprocessing to the learning model. As will be described later, such an estimation model can output at least the target sleep category data for identifying the sleep category to which the target user belongs in response to the input of the target behavior data for identifying the history of the behavior executed by the target user as the explanatory variable.

(2C) Second plurality of sets of teacher data included in plurality of sets of teacher data

In one example, the information processing device can generate (update) the estimation model by using a second plurality of sets of teacher data included in the plurality of sets of teacher data. Each set of teacher data included in the second plurality of sets of teacher data can include sample behavior data of the sample user corresponding to the set and sample sleep state data of the sample user corresponding to the set. Here, the sample sleep state data in each set of teacher data can include sample sleep category data of the sample user corresponding to the set.

In this case, the information processing device can generate (update) the estimation model by inputting the second plurality of sets of teacher data (each set of teacher data includes sample behavior data of the sample user corresponding to the set and sample sleep category data of the sample user) to the learning model. As will be described later, such an estimation model can output at least the target sleep category data for identifying the sleep category to which the target user belongs in response to the input of the target behavior data for identifying the history of the behavior executed by the target user as the explanatory variable.

(2D) Third plurality of sets of teacher data included in plurality of sets of teacher data

In one example, the information processing device can generate (update) the estimation model by using a third plurality of sets of teacher data included in the plurality of sets of teacher data. Each set of teacher data included in the third plurality of sets of teacher data can include sample behavior data of the sample user corresponding to the set and sample sleep state data of the sample user corresponding to the set. Here, the sample sleep state data in each set of teacher data can include sample property data of the sample user corresponding to the set and sample rhythm data of the sample user.

In this case, the information processing device can generate (update) the estimation model by inputting the third plurality of sets of teacher data (each set of teacher data includes sample behavior data of the sample user corresponding to the set, sample property data of the sample user, and sample rhythm data of the sample user) to the learning model. As will be described later, such an estimation model can output at least target property data for identifying the sleep property of the target user and target rhythm data for identifying the sleep rhythm of the target user in response to the input of the target behavior data for identifying the history of the behavior executed by the target user as the explanatory variable.

(2E) Fourth plurality of sets of teacher data included in plurality of sets of teacher data

In one example, the information processing device can generate (update) the estimation model by using a fourth plurality of sets of teacher data included in the plurality of sets of teacher data. Each set of teacher data included in the fourth plurality of sets of teacher data can include sample behavior data of the sample user corresponding to the set and sample sleep state data of the sample user corresponding to the set. Here, the sample sleep state data in each set of teacher data can include sample property data of the sample user corresponding to the set.

In this case, the information processing device can generate (update) the estimation model by inputting the fourth plurality of sets of teacher data (each set of teacher data includes sample behavior data of the sample user corresponding to the set and sample property data of the sample user) to the learning model. As will be described later, such an estimation model can output at least the target property data for identifying a sleep property of the target user in response to the input of the target behavior data for identifying the history of the behavior executed by the target user as the explanatory variable.

(2F) Fifth plurality of sets of teacher data included in plurality of sets of teacher data

In one example, the information processing device can generate (update) the estimation model by using a fifth plurality of sets of teacher data included in the plurality of sets of teacher data. Each set of teacher data included in the fifth plurality of sets of teacher data can include sample behavior data of the sample user corresponding to the set and sample sleep state data of the sample user corresponding to the set. Here, the sample sleep state data in each set of teacher data can include sample rhythm data of the sample user corresponding to the set.

In this case, the information processing device can generate (update) the estimation model by inputting the fifth plurality of sets of teacher data (each set of teacher data includes sample behavior data of the sample user corresponding to the set and sample rhythm data of the sample user) to the learning model. As will be described later, such an estimation model can output at least the target rhythm data for identifying a sleep rhythm of the target user in response to the input of the target behavior data for identifying the history of the behavior executed by the target user as the explanatory variable.

(2G) Sixth plurality of sets of teacher data included in plurality of sets of teacher data

In one example, the information processing device can generate (update) the estimation model by using a fifth plurality of sets of teacher data included in the plurality of sets of teacher data. Each set of teacher data included in the sixth plurality of sets of teacher data can include sample behavior data of the sample user corresponding to the set and sample sleep state data of the sample user corresponding to the set. Here, the sample sleep state data in each set of teacher data can include sample time data of the sample user corresponding to the set.

In this case, the information processing device can generate (update) the estimation model by inputting the sixth plurality of sets of teacher data (each set of teacher data includes sample behavior data of the sample user corresponding to the set and sample time data of the sample user) to the learning model. As will be described later, such an estimation model can output at least the target time data for identifying a sleep time of the target user in response to the input of the target behavior data for identifying the history of the behavior executed by the target user as the explanatory variable.

Generation (update) of such an estimation model can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like. The estimation model generated (updated) by the above-described another server device 10 (terminal device 20) can be transmitted to an optional server device 10 including the server device 10A, or can be accessed and used by an optional server device 10 including the server device 10A.

Note that the names "first" to "sixth" added immediately before the "plurality of sets of teacher data" are used for convenience to distinguish between the "plurality of sets of teacher data" given a certain name among the names and the "plurality of sets of teacher data" given another certain name among the names, and do not limit any order, content, or the like.

(3) Step 1004

Referring again to Fig. 3, next, in ST1004, the server device 10A (and/or the terminal device 20), which is an information processing device, for example, managed by the company that operates an estimation service or the like, can acquire target behavior data for identifying a history of behavior executed by the target user to be estimated, for example, from the terminal device 20 (or another server device 10) that executes a specific application (an application for receiving provision of an estimation service or the like) to access the server device 10A and/or from the terminal device 20 that executes a browser to access the server device 10A. The target behavior data may be data for identifying at least one behavior (one or more) executed by the target user.

Such acquisition of the target behavior data can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like.

(4) Step 1006

Next, in ST1006, the server device 10A (and/or the terminal device 20), which is an information processing device, for example, managed by a company that operates an estimation service or the like, can acquire target sleep state data for identifying a sleep state of the target user by using the target behavior data acquired in ST1004 and the estimation model generated in ST1002. Specifically, the server device 10A can output the target sleep state data as the objective variable from the estimation model by inputting the target behavior data as the explanatory variable to the estimation model.

The target sleep state data can include at least one of the following data.

- Data ("target property data") for identifying a sleep property of a target user
- Data ("target rhythm data") for identifying a sleep rhythm of a target user
- Data ("target time data") for identifying a sleep time of a target user
- Data ("target sleep category data") for identifying a sleep category to which (the sleep of) a target user belongs

Which data the estimation model outputs as the target sleep state data may depend on which data is used as the sample sleep state data when the estimation model is generated (updated).

For example, in a case where the first plurality of sets of teacher data is used as the sample sleep state data at the time of generating (updating) the estimation model (the above "4(2)(2B)" section), the estimation model can output at least the target sleep category data as the target sleep state data.

For example, in a case where the second plurality of sets of teacher data is used as the sample sleep state data at the time of generating (updating) the estimation model (the above "4(2)(2C)" section), the estimation model can output at least the target sleep category data as the target sleep state data.

For example, in a case where the third plurality of sets of teacher data is used as the sample sleep state data at the time of generating (updating) the estimation model (the above "4(2)(2D)" section), the estimation model can output at least the target property data and target rhythm data as the target sleep state data.

For example, in a case where the fourth plurality of sets of teacher data is used as the sample sleep state data at the time of generating (updating) the estimation model (the above "4(2)(2E)" section), the estimation model can output at least the target property data as the target sleep state data.

For example, in a case where the fifth plurality of sets of teacher data is used as the sample sleep state data at the time of generating (updating) the estimation model (the above "4(2)(2F)" section), the estimation model can output at least the target rhythm data as the target sleep state data.

For example, in a case where the sixth plurality of sets of teacher data is used as the sample sleep state data at the time of generating (updating) the estimation model (the above "4(2)(2G)" section), the estimation model can output at least the target time data as the target sleep state data.

For example, in a case where the estimation model is generated (updated) using the first plurality of sets of teacher data as the sample sleep state data (the above "4(2)(2B)" section), and thereafter, the estimation model is generated (updated) using the sixth plurality of sets of teacher data as the sample sleep state data (the above "4(2)(2G)" section), the estimation model can output at least the target sleep category data and the target time data as the target sleep state data. Further, thereafter, when the estimation model is generated (updated) using the fifth plurality of sets of teacher data as the sample sleep state data (the above "4(2)(2F)" section), the estimation model can output at least the target sleep category data, the target time data, and the target rhythm data as the target sleep state data.

As a method for using the estimation model, any one of the following methods can be used.

- The server device 10A acquires and holds the estimation model generated in ST1004, inputs the target behavior data to the estimation model, and acquires the target sleep state data output from the estimation model.
- The server device 10A transmits the target behavior data via the communication network 2 to an external device (any other server device 10 or any terminal device 20) that acquires and holds the estimation model generated in ST1004, thereby causing the estimation model having the target behavior data input as an explanatory variable to output the target sleep state data. Thereafter, the server device 10A receives the target sleep state data output by the estimation model from the external device via the communication network 2.

Such acquisition of the target sleep state data can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like.

(5) Step 1008

Next, in ST1008, the server device 10A (and/or the terminal device 20), which is an information processing device, for example, managed by the company that operates an estimation service or the like, can determine at least one target product/one target service corresponding to the target sleep state data by using the target sleep state data acquired in ST1006. Such a target product/target service may be suitable for the current (estimated) sleep state of the target user identified by the target sleep state data.

Specifically, in one example, the server device 10A can first prepare and store a search table that stores the target sleep state data and a plurality of products and/or a plurality of services in association with each other. Next, by inputting the target sleep state data acquired in ST1006 to the search table, the server device 10A can acquire data ("proposal data") for identifying at least one target product and/or at least one target service from the search table.

Fig. 11 is a diagram conceptually illustrating an example of the search table used in the communication system 1 illustrated in Fig. 1. The search table illustrated in Fig. 11 stores at least one target product and/or at least one target service to be proposed in association with each data (each of the target property data, the target rhythm data, the target time data, and the target sleep category data) that can be included in the target sleep state data.

In this example, for simplification of description, at least one target product and/or at least one target service to be proposed can be assigned in association with each of three mutually different levels indicated by each of the target property data, the target rhythm data, the target time data, and the target sleep category data. Taking the target property data as an example, at least one target product and/or at least one target service to be proposed can be assigned in association with each of a class V1 (class having good sleep property of target user), a class V2 (class having standard sleep property of target user), and a class V3 (class having bad sleep property of target user) indicated by the target property data.

Classes W1 to W3 may also indicate that the sleep rhythm of the target user is "good", "standard", and "bad" in this order. Classes X1 to X3 may also indicate that the sleep time of the target user is "long", "standard", and "short" in this order.

Classes Y1 to Y3 may also indicate that the sleep categories to which (the sleep of) the target user belongs are "good", "standard", and "bad" in this order. In one example, it can be considered that the class Y1 corresponds to the "sleep category 21" illustrated in Fig. 9, the class Y2 corresponds to the "sleep category 20" or the "sleep category 11" in Fig. 9, and the class Y3 corresponds to the "sleep category 10" in Fig. 9.

All of products P1 to P11 illustrated in Fig. 11 indicate optional products suitable for improving the sleep state of the user, and all of services S1 to S8 illustrated in Fig. 11 indicate optional services suitable for improving the sleep state of the user. These products can include, but are not limited to, foods, daily necessities, medicines, exercise equipment, electrical appliances, and the like. In addition, these services can include, but are not limited to, a hospital, a clinic, a sports gym, and the like.

For example, in a case where the target property data in the target sleep state data indicates the class V3, the server device 10A can determine the product P4 and the service S1 as the target product and the target service, respectively. Furthermore, for example, in a case where the target sleep state data includes the target time data (indicating class X2) and the target rhythm data (indicating class W3), the server device 10A can determine the product P8 as the target product and the services S1, S3, and S4 as the target service.

Note that, for each data included in the target sleep state data, the total number of classes to be used, the total number of products and/or services assigned to each class, and the like can be optionally determined.

In another example, the server device 10A can determine at least one target product and/or at least one target service by using another estimation model generated by executing supervised learning (machine learning). The another estimation model can be generated (updated) by executing supervised learning with data indicating each class of each data included in the target sleep state data as an explanatory variable and data indicating at least one target product and/or at least one target service as an objective variable.

The machine learning for learning the another estimation model may include, for example, any known machine learning, for example, a gradient boosting tree (for example, LightGBM), a neural network, a linear regression, or the like without limitation, as described in the "2A" section above.

By inputting the target sleep state data to the another estimation model, the server device 10A can output data ("proposal data") for identifying at least one target product and/or at least one target service from the another estimation model.

As a method for using the another estimation model, any one of the following methods can be used.

• The server device 10A acquires and holds the generated another estimation model, inputs the target sleep state data to the another estimation model, and acquires the proposal data output from the another estimation model.
- The server device 10A transmits target sleep state data via the communication network 2 to an external device (any other server device 10 or any terminal device 20) that acquires and holds the another generated estimation model, thereby causing the another estimation model having the target sleep state data input as an explanatory variable to output the proposal data. Thereafter, the server device 10A receives the proposal data output by the another estimation model from the external device via the communication network 2.

Such acquisition of the proposal data can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like.

(6) Step 1010

Referring again to Fig. 3, next, for example, the server device 10A (and/or the terminal device 20), which is the information processing device, for example, managed by the company that operates the estimation service and the like, can provide (transmit) the proposal data acquired in ST1008 to at least one terminal device 20 (which may be another server device 10) managed by the company user and/or the individual user via the communication network 2. The "individual user" as used herein may include a "target user".

The provision of the proposal data can be provided to at least one terminal device 20 (which may be another server device 10) managed by the company user and/or the individual user by any means including a web page, an e-mail, a chat, an electronic file, a recording medium (USB memory, CD-ROM, or the like), and/or a paper medium.

Such provision of the proposal data can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like.

5. Modification

In the various examples described above, the case where the estimation model is generated and used using the sample property data, the sample rhythm data, the sample time data, and the sample sleep category data as the objective variable has been described. In another example, it is also possible to generate and use the estimation model using at least one (up to three) of the sample property data, the sample rhythm data, the sample time data, and the sample sleep category data as an explanatory variable rather than an objective variable.

Furthermore, in the various examples described above, a case where the execution subject of the various steps (for example, ST1000 to ST1010) illustrated in Fig. 3 is mainly the server device 10A (and/or the terminal device 20) managed by the company that operates the estimation service and the like has been described. However, it is also possible to cause a plurality of optional server devices 10 and/or a plurality of optional terminal devices 20 to share and execute various steps illustrated in Fig. 3. Here, the plurality of optional server devices 10 and/or the plurality of optional terminal devices 20 may include, in one example, the server device 10A (and/or the terminal device 20) managed by the company that operates the estimation service and the like, and in another example, the server device 10A (and/or the terminal device 20) managed by the company that operates the estimation service and the like is not included.

Note that, in "4 (4) Step 1006" described above, the definitions of the target property data, the target rhythm data, the target time data, and the target sleep category data that can be included in the target sleep state data are indicated. In another embodiment, the target sleep state data can include at least one of the following data instead of the data shown in the above "4 (4) Step 1006".

- Data ("target property data") for "uniquely" identifying a sleep property of a target user
- Data ("target rhythm data") for "uniquely" identifying a sleep rhythm of a target user
- Data ("target time data") for "uniquely" identifying a sleep time of a target user
- Data ("target sleep category data") for "uniquely" identifying a sleep category to which (the sleep of) a target user belongs

Here, "uniquely identifying" the "target property data" means that the sleep property of the target user can be identified without using other data other than the "target property data". That is, in a case where a plurality of pieces of data cooperatively identify the sleep property of the target user, the plurality of pieces of data does not correspond to data that "uniquely identifies" the sleep property of the target user. For example, a case is considered in which the first data includes data indicating that the sleep quality of the target user is "poor" and a flag "0", the second data includes data indicating that the sleep quality of the target user is "standard" and a flag "0", and the third data includes data indicating that the sleep quality of the target user is "high" and a flag "1". In this case, the sleep property of the target user cannot be determined only by the first data and the second data. Only by checking the third data with the flag "1", it can be determined that the sleep quality of the target user is "high". Therefore, none of these pieces of data is data for "uniquely" identifying the quality of sleep of the target user.

The term "uniquely identify" included in each of the definitions of "target rhythm data", "target time data", and "target sleep category data" can be similarly considered.

As described above, according to the technology disclosed in the present application, the target behavior data for identifying the history of at least one behavior executed by the target user is input to the estimation model, whereby the target sleep state data for identifying the current (predicted) sleep state of the target user can be acquired from the estimation model. Furthermore, among the plurality of target products and/or the plurality of target services prepared in advance, at least one target product and/or at least one target service suitable for the target sleep state data acquired in this manner can be proposed to any user including the target user in order to improve the sleep state of the target user.

As a result, it is possible to provide a computer program, an information processing device, and a method that are used to estimate the sleep state of the target user based on the history of at least one behavior executed by the target user, and have at least partially improved performance.

6. Operation performed by communication system 2 to provide menopause state data on menopause symptom state

Next, a specific example of the operation executed by the communication system 2 described above in order to provide the menopause state data on the menopause symptom state will be further described with reference to Fig. 12. Fig. 12 is a flowchart illustrating another example of an operation executed by the communication system 1 illustrated in Fig. 1.

(1) Step 1100

First, in ST1100, the server device 10A, which is an information processing device, for example, managed by a company that operates an estimation service or the like, can acquire and store a plurality of sets of teacher data used to generate the estimation model.

Each set of teacher data constituting the plurality of sets of teacher data can include data ("sample behavior data") for identifying a history of behavior executed by one sample user corresponding to the set and data ("sample menopause state data") for identifying a menopause symptom state of one sample user corresponding to the set. For example, the first set of teacher data can include sample behavior data for identifying a history of behaviors executed by one sample user (Mr. V) corresponding to the first set and sample menopause state data for identifying a menopause symptom state of Mr. V, and the second set of teacher data can include sample behavior data for identifying a history of behaviors executed by one sample user (Mr. W) corresponding to the second set and sample menopause state data for identifying a menopause symptom state of Mr. W.

(1A) Sample behavior data

Each sample behavior data may be data for identifying at least one (one or plurality of) behavior of a plurality of predetermined behaviors. Here, each of the plurality of predetermined behaviors may be a behavior that can at least partially positively or negatively affect the menopause symptom state of the human who has executed the behavior. Such a plurality of predetermined behaviors can include, for example, those exemplified below without being limited thereto.

(Example of behavior that can at least partially positively affect human menopause symptom state)

- Aerobic exercise was performed for 1 hour or more at a pace of 2 days or more per week.
- Waking up at a constant time every day.
- Time to relax was secured every week.
- 10 or more cans of beer were purchased (this behavior can be identified, for example, by data including a product category to which beer belongs and its purchase quantity).

(Example of behavior that may at least partially adversely affect human menopause symptom state)

- Aerobic exercise was performed for 1 hour or more at a pace of 1 day or less per week.
- Waking up at different times each day.
- Working without a single day off a week.
- Five or more bottles of green juice were purchased (this behavior can be identified, for example, by data including a product category to which green juice belongs and its purchase quantity).

Each sample behavior data can be data for identifying each of at least one behavior among the plurality of predetermined behaviors exemplified in this manner.

Furthermore, in one example, since the product purchased by the user and/or the service used by the user can tend to have a positive influence or a negative influence on the menopause symptom state of the user, the plurality of predetermined behaviors can include the product purchased by the sample user (or the category to which the product belongs) and the service used by the sample user (or the category to which the service belongs). This type of behavior can be generated using, for example, point of sales (POS) data held by a retail store (drug stores, supermarkets, convenience stores, or the like) or the like.

In one example, unique identification data (alphabetic characters, numbers, combinations thereof, or the like) is assigned to each of the plurality of predetermined behaviors, and each sample behavior data can include such identification data. For example, when POS data is used, each of the plurality of sample behavior data can include, but is not limited to, JICFS classification (classification code), a product category, a purchase amount, a purchase quantity, a purchase count, and/or a purchase date corresponding to a purchased product.

For example, the server device 10A, which is an information processing device and is managed by a company that operates an estimation service or the like, can acquire such sample behavior data by, for example, at least one of the following methods.

- The information processing device receives the POS data from at least one other server device 10 (for example, another server device 10 that stores POS data installed in a drug store or the like, or another server device 10 that can access the POS data) via the communication network 2.
- The information processing device receives, from at least one terminal device 20 and/or at least one server device 10 via the communication network 2, a mail or a web page describing an answer to a predetermined questionnaire (questionnaire regarding behavior executed by sample users).
- The information processing device receives content of answers from at least one server device 10 that has collected answers from a plurality of users to the predetermined questionnaire via the communication network 2.
- The information processing device receives content of answers from a plurality of users to the predetermined questionnaire via a recording medium (USB memory, DVD-ROM, or the like) that has collected the answers.

(1B) Sample menopause state data

The sample menopause state data included in each set of teacher data can include, but is not limited to, at least one of the data exemplified below.

- Data ("sample comprehensive state data") for identifying a comprehensive state of one sample user corresponding to the set
- Data ("sample first state data") for identifying a first symptom state of one sample user corresponding to the set
- Data ("sample second state data") for identifying a second symptom state of one sample user corresponding to the set

First of all, the sample comprehensive state data may be data for identifying whether the menopause symptom state of the sample user is good (for example, how good) or bad (for example, how bad). In one example, the sample comprehensive state data may be data for identifying a score that has comprehensively evaluated the menopause symptom state of the sample user. In this case, the menopause symptom state of the sample user may be, for example, data for identifying a score calculated by any of the methods exemplified below.

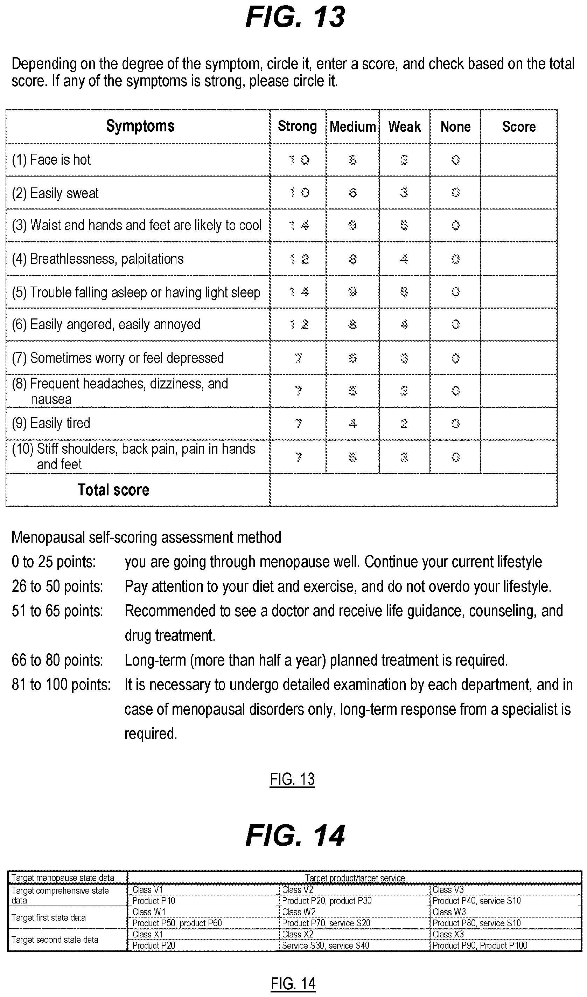

- A score calculated by a method described in a simplified menopause index (SMI) (refer to Fig. 13) based on the answer content of a sample user to the question items described in the SMI, or a corrected score obtained by calculating (adding, subtracting, multiplying, dividing, or the like) this score by an optional method.
- A score calculated by a method described in a menopause rating scale based on the answer content of a sample user to the question items described in the menopause rating scale, or a corrected score obtained by calculating (adding, subtracting, multiplying, dividing, or the like) this score by an optional method.
- A score calculated by a method described in a Kupperman index based on the answer content of a sample user to the question items described in the Kupperman index, or a corrected score obtained by calculating (adding, subtracting, multiplying, dividing, or the like) this score by an optional method.

- A score calculated by a method described in a greene climacteric scale based on the answer content of a sample user to the question items described in the greene climacteric scale, or a corrected score obtained by calculating (adding, subtracting, multiplying, dividing, or the like) this score by an optional method.
- A score calculated by a method described in a PSST (Premenstrual Symptoms Screening Tool) based on the answer content of a sample user to the question items described in the PSST, or a corrected score obtained by calculating (adding, subtracting, multiplying, dividing, or the like) this score by an optional method.
- A score calculated by a method described in a WHQ (Women's Health Questionnaire) based on the answer content of a sample user to the question items described in the WHQ, or a corrected score obtained by calculating (adding, subtracting, multiplying, dividing, or the like) this score by an optional method.
- A score calculated by a method described in a VAS (Visual Analogue Scale) based on the answer content of a sample user to the question items described in the VAS, or a corrected score obtained by calculating (adding, subtracting, multiplying, dividing, or the like) this score by an optional method.
- A score calculated by a method described in a HFRDI (Hot Flash Related Daily Interference Scale) based on the answer content of a sample user to the question items described in the HFRDI, or a corrected score obtained by calculating (adding, subtracting, multiplying, dividing, or the like) this score by an optional method.
- A score calculated by a method described in a HFCS (Hot Flash Composite Score) based on the answer content of a sample user to the question items described in the HFCS, or a corrected score obtained by calculating (adding, subtracting, multiplying, dividing, or the like) this score by an optional method.
- A score calculated by a method described in a MENQOL (Menopause-Specific Quality Of Life) based on the answer content of a sample user to the question items described in the MENQOL, or a corrected score obtained by calculating (adding, subtracting, multiplying, dividing, or the like) this score by an optional method.
- A score calculated by a method described in a menopause symptoms assessment table for Japanese women based on the answer content of a sample user to the question items described in the menopause symptoms assessment table for Japanese women, or a corrected score obtained by calculating (adding, subtracting, multiplying, dividing, or the like) this score by an optional method.
- A score calculated by a method described in a questionnaire or scale based on the answer content of the sample user to the question items described in the any other questionnaire or scale, or a corrected score obtained by calculating this score by an optional method.

Second, the sample first state data may be data for identifying whether one symptom state of the sample user among the plurality of menopause symptoms is good (for example, how good) or bad (for example, how bad). In one example, the sample first state data may be data for identifying a score that has evaluated one symptom state of the sample user.

Taking SMI as an example, the plurality of menopause symptoms may be a plurality of menopause symptoms described in SMI such as "face is hot", "easily sweat", and "waist and hands and feet are likely to cool". The plurality of menopause symptoms may be a plurality of menopause symptoms described in any index or the like listed above in addition to SMI. The one symptom may be any one symptom selected from the plurality of menopause symptoms.

Taking SMI as an example, the sample first state data can be extracted from the answer content by the sample user to the question items described in SMI (refer to Fig. 13) as an example. In this case, the sample first state data is, for example, data for identifying a state of a certain symptom such as "breathlessness, palpitations", and may be, for example, data for identifying a score of 0, 4, 8, or 12 (refer to Fig. 13). In another example, the sample first state data may be obtained from the sample user via an email, a web page, or the like.

Third, the sample second state data may be data for identifying whether another symptom state of the sample user among the plurality of menopause symptoms is good (for example, how good) or bad (for example, how bad). In one example, the sample second state data may be data for identifying a score that has evaluated the another symptom state of the sample user.

Taking SMI as an example, the plurality of menopause symptoms may be a plurality of menopause symptoms described in SMI such as "face is hot", "easily sweat", and "waist and hands and feet are likely to cool". The plurality of menopause symptoms may be a plurality of menopause symptoms described in any index or the like listed above in addition to SMI. The one symptom may be another symptom selected from the plurality of menopause symptoms, but may be a symptom different from the one symptom.

Taking SMI as an example, the sample second state data can be extracted from the answer content by the sample user to the question items described in SMI (refer to Fig. 13) as an example. In this case, the sample second state data is, for example, data for identifying another symptom state such as "easily tired", and may be, for example, data for identifying a score of 0, 2, 4, or 7 (refer to Fig. 13). In another example, the sample second state data may be obtained from the sample user via an email, a web page, or the like.

For example, the server device 10A (and/or the terminal device 20) which is an information processing device managed by a company that operates an estimation service or the like, can generate and store a set of teacher data for each sample user by combining such sample behavior data and sample menopause state data for each sample user. In order to achieve this, for example, the information processing device can extract sample behavior data to which identification data for identifying a certain sample user is attached from a plurality of acquired sample behavior data, extract sample menopause state data to which identification data for identifying the same sample user is attached from a plurality of acquired sample menopause state data, and combine these to generate and store a set of teacher data for the sample user. By executing similar processing for each of the plurality of sample users, the information processing device can generate and store a plurality of sets of teacher data.

(2) Step 1102

Returning to Fig. 3, next, in ST1102, the server device 10A (and/or the terminal device 20), which is an information processing device, for example, managed by a company that operates an estimation service or the like, can generate an estimation model by causing a learning model to learn by machine learning using a plurality of sets of teacher data acquired in ST1100. The machine learning herein may include, but is not limited to, any known machine learning, for example, a gradient boosting tree (for example, LightGBM), a neural network, a linear regression, or the like.

(2A) First plurality of sets of teacher data included in plurality of sets of teacher data

In one example, the information processing device can generate (update) the estimation model by using a first plurality of sets of teacher data included in the plurality of sets of teacher data. Each set of teacher data included in the first plurality of sets of teacher data can include sample behavior data of the sample user corresponding to the set and sample menopause state data of the sample user corresponding to the set. Here, the sample menopause state data in each set of teacher data can include sample comprehensive state data of the sample user corresponding to the set.

In this case, the information processing device can generate (update) the estimation model by inputting the first plurality of sets of teacher data (each set of teacher data includes sample behavior data of the sample user corresponding to the set and sample comprehensive state data of the sample user) to the learning model. As will be described later, such an estimation model can output at least the target comprehensive state data for identifying a comprehensive state of the target user in response to the input of the target behavior data for identifying the history of the behavior executed by the target user as the explanatory variable.

Note that, in one embodiment, a case is considered in which the sample menopause state data in each set of teacher data identifies "score corresponding to" the comprehensive state of the menopause symptoms of one sample user corresponding to the set.

In this case, the information processing device can execute preprocessing for each set of teacher data included in the first plurality of sets of teacher data. Specifically, for each set of teacher data, the information processing device can execute preprocessing of converting the score identified by the sample comprehensive state data corresponding to this set into any category of the plurality of categories using at least one threshold. For example, in a case where one threshold (first threshold) is used, the information processing device can convert the score identified by the sample comprehensive state data corresponding to each set into one of two categories ("high" and "low") according to whether the score is the first threshold or more or less than the first threshold. Alternatively, for example, in a case where two thresholds (first threshold and second threshold smaller than first threshold) are used, the information processing device can convert the score identified by the sample comprehensive state data corresponding to each set into one of three categories ("high", "middle", and "low") according to whether the score is the first threshold or more, the second threshold or more and less than the first threshold, or less than the second threshold.

As described above, for each set of teacher data included in the first plurality of sets of teacher data, the information processing device can execute preprocessing of converting sample comprehensive state data for identifying "score corresponding to" the comprehensive state of the menopause symptoms of one sample user corresponding to this set into sample comprehensive state data for identifying "category corresponding to" the comprehensive state of the menopause symptoms of one sample user corresponding to this set.

As a result, the information processing device can generate (update) the estimation model by inputting the first plurality of sets of teacher data (each set of teacher data includes sample behavior data of the sample user corresponding to the set and sample comprehensive state data of the sample user) after the preprocessing to the learning model.

As will be described later, such an estimation model can output at least the target comprehensive state data for identifying a category corresponding to the comprehensive state of the menopause symptom of the target user in response to the input of the target behavior data for identifying the history of the behavior executed by the target user as the explanatory variable.

(2B) Second plurality of sets of teacher data included in plurality of sets of teacher data

In one example, the information processing device can generate (update) the estimation model by using a second plurality of sets of teacher data included in the plurality of sets of teacher data. Each set of teacher data included in the second plurality of sets of teacher data can include sample behavior data of the sample user corresponding to the set and sample menopause state data of the sample user corresponding to the set. Here, the sample menopause state data in each set of teacher data can include sample first state data of the sample user corresponding to the set.

In this case, the information processing device can generate (update) the estimation model by inputting the second plurality of sets of teacher data (each set of teacher data includes sample behavior data of the sample user corresponding to the set and sample first state data of the sample user) to the learning model. As will be described later, such an estimation model can output at least the target first state data for identifying a first symptom state of the target user in response to the input of the target behavior data for identifying the history of the behavior executed by the target user as the explanatory variable.

Note that, in one embodiment, a case is considered in which the sample first state data in each set of teacher data identifies "score corresponding to" the first symptom state of one sample user corresponding to the set.

In this case, the information processing device can execute preprocessing for each set of teacher data included in the second plurality of sets of teacher data. Specifically, for each set of teacher data, the information processing device can execute preprocessing of converting the score identified by the sample first state data corresponding to this set into any category of the plurality of categories using at least one threshold. For example, in a case where one threshold (first threshold) is used, the information processing device can convert the score identified by the sample first state data corresponding to each set into one of two categories ("high" and "low") according to whether the score is the first threshold or more or less than the first threshold. Alternatively, for example, in a case where two thresholds (first threshold and second threshold smaller than first threshold) are used, the information processing device can convert the score identified by the sample first state data corresponding to each set into one of three categories ("high", "middle", and "low") according to whether the score is the first threshold or more, the second threshold or more and less than the first threshold, or less than the second threshold.

As described above, for each set of teacher data included in the second plurality of sets of teacher data, the information processing device can execute preprocessing of converting sample first state data for identifying the "score corresponding to" the first symptom state of one sample user corresponding to this set into sample first state data for identifying "category corresponding to" the first symptom state of one sample user corresponding to this set.

As a result, the information processing device can generate (update) the estimation model by inputting the second plurality of sets of teacher data (each set of teacher data includes sample behavior data of the sample user corresponding to the set and sample first state data of the sample user) after the preprocessing to the learning model.

As will be described later, such an estimation model can output at least the target first state data for identifying the category corresponding to a first symptom state of the target user in response to the input of the target behavior data for identifying the history of the behavior executed by the target user as the explanatory variable.

Furthermore, the information processing device can further execute preprocessing ("preprocessing B" for convenience) in addition to the preprocessing ("preprocessing A" for convenience) as described above. Specifically, first, the information processing device can execute "preprocessing B" of extracting another plurality of sets of teacher data from the second plurality of sets of teacher data. Each set of teacher data included in the above another plurality of sets of teacher data has sample first state data for identifying a score equal to or higher than a threshold.

For example, when the first symptom is a symptom of "easily sweat" in SMI (refer to Fig. 13), each set of teacher data included in the above another plurality of sets of teacher data may be sample first state data for identifying a score (10 or 6) equal to or higher than a threshold of 5. That is, each set of teacher data included in the above another plurality of sets of teacher data includes teacher data related only to the sample user having the stronger first symptom (here, "easily sweating").

Furthermore, the information processing device can set the above another plurality of sets of teacher data extracted in this manner as "new" second plurality of sets of teacher data. Thereafter, the information processing device can execute the preprocessing A described above on the "new" second plurality of sets of teacher data. In the preprocessing A, at least one threshold to be used can be set to a larger value as compared with the above-described example in which the preprocessing B is not executed. Next, the information processing device can generate (update) the estimation model by inputting the "new" second plurality of sets of teacher data after the preprocessing A to the learning model.

Such a "new" second plurality of sets of teacher data include sample behavior data and sample first state data for only the sample user having the stronger first symptom. Therefore, such an estimation model that performs learning using the "new" second plurality of sets of teacher data can more easily estimate the target user who has performed a behavior that can lead to the strong first symptom as the user having the strong first symptom, and can further finely estimate the level of the strength of the first symptom of such a target user.

(2C) Third plurality of sets of teacher data included in plurality of sets of teacher data

In one example, the information processing device can generate (update) the estimation model by using a third plurality of sets of teacher data included in the plurality of sets of teacher data. Each set of teacher data included in the third plurality of sets of teacher data can include sample behavior data of the sample user corresponding to the set and sample menopause state data of the sample user corresponding to the set. Here, the sample menopause state data in each set of teacher data can include sample second state data of the sample user corresponding to the set.

In this case, the information processing device can generate (update) the estimation model by inputting the third plurality of sets of teacher data (each set of teacher data includes sample behavior data of the sample user corresponding to the set and sample second state data of the sample user) to the learning model. As will be described later, such an estimation model can output at least the target second state data for identifying a second symptom state of the target user in response to the input of the target behavior data for identifying the history of the behavior executed by the target user as the explanatory variable.

Note that, in one embodiment, a case is considered in which the sample second state data in each set of teacher data identifies "score corresponding to" the second symptom state of one sample user corresponding to the set.

In this case, the information processing device can execute preprocessing for each set of teacher data included in the third plurality of sets of teacher data. Specifically, for each set of teacher data, the information processing device can execute preprocessing of converting the score identified by the sample second state data corresponding to this set into any category of the plurality of categories using at least one threshold. For example, in a case where one threshold (first threshold) is used, the information processing device can convert the score identified by the sample second state data corresponding to each set into one of two categories ("high" and "low") according to whether the score is the first threshold or more or less than the first threshold. Alternatively, for example, in a case where two thresholds (first threshold and second threshold smaller than first threshold) are used, the information processing device can convert the score identified by the sample second state data corresponding to each set into one of three categories ("high", "middle", and "low") according to whether the score is the first threshold or more, the second threshold or more and less than the first threshold, or less than the second threshold.

As described above, for each set of teacher data included in the third plurality of sets of teacher data, the information processing device can execute preprocessing of converting sample second state data for identifying the "score corresponding to" the second symptom state of one sample user corresponding to this set into sample second state data for identifying "category corresponding to" the second symptom state one sample user corresponding to this set.

As a result, the information processing device can generate (update) the estimation model by inputting the third plurality of sets of teacher data (each set of teacher data includes sample behavior data of the sample user corresponding to the set and sample first state data of the sample user) after the preprocessing to the learning model.

As will be described later, such an estimation model can output at least the target second state data for identifying the category corresponding to a second symptom state of the target user in response to the input of the target behavior data for identifying the history of the behavior executed by the target user as the explanatory variable.

Furthermore, the information processing device can further execute preprocessing ("preprocessing B" for convenience) in addition to the preprocessing ("preprocessing A" for convenience) as described above. Specifically, first, the information processing device can execute "preprocessing B" of extracting another plurality of sets of teacher data from the third plurality of sets of teacher data. Each set of teacher data included in the above another plurality of sets of teacher data has sample second state data for identifying a score equal to or higher than a threshold.

For example, when the second symptom is a symptom of "easily tired" in SMI (refer to Fig. 13), each set of teacher data included in the above another plurality of sets of teacher data may be sample second state data for identifying a score (7 or 4) equal to or higher than a threshold of 3. That is, each set of teacher data included in the above another plurality of sets of teacher data includes teacher data related only to the sample user having the stronger second symptom (here, "easily tired").

Furthermore, the information processing device can set the above another plurality of sets of teacher data extracted in this manner as "new" third plurality of sets of teacher data. Thereafter, the information processing device can execute the preprocessing A described above on the "new" third plurality of sets of teacher data. In the preprocessing A, at least one threshold to be used can be set to a larger value as compared with the above-described example in which the preprocessing B is not executed. Next, the information processing device can generate (update) the estimation model by inputting the "new" third plurality of sets of teacher data after the preprocessing A to the learning model.

Such a "new" third plurality of sets of teacher data include sample behavior data and sample second state data for only the sample user having the stronger second symptom. Therefore, such an estimation model that performs learning using the "new" third plurality of sets of teacher data can more easily estimate the target user who has performed a behavior that can lead to the strong second symptom as the user having the strong second symptom, and can further finely estimate the level of the strength of the second symptom of such a target user.

As described above, generation (update) of the estimation model can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like. The estimation model generated (updated) by the above-described another server device 10 (terminal device 20) can be transmitted to an optional server device 10 including the server device 10A, or can be accessed and used by an optional server device 10 including the server device 10A.

Note that the names "first" to "third" added immediately before the "plurality of sets of teacher data" are used for convenience to distinguish between the "plurality of sets of teacher data" given a certain name among the names and the "plurality of sets of teacher data" given another name among the names, and do not limit any order, content, or the like.

(3) Step 1104

Referring again to Fig. 12, next, in ST1104, the server device 10A (and/or the terminal device 20), which is an information processing device, for example, managed by a company that operates an estimation service or the like, can acquire target behavior data for identifying a history of behavior executed by the target user to be estimated, for example, from the terminal device 20 (or another server device 10) that executes a specific application (an application for receiving provision of an estimation service or the like) to access the server device 10A and/or from the terminal device 20 that executes a browser to access the server device 10A. The target behavior data may be data for identifying at least one behavior (one or more) executed by the target user.

Such acquisition of the target behavior data can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like.

(4) Step 1106

Next, in ST1106, the server device 10A (and/or the terminal device 20), which is an information processing device, for example, managed by a company that operates an estimation service or the like, can acquire target menopause state data for identifying a menopause symptom state of the target user by using the target behavior data acquired in ST1104 and the estimation model generated in ST1102. Specifically, the server device 10A can output the target menopause state data as the objective variable from the estimation model by inputting the target behavior data as the explanatory variable to the estimation model.

The target menopause state data can include at least one of the following data.

- Data for identifying a comprehensive state of the menopause symptom of a target user ("target comprehensive state data")
- Data for identifying a first symptom state of a target user ("target first state data")
- Data for identifying a second symptom state of a target user ("target second state data")

Which data the estimation model outputs as the target menopause state data may depend on which data is used as the sample menopause state data when the estimation model is generated (updated).

For example, in a case where the first plurality of sets of teacher data is used as the sample menopause state data at the time of generating (updating) the estimation model (the above "6(2) (2A)" section), the estimation model can output at least the target comprehensive state data as the target menopause state data.

For example, in a case where the second plurality of sets of teacher data is used as the sample menopause state data at the time of generating (updating) the estimation model (the above "6(2) (2B)" section), the estimation model can output at least the target first state data as the target menopause state data.

For example, in a case where the third plurality of sets of teacher data is used as the sample menopause state data at the time of generating (updating) the estimation model (the above "6(2) (2C)" section), the estimation model can output at least the target second state data as the target menopause state data.

For example, in a case where the estimation model is generated (updated) using the first plurality of sets of teacher data as the sample menopause state data (the above "6(2) (2A)" section), and thereafter, the estimation model is generated (updated) using the second plurality of sets of teacher data as the sample menopause state data (the above "6(2) (2B)" section), the estimation model can output at least the target comprehensive state data and the target first state data as the target menopause state data. Further, thereafter, when the estimation model is generated (updated) using the third plurality of sets of teacher data as the sample menopause state data (the above "6(2) (2C)" section), the estimation model can output at least the target comprehensive state data, the target first state data, and the target second state data as the target menopause state data.

As a method for using the estimation model, any one of the following methods can be used.

- The server device 10A acquires and holds the estimation model generated in ST1104, inputs the target behavior data to the estimation model, and acquires the target menopause state data output from the estimation model.
- The server device 10A transmits target behavior data via the communication network 2 to an external device (any other server device 10 or any terminal device 20) that acquires and holds the estimation model generated in ST1104, thereby causing the estimation model having the target behavior data input as an explanatory variable to output the target menopause state data. Thereafter, the server device 10A receives the target menopause state data output by the estimation model from the external device via the communication network 2.

Such acquisition of the target menopause state data can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like.

(5) Step 1108

Next, in ST1108, the server device 10A (and/or the terminal device 20), which is an information processing device, for example, managed by a company that operates an estimation service or the like, can determine at least one target product/one target service corresponding to the target menopause state data by using the target menopause state data acquired in ST1106. Such a target product/target service may be suitable for the current (estimated) menopause symptom state of the target user identified by the target menopause state data.

Specifically, in one example, the server device 10A can first prepare and store a search table that stores the target menopause state data and a plurality of products and/or a plurality of services in association with each other. Next, by inputting the target menopause state data acquired in ST1106 to the search table, the server device 10A can acquire data ("proposal data") for identifying at least one target product and/or at least one target service from the search table.

Fig. 14 is a diagram conceptually illustrating another example of a search table used in the communication system 1 illustrated in Fig. 1. The search table illustrated in Fig. 14 stores at least one target product and/or at least one target service to be proposed in association with each data (each of the target comprehensive state data, the target first state data, and the target second state data) that can be included in the target menopause state data.

In this example, for simplification of description, at least one target product and/or at least one target service to be proposed can be assigned in association with each of three mutually different levels indicated by each of the target comprehensive state data, the target first state data, and the target second state data. Taking the target comprehensive state data as an example, at least one target product and/or at least one target service to be proposed can be assigned in association with each of class V1 (class having good comprehensive state of the menopause symptom of the target user), class V2 (class having standard comprehensive state of the menopause symptom of the target user), and class V3 (class having bad comprehensive state of the menopause symptom of the target user) indicated by the target comprehensive state data.

The classes W1 to W3 may also indicate that the first symptom state of the target user is "good", "standard", and "bad" in this order. The classes X1 to X3 may also indicate that the second symptom state of the target user is "good", "standard", and "bad" in this order.

Each of the products (products to which reference numerals including "P" and numbers are attached, such as P10 and P20) illustrated in Fig. 14 indicates an optional product suitable for improving the menopause symptom state of the user, and each of the services (services denoted by reference numerals including "S" and a number, such as S10 and S20) illustrated in Fig. 14 indicates an optional service suitable for improving the menopause symptom state of the user. These products can include, but are not limited to, foods, daily necessities, medicines, exercise equipment, electrical appliances, and the like. In addition, these services can include, but are not limited to, a hospital, a clinic, a sports gym, and the like.

For example, in a case where the target comprehensive state data in the target menopause state data indicates the class V3, the server device 10A can determine the product P40 and the service S10 as the target product and the target service, respectively. Furthermore, for example, in a case where the target menopause state data includes the target second state data (indicating class X2) and the target first state data (indicating class W3), the server device 10A can determine the product P80 as the target product and the services S10, S30, and S40 as the target service.

Note that, for each data included in the target menopause state data, the total number of classes to be used, the total number of products and/or services assigned to each class, and the like can be optionally determined.

In another example, the server device 10A can determine at least one target product and/or at least one target service by using another estimation model generated by executing supervised learning (machine learning). The another estimation model can be generated (updated) by executing supervised learning with data indicating each class of each data included in the target menopause state data as an explanatory variable and data indicating at least one target product and/or at least one target service as an objective variable.

The machine learning for learning the another estimation model may include, for example, any known machine learning, for example, a gradient boosting tree (for example, LightGBM), a neural network, a linear regression, or the like without limitation, as described above.

By inputting the target menopause state data to the another estimation model, the server device 10A can output data ("proposal data") for identifying at least one target product and/or at least one target service from the another estimation model.

As a method for using the another estimation model, any one of the following methods can be used.

- The server device 10A acquires and holds the generated another estimation model, inputs the target menopause state data to the another estimation model, and acquires the proposal data output from the another estimation model.
- The server device 10A transmits target menopause state data via the communication network 2 to an external device (any other server device 10 or any terminal device 20) that acquires and holds the another generated estimation model, thereby causing the another estimation model having the target menopause state data input as an explanatory variable to output the proposal data. Thereafter, the server device 10A receives the proposal data output by the another estimation model from the external device via the communication network 2.

Such acquisition of the proposal data can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like.

(6) Step 1110

Referring again to Fig. 12, next, for example, the server device 10A (and/or the terminal device 20), which is the information processing device, for example, managed by the company that operates the estimation service and the like, can provide (transmit) the proposal data acquired in ST1108 to at least one terminal device 20 (which can be another server device 10) managed by the company user and/or the individual user via the communication network 2. The "individual user" as used herein may include a "target user".

The provision of the proposal data can be provided to at least one terminal device 20 (which may be another server device 10) managed by the company user and/or the individual user by any means including a web page, an e-mail, a chat, an electronic file, a recording medium (USB memory, CD-ROM, or the like), and/or a paper medium.

Such provision of the proposal data can be executed by another server device 10 (and/or terminal device 20) managed by another company, instead of the server device 10A (and/or terminal device 20) managed by the company that operates the estimation service or the like, or together with the server device 10A (and/or terminal device 20) managed by the company that operates the estimation service or the like.

7. Modification

In the various examples described above, the case where the estimation model is generated and used using the sample comprehensive state data, the sample first state data, and the sample second state data as the objective variable has been described. In another example, it is also possible to generate and use the estimation model using at least one (up to two) of the sample comprehensive state data, the sample first state data, and the sample second state data as an explanatory variable rather than an objective variable.

Furthermore, in the various examples described above, a case where the execution subject of the various steps (for example, ST1100 to ST1110) illustrated in Fig. 12 is mainly the server device 10A (and/or the terminal device 20) managed by the company that operates the estimation service and the like has been described. However, it is also possible to cause a plurality of optional server devices 10 and/or a plurality of optional terminal devices 20 to share and execute various steps illustrated in Fig. 12. Here, the plurality of optional server devices 10 and/or the plurality of optional terminal devices 20 may include, in one example, the server device 10A (and/or the terminal device 20) managed by the company that operates the estimation service and the like, and in another example, the server device 10A (and/or the terminal device 20) managed by the company that operates the estimation service and the like is not included.

As described above, according to the technology disclosed in the present application, the target behavior data for identifying the history of at least one behavior executed by the target user is input to the estimation model, whereby the target menopause state data for identifying the current (predicted) menopause symptom state of the target user can be acquired from the estimation model. Furthermore, among the plurality of target products and/or the plurality of target services prepared in advance, at least one target product and/or at least one target service suitable for the target menopause state data acquired in this manner can be proposed to any user including the target user in order to improve the menopause symptom of the target user.

As a result, it is possible to provide a computer program, an information processing device, and a method that are used to estimate the menopause symptom state of the target user based on the history of at least one behavior executed by the target user, and have at least partially improved performance.

8. Regarding estimation models for predicting various health states

The technology disclosed in the present application is applicable not only to prediction of the sleep state and the menopause symptom state described above but also to prediction of various health states exemplified below.

- Exercise state
- Immune state
- Hydration state
- Nutritional state

(1) Regarding exercise state

An estimation model for predicting an exercise state is used for predicting a state related to a lifestyle disease or the like. In order to generate such an estimation model, the sample behavior data as an explanatory variable may be at least one of a plurality of pieces of data exemplified below.

- Data for identifying a product (or a category to which the product belongs) purchased by a sample user in a certain period (for example, one year or the like)
- Data for identifying a service (or a category to which the service belongs) used by a sample user in a certain period (for example, one year or the like)
- Data for identifying whether or not a sample user is in an obese state
- Data for identifying an amount of change in the body weight of a sample user in a certain period (for example, a period from 30's to the present)
- Data for identifying an amount of exercise (for example, the number of steps, or the like) of a sample user

In addition, the data for identifying the symptom that is the objective variable can be at least one of a plurality of pieces of data (which may be measured values) exemplified below.

- Data for identifying a body weight of a sample user
- Data for identifying BMI (Body Mass Index) of a sample user
- Data for identifying the number of steps of a sample user
- Data for identifying whether a sample user smokes
- Data for identifying a metabolic syndrome diagnostic reference value of a sample user

The metabolic syndrome diagnostic reference value includes the visceral fat accumulation and/or the waist circumference length of the sample user, and at least two values among a plurality of values exemplified below.

- Threshold established for diagnosis of hyperglyceridemia and/or hypo-HDL cholesterolemia for a sample user
- Systolic and/or diastolic blood pressure of a sample user
- Threshold established for the diagnosis of fasting hyperglycemia for a sample user

The information processing device can generate an estimation model that predicts an exercise state by executing machine learning using such an explanatory variable and an objective variable. The operation executed by the communication system 2 in relation to the method for generating and using such an estimation model can be substantially the same as the operation described in the above "6" and "7" in relation to the mode of predicting the menopause symptom state. In the description given in the above "6" and "7" (particularly referring to Figs. 12 and 14), the operation executed by the communication system 2 in relation to the method for generating and using the estimation model for predicting the exercise state will be understood by a person skilled in the art by replacing the explanatory variable and the objective variable used in the estimation model for predicting the menopause symptom state with the explanatory variable and the objective variable described in the present "8(1)".

As described above, according to the technology disclosed in the present application, the target behavior data for identifying the history of at least one behavior executed by the target user is input to the estimation model, whereby the target state data for identifying the current (predicted) exercise state of the target user can be acquired from the estimation model. Furthermore, among the plurality of target products and/or the plurality of target services prepared in advance, at least one target product and/or at least one target service suitable for the target state data acquired in this manner can be proposed to any user including the target user in order to improve the exercise state of the target user.

As a result, it is possible to provide a computer program, an information processing device, and a method that are used to estimate the exercise state of the target user based on the history of at least one behavior executed by the target user, and have at least partially improved performance.

(2) Regarding immune state

An estimation model for predicting an immune state is used to predict the degree of symptoms of a person whose immunity of the body is reduced. In order to generate such an estimation model, the sample behavior data as an explanatory variable may be at least one of a plurality of pieces of data exemplified below.

- Data for identifying a product (or a category to which the product belongs) purchased by a sample user in a certain period (for example, one year or the like)
- Data for identifying a service (or a category to which the service belongs) used by a sample user in a certain period (for example, one year or the like)

In addition, the data for identifying the symptom that is the objective variable can be at least one of a plurality of pieces of data (which may be measured values) exemplified below.

- Data for identifying susceptibility of a sample user to cold
- Data for identifying a frequency of causing cold of a sample user
- Data for identifying SIgA concentration of a sample user
- Data for identifying allergic symptoms of a sample user
- Data for identifying oral environment and/or a state of immunity of a sample user
- Data for identifying a sleep state of a sample user
- Data for identifying an exercise state of a sample user
- Data for identifying a stress state of a sample user

Note that the data for identifying such symptoms may be subjective symptoms of the sample user acquired from a questionnaire or the like, or may be acquired from a reception history of the sample user in a medical institution. The data for identifying such symptoms may identify any of a plurality of (preferably three or more) categories.

The information processing device can generate an estimation model that predicts an immune state by executing machine learning using such an explanatory variable and an objective variable. The operation executed by the communication system 2 in relation to the method for generating and using such an estimation model can be substantially the same as the operation described in the above "6" and "7" in relation to the mode of predicting the menopause symptom state. In the description given in the above "6" and "7" (particularly referring to Figs. 12 and 14), the operation executed by the communication system 2 in relation to the method for generating and using the estimation model for predicting the immune state will be understood by a person skilled in the art by replacing the explanatory variable and the objective variable used in the estimation model for predicting the menopause symptom state with the explanatory variable and the objective variable described in the present "8(2)".

As described above, according to the technology disclosed in the present application, the target behavior data for identifying the history of at least one behavior executed by the target user is input to the estimation model, whereby the target state data for identifying the current (predicted) immune state of the target user can be acquired from the estimation model. Furthermore, among the plurality of target products and/or the plurality of target services prepared in advance, at least one target product and/or at least one target service suitable for the target state data acquired in this manner can be proposed to any user including the target user in order to improve the immune state of the target user.

As a result, it is possible to provide a computer program, an information processing device, and a method that are used to estimate the immune state of the target user based on the history of at least one behavior executed by the target user, and have at least partially improved performance.

(3) Regarding hydration state

An estimation model for predicting a hydration state is used to predict the degree of symptoms of a person whose immunity of the body is reduced. In order to generate such an estimation model, the sample behavior data as an explanatory variable may be at least one of a plurality of pieces of data exemplified below.

- Data for identifying a product (or a category to which the product belongs) purchased by a sample user in a certain period (for example, one year or the like)
- Data for identifying a service (or a category to which the service belongs) used by a sample user in a certain period (for example, one year or the like)

In addition, the data for identifying the symptom that is the objective variable can be at least one of a plurality of pieces of data (which may be measured values) exemplified below.

- Data for identifying a serum Na value of a sample user
- Data for identifying a urine osmotic pressure of a sample user
- Data for identifying urine specific gravity of a sample user
- Data for identifying a urine color of a sample user
- Data for identifying a BUN (urea nitrogen)/creatinine ratio of a sample user
- Data for identifying a dehydration evaluation scale of a sample user
- Data for identifying a subjective symptom associated with moisture deficiency of a sample user

These data can be generated from the clinical test values obtained by the medical examination or the complete medical checkup received by the sample user, or from the answer content of the sample user to the health questionnaire. Note that the data for identifying the subjective symptom associated with the moisture deficiency of the sample user can be generated from the answer content of the sample user to the questionnaire. The degree of the subjective symptom can be grasped from a serum Na value, a urine osmotic pressure, urine specific gravity, urine color, a BUN/creatinine ratio, an amount of water consumed, and/or an amount of physical activity, and the like.

The information processing device can generate an estimation model that predicts a hydration state by executing machine learning using such an explanatory variable and an objective variable. The operation executed by the communication system 2 in relation to the method for generating and using such an estimation model can be substantially the same as the operation described in the above "6" and "7" in relation to the mode of predicting the menopause symptom state. In the description given in the above "6" and "7" (particularly referring to Figs. 12 and 14), the operation executed by the communication system 2 in relation to the method for generating and using the estimation model for predicting the hydration state will be understood by a person skilled in the art by replacing the explanatory variable and the objective variable used in the estimation model for predicting the menopause symptom state with the explanatory variable and the objective variable described in the present "8(3)".

As described above, according to the technology disclosed in the present application, the target behavior data for identifying the history of at least one behavior executed by the target user is input to the estimation model, whereby the target state data for identifying the current (predicted) hydration state of the target user can be acquired from the estimation model. Furthermore, among the plurality of target products and/or the plurality of target services prepared in advance, at least one target product and/or at least one target service suitable for the target state data acquired in this manner can be proposed to any user including the target user in order to improve the hydration state of the target user.

As a result, it is possible to provide a computer program, an information processing device, and a method that are used to estimate the hydration state of the target user based on the history of at least one behavior executed by the target user, and have at least partially improved performance.

(4) Regarding nutritional state

An estimation model for predicting a nutritional state is used to predict the degree of symptoms in undernourished individuals. In order to generate such an estimation model, the sample behavior data as an explanatory variable may be at least one of a plurality of pieces of data exemplified below.

- Data for identifying a product (or a category to which the product belongs) purchased by a sample user in a certain period (for example, one year or the like)
- Data for identifying a service (or a category to which the service belongs) used by a sample user in a certain period (for example, one year or the like)

In addition, the data for identifying the symptom that is the objective variable can be at least one of a plurality of pieces of data (which may be measured values) exemplified below.

- Data for identifying the presence or absence of subjective symptoms associated with nutritional deficiencies of a sample user
- Data for identifying a food intake diversity score (DVS) of a sample user

# EP 4 498 376 A1

- Data for identifying a simplified nutritional appetite questionnaire (SNAQ) of a sample user
- Data for identifying BMI (Body Mass Index) of a sample user

Note that the data for identifying such symptoms may be subjective symptoms of the sample user acquired from a questionnaire or the like, or may be acquired from a reception history of the sample user in a medical institution. The data for identifying such symptoms may identify any of a plurality of (preferably three or more) categories.

The information processing device can generate an estimation model that predicts a nutritional state by executing machine learning using such an explanatory variable and an objective variable. The operation executed by the communication system 2 in relation to the method for generating and using such an estimation model can be substantially the same as the operation described in the above "6" and "7" in relation to the mode of predicting the menopause symptom state. In the description given in the above "6" and "7" (particularly referring to Figs. 12 and 14), the operation executed by the communication system 2 in relation to the method for generating and using the estimation model for predicting the nutritional state will be understood by a person skilled in the art by replacing the explanatory variable and the objective variable used in the estimation model for predicting the menopause symptom state with the explanatory variable and the objective variable described in the present "8(4)".

As described above, according to the technology disclosed in the present application, the target behavior data for identifying the history of at least one behavior executed by the target user is input to the estimation model, whereby the target state data for identifying the current (predicted) nutritional state of the target user can be acquired from the estimation model. Furthermore, among the plurality of target products and/or the plurality of target services prepared in advance, at least one target product and/or at least one target service suitable for the target state data acquired in this manner can be proposed to any user including the target user in order to improve the nutritional state of the target user.

As a result, it is possible to provide a computer program, an information processing device, and a method that are used to estimate the nutritional state of the target user based on the history of at least one behavior executed by the target user, and have at least partially improved performance.

9. Various aspects

According to a first aspect, a computer program can "cause, by being executed by at least one processor, the at least one processor to function to: acquire target behavior data for identifying a history of behavior executed by a target user; and output, from an estimation model generated by executing supervised learning, target sleep state data for identifying a sleep state of the target user, the target sleep state data including at least one of target property data for identifying a sleep property of the target user, target rhythm data for identifying a sleep rhythm of the target user, target time data for identifying a sleep time of the target user, and target category data for identifying a sleep category to which the target user belongs, by inputting the target behavior data to the estimation model".

According to a second aspect, the computer program can "cause the at least one processor to function to: acquire the estimation model generated by inputting a plurality of sets of teacher data, each set of teacher data including sample behavior data for identifying a history of behaviors executed by one sample user corresponding to the set and sample sleep state data for identifying a sleep state of one sample user corresponding to the set, to a learning model" in the first aspect.

According to a third aspect, the computer program can "cause the at least one processor to function to: be connected, via a communication line, to the estimation model generated by inputting the plurality of sets of teacher data, each set of teacher data including sample behavior data for identifying a history of behaviors executed by one sample user corresponding to the set and sample sleep state data for identifying a sleep state of one sample user corresponding to the set, to a learning model" in the first aspect.

According to a fourth aspect, the computer program can "cause the at least one processor to function to: acquire a plurality of sets of teacher data, each set of teacher data including sample behavior data for identifying a history of behaviors executed by one sample user corresponding to the set and sample sleep state data for identifying a sleep state of one sample user corresponding to the set, and generate the estimation model by inputting the plurality of sets of teacher data to a learning model for learning" in the first aspect.

According to a fifth aspect, the computer program can "cause the at least one processor to function such that the acquired plurality of sets of teacher data include a first plurality of sets of teacher data, each set of teacher data included in the first plurality of sets of teacher data includes at least sample property data for identifying the sleep property of one sample user corresponding to the set and sample rhythm data for identifying the sleep rhythm of one sample user corresponding to the set as the sample sleep state data, regarding the each set of teacher data included in the acquired first plurality of sets of teacher data, preprocessing of generating sample sleep category data for identifying a sleep category to which one sample user corresponding to the set belongs is executed based on the sample property data for identifying the sleep property of one sample user corresponding to the set and the sample rhythm data for identifying the sleep rhythm of one sample user corresponding to the set, the first plurality of sets of teacher data after the preprocessing, each set of the teacher data including the sample behavior data of one sample user corresponding to the set and the sample sleep category data of one sample user corresponding to the set, is input to the learning model to generate the estimation model, and as the target sleep state data, target sleep category data for identifying sleep category to which the target user belongs is output from the estimation model" in the fourth aspect.

According to a sixth aspect, the computer program can "cause the at least one processor to function such that the plurality of sets of teacher data include a second plurality of sets of teacher data, each set of teacher data included in the second plurality of sets of teacher data includes, as the sample sleep state data, sample sleep category data for identifying a sleep category to which one sample user corresponding to the set belongs, the sample sleep category data being determined based on at least the sample property data for identifying a sleep property of one sample user corresponding to the set and the sample rhythm data for identifying sleep rhythm of one sample user corresponding to the set, and as the target sleep state data, target sleep category data for identifying a sleep category to which the target user belongs is output from the estimation model" in any one of the second to fourth aspects.

According to a seventh aspect, in the computer program, "the sample property data for identifying a sleep property of one sample user corresponding to the set indicates a score calculated based on an answer content by the sample user to the Athens Insomnia Scale, the Pittsburgh Sleep Quality Index, the three-dimensional sleep scale, or the insomnia severity index, the sample rhythm data for identifying a sleep rhythm of one sample user corresponding to the set indicates a social jet lag that is an absolute value of a difference between a median time of a sleep time zone of a weekday and a median time of a sleep time zone of a holiday of the sample user, and the sleep category to which one sample user corresponding to the set belongs may be at least any one of a plurality of categories divided by at least one threshold arranged in association with the score on one of a horizontal axis and a vertical axis and at least one threshold arranged in association with the social jet lug on the other one of the horizontal axis and the vertical axis" in the fifth aspect or the sixth aspect.

According to an eighth aspect, the computer program can "cause the at least one processor to function such that the plurality of sets of teacher data include a third plurality of sets of teacher data, each set of teacher data included in the third plurality of sets of teacher data includes, as the sample sleep state data, at least the sample property data for identifying a sleep property of one sample user corresponding to the set and the sample rhythm data for identifying sleep rhythm of one sample user corresponding to the set, and as the target sleep state data, target property data for identifying a sleep property of the target user and target rhythm data for identifying a sleep rhythm of the target user are output from the estimation model" in any one of the second to fourth aspects.

According to a ninth aspect, the computer program can "cause the at least one processor to function such that the plurality of sets of teacher data include a fourth plurality of sets of teacher data, each set of teacher data included in the fourth plurality of sets of teacher data includes, as the sample sleep state data, at least the sample property data for identifying a sleep property of one sample user corresponding to the set, and as the target sleep state data, target property data for identifying a sleep property of the target user is output from the estimation model" in any one of the second to fourth aspects.

According to a tenth aspect, the computer program can "cause the at least one processor to function such that the plurality of sets of teacher data include a fifth plurality of sets of teacher data, each set of teacher data included in the fifth plurality of sets of teacher data includes, as the sample sleep state data, at least the sample rhythm data for identifying the sleep rhythm of one sample user corresponding to the set, and as the target sleep state data, target rhythm data for identifying a sleep rhythm of the target user is output from the estimation model" in any one of the second to fourth aspects.

According to an 11th aspect, the computer program can "cause the at least one processor to function such that the plurality of sets of teacher data include a sixth plurality of sets of teacher data, each set of teacher data included in the sixth plurality of sets of teacher data includes, as the sample sleep state data, at least the sample time data for identifying a sleep time of one sample user corresponding to the set, and as the target sleep state data, target time data for identifying a sleep time of the target user is output from the estimation model" in any one of the second to fourth aspects.

According to a 12th aspect, the computer program can "cause the at least one processor to function to determine at least one target product and/or at least one target service corresponding to the target sleep state data among a plurality of products and/or a plurality of services" in any one of the first to 11th aspects.

According to a 13th aspect, the computer program can "cause the at least one processor to function such that proposal data for identifying the at least one target product and/or the at least one target service is output from another estimation model by inputting the target sleep state data into the another estimation model generated by executing supervised learning" in the 12th aspect.

According to a 14th aspect, the computer program can "cause the at least one processor to function such that proposal data for identifying the at least one target product and/or the at least one target service is acquired from a search table by inputting the target sleep state data to the search table in which the target sleep state data is associated with the plurality of products and/or the plurality of services" in the 12th aspect.

According to a 15th aspect, in the computer program, "at least one processor can include a central processing unit (CPU), a microprocessor, and/or a graphics processing unit (GPU)" in any one of the first to 14th aspects.

According to 16th aspect, an information processing device may include "at least one processor, in which the at least one processor is configured to: acquire target behavior data for identifying a history of behavior executed by a target user; and output, from an estimation model generated by executing supervised learning, target sleep state data for identifying a sleep state of the target user, the target sleep state data including at least one of target property data for identifying a sleep property of the target user, target rhythm data for identifying a sleep rhythm of the target user, target time data for identifying a sleep time of the target user, and target category data for identifying a sleep category to which the target user belongs, by inputting the target behavior data to the estimation model".

According to a 17th aspect, the information processing device may be "a terminal device or a server device" in the 16th aspect.

According to an 18th aspect, in the information processing device, "the at least one processor can include a central processing unit (CPU), a microprocessor, and/or a graphics processing unit (GPU)" in the 17th aspect.

According to a 19th aspect, a method is executed by at least one processor that executes a computer readable instruction, the method including: by the at least one processor executing the instruction, a step of acquiring target behavior data for identifying a history of behavior executed by a target user; and a step of outputting, from an estimation model generated by executing supervised learning, target sleep state data for identifying a sleep state of the target user, the target sleep state data including at least one of target property data for identifying a sleep property of the target user, target rhythm data for identifying a sleep rhythm of the target user, target time data for identifying a sleep time of the target user, and target category data for identifying a sleep category to which the target user belongs, by inputting the target behavior data to the estimation model".

According to a 20th aspect, in the method, "the at least one processor can include a central processing unit (CPU), a microprocessor, and/or a graphics processing unit (GPU)" in the 19th aspect.

According to a 21st aspect, a method is "executed by at least one processor that executes a computer readable instruction, the method including: by the at least one processor executing the instruction, an acquisition step of acquiring a plurality of sets of teacher data, each set of teacher data including sample behavior data for identifying a history of behaviors executed by one sample user corresponding to the set and sample sleep state data for identifying a sleep state of one sample user corresponding to the set; a generation step of generating an estimation model configured to output target sleep state data for identifying a sleep state of the target user, the target sleep state data including at least one of target property data for identifying a sleep property of the target user, target rhythm data for identifying a sleep rhythm of the target user, target time data for identifying a sleep time of the target user, and target category data for identifying a sleep category to which the target user belongs, by inputting target behavior data for identifying a history of behavior executed by the target user when inputting the plurality of sets of teacher data into a learning model and learning the plurality of sets of teacher data".

According to a 22nd aspect, in the method, "the acquisition step can include a step of acquiring a first plurality of sets of teacher data included in the plurality of sets of teacher data, each set of teacher data included in the first plurality of sets of teacher data includes at least sample property data for identifying the sleep property of one sample user corresponding to the set and sample rhythm data for identifying the sleep rhythm of one sample user corresponding to the set as the sample sleep state data, and the generation step can include a step of, regarding the each set of teacher data included in the acquired first plurality of sets of teacher data, executing preprocessing of generating sample sleep category data for identifying a sleep category to which one sample user corresponding to the set belongs based on the sample property data for identifying the sleep property of one sample user corresponding to the set and the sample rhythm data for identifying the sleep rhythm of one sample user corresponding to the set, and a step of generating the estimation model by inputting the first plurality of sets of teacher data after preprocessing, each set of teacher data including the sample behavior data of one sample user corresponding to the set and the sample sleep category data of one sample user corresponding to the set, to the learning model" in the 21st aspect.

According to a 23rd aspect, in the method, "the acquisition step can include a step of acquiring a second plurality of sets of teacher data included in the plurality of sets of teacher data, and each set of teacher data included in the second plurality of sets of teacher data includes, as the sample sleep state data, sample sleep category data for identifying a sleep category to which one sample user corresponding to the set belongs, the sample sleep category data being determined based on at least the sample property data for identifying a sleep property of one sample user corresponding to the set and the sample rhythm data for identifying sleep rhythm of one sample user corresponding to the set, and a step of generating the estimation model by inputting the second plurality of sets of teacher data to the learning model" in the 21st aspect.

According to a 24th aspect, in the method, "the sample property data for identifying a sleep property of one sample user corresponding to the set indicates a score calculated based on an answer content by the sample user to the Athens Insomnia Scale, the Pittsburgh Sleep Quality Index, the three-dimensional sleep scale, or the insomnia severity index, the sample rhythm data for identifying a sleep rhythm of one sample user corresponding to the set indicates a social jet lag that is an absolute value of a difference between a median time of a sleep time zone of a weekday and a median time of a sleep time zone of a holiday of the sample user, and the sleep category to which one sample user corresponding to the set belongs may be at least any one of a plurality of categories divided by at least one threshold arranged in association with the score on one of a horizontal axis and a vertical axis and at least one threshold arranged in association with the social jet lag on the other one of the horizontal axis and the vertical axis" in the 23rd aspect.

According to a 25th aspect, the method, "can further include an estimation step of outputting target sleep state data for identifying a sleep state of the target user from the estimation model by inputting the target behavior data for identifying a history of behavior executed by the target user to the estimation model" in any one of the 21st to 24th aspects.

According to a 26th aspect, in the method, "the at least one processor can include a central processing unit (CPU), a microprocessor, and/or a graphics processing unit (GPU)" in any one of the 21st to 25th aspect.

According to a 27th aspect, a computer program can "cause, by being executed by at least one processor, the at least one processor to function to: acquire target behavior data for identifying a history of behavior executed by a target user; and output, from an estimation model generated by executing supervised learning, target menopause state data for identifying a menopause symptom state of the target user, the target menopause state data including at least one of target comprehensive state data for identifying a comprehensive state of a menopause symptom of the target user, target first state data for identifying a first symptom state of the target user, and target second state data for identifying a second symptom state of the target user, by inputting the target behavior data to the estimation model".

According to a 28th aspect, the computer program can "cause the at least one processor to function to: acquire the estimation model generated by inputting a plurality of sets of teacher data, each set of teacher data including sample behavior data for identifying a history of behaviors executed by one sample user corresponding to the set and sample menopause state data for identifying a menopause symptom state of one sample user corresponding to the set, to a learning model" in the 27th aspect.

According to a 29th aspect, the computer program can "cause the at least one processor to function to: be connected, via a communication line, to the estimation model generated by inputting a plurality of sets of teacher data, each set of teacher data including sample behavior data for identifying a history of behaviors executed by one sample user corresponding to the set and sample menopause state data for identifying a menopause symptom state of one sample user corresponding to the set, to a learning model" in the 27th aspect.

According to a 30th aspect, the computer program can "cause the at least one processor to function to: acquire a plurality of sets of teacher data, each set of teacher data including sample behavior data for identifying a history of behaviors executed by one sample user corresponding to the set and sample menopause state data for identifying a menopause symptom state of one sample user corresponding to the set, and generate the estimation model by inputting the plurality of sets of teacher data to a learning model for learning" in the 27th aspect.

According to a 31st aspect, the computer program can "cause the at least one processor to function such that the acquired plurality of sets of teacher data include a first plurality of sets of teacher data, each set of teacher data included in the first plurality of sets of teacher data includes, as the sample menopause state data, at least the sample comprehensive state data for identifying a comprehensive state of the menopause symptom of one sample user corresponding to the set, and as the target menopause state data, target comprehensive state data for identifying a comprehensive state of the menopause symptom of the target user is output from the estimation model" in the 30th aspect.

According to a 32nd aspect, the computer program can "cause the at least one processor to function such that each set of teacher data included in the first plurality of sets of teacher data includes, as the sample menopause state data, at least sample comprehensive state data for identifying a score corresponding to the comprehensive state of the menopause symptom of one sample user corresponding to the set, for each set of teacher data included in the first plurality of sets of teacher data, preprocessing of converting sample comprehensive state data for identifying a score corresponding to the comprehensive state of the menopause symptom of one sample user corresponding to the set into sample comprehensive state data for identifying a category corresponding to the comprehensive state of the menopause symptom of one sample user corresponding to the set is executed, and the estimation model is generated by inputting the first plurality of sets of teacher data after the preprocessing into the learning model to output target comprehensive state data for identifying a category corresponding to a comprehensive state of a menopause symptom of the target user, as the target menopause state data" in the 31st aspect.

According to a 33rd aspect, in the computer program, "the score corresponding to the comprehensive state of the menopause symptom of the one sample user corresponding to the set may be a score calculated based on the answer content of the one sample user corresponding to the set with respect to a simplified menopause index (SMI), a Menopause Rating Scale, a Kupperman index, a Greene Climacteric Scale, a PSST (Premenstrual Symptoms Screening Tool), a WHQ (Women's Health Questionnaire), a VAS (Visual Analogue Scale), a HFRDI (Hot Flash Related Daily Interference Scale), a HFCS (Hot Flash Composite Score), and a MENQOL (Menopause-Specific Quality Of Life), or a menopause symptoms assessment table for Japanese women" in the 32nd aspect.

According to a 34th aspect, the computer program can "cause the at least one processor to function such that the acquired plurality of sets of teacher data include a second plurality of sets of teacher data, each set of teacher data included in the second plurality of sets of teacher data includes, as the sample menopause state data, at least the sample first state data for identifying a first symptom state of one sample user corresponding to the set, and as the target menopause state data, target first state data for identifying a first symptom state of the target user is output from the estimation model" in the 30th aspect.

According to a 35th aspect, the computer program can "cause the at least one processor to function such that each set of teacher data included in the second plurality of sets of teacher data includes, as the sample menopause state data, at least sample first state data for identifying a score corresponding to the first symptom state of one sample user corresponding to the set, for each set of teacher data included in the second plurality of sets of teacher data, preprocessing of converting sample first state data for identifying a score corresponding to the first symptom state of one sample user corresponding to the set into sample first state data for identifying a category corresponding to the first symptom state of one sample user corresponding to the set is executed, and the estimation model is generated by inputting the second plurality of sets of teacher data after the preprocessing into the learning model to output target first state data for identifying a category corresponding to the first symptom state of the target user from the estimation model, as the target menopause state data" in the 34th aspect.

According to a 36th aspect, the computer program can "cause the at least one processor to function such that the acquired plurality of sets of teacher data include a third plurality of sets of teacher data, each set of teacher data included in the third plurality of sets of teacher data includes, as the sample menopause state data, at least the sample second state data for identifying a second symptom state of one sample user corresponding to the set, and as the target menopause state data, target second state data for identifying a second symptom state of the target user is output from the estimation model" in the 30th aspect.

According to a 37th aspect, the computer program can "cause the at least one processor to function such that each set of teacher data included in the third plurality of sets of teacher data includes, as the sample menopause state data, at least sample second state data for identifying a score corresponding to the second symptom state of one sample user corresponding to the set, for each set of teacher data included in the third plurality of sets of teacher data, preprocessing of converting the sample second state data for identifying a score corresponding to the second symptom state of one sample user corresponding to the set into sample second state data for identifying a category corresponding to the second symptom state of one sample user corresponding to the set is executed, and the estimation model is generated by inputting the third plurality of sets of teacher data after the preprocessing into the learning model to output target second state data for identifying a category corresponding to a second symptom state of the target user from the estimation model, as the target menopause state data" in the 36th aspect.

According to a 38th aspect, the computer program can "cause the at least one processor to function to determine at least one target product and/or at least one target service corresponding to the target menopause state data among a plurality of products and/or a plurality of services" in any one of the 27th to 37th aspects.

According to a 39th aspect, the computer program can "cause the at least one processor to function such that proposal data for identifying the at least one target product and/or the at least one target service is output from another estimation model by inputting the target menopause state data into the another estimation model generated by executing supervised learning" in the 38th aspect.

According to a 40th aspect, the computer program can "cause the at least one processor to function such that proposal data for identifying the at least one target product and/or the at least one target service is acquired from a search table by inputting the target menopause state data to the search table in which the target menopause state data is associated with the plurality of products and/or the plurality of services" in the 38th aspect.

According to a 41st aspect, in the computer program, "the at least one processor can include a central processing unit (CPU), a microprocessor, and/or a graphics processing unit (GPU)" in any one of the 27th to 40th aspects.

According to a 42nd aspect, an information processing device may "include at least one processor, in which the at least one processor acquires target behavior data for identifying a history of behavior executed by a target user; and outputs, from an estimation model generated by executing supervised learning, target menopause state data for identifying a menopause symptom state of the target user, the target menopause state data including at least one of target comprehensive state data for identifying a comprehensive state of a menopause symptom of the target user, target first state data for identifying a first symptom state of the target user, and target second state data for identifying a second symptom state of the target user, by inputting the target behavior data to the estimation model".

According to a 43rd aspect, the information processing device may be "a terminal device or a server device" in the 42nd aspect.

According to a 44th aspect, in the information processing device, "the at least one processor can include a central processing unit (CPU), a microprocessor, and/or a graphics processing unit (GPU)" in the 42nd or 43rd aspect.

According to a 45th aspect, a method is "executed by at least one processor that executes a computer readable instruction, the method including: by the at least one processor executing the instruction, a step of acquiring target behavior data for identifying a history of behavior executed by a target user; and a step of outputting, from an estimation model generated by executing supervised learning, target menopause state data for identifying a menopause symptom state of the target user, the target menopause state data including at least one of target comprehensive state data for identifying a comprehensive state of a menopause symptom of the target user, target first state data for identifying a first symptom state of the target user, and target second state data for identifying a second symptom state of the target user, by inputting the target behavior data to the estimation model".

According to a 46th aspect, in the method, "the at least one processor can include a central processing unit (CPU), a microprocessor, and/or a graphics processing unit (GPU)" in the 45th aspect.

According to a 47th aspect, a method is executed by at least one processor that executes a computer readable instruction, the method including: by the at least one processor executing the instruction, an acquisition step of acquiring a plurality of sets of teacher data, each set of teacher data including sample behavior data for identifying a history of behaviors executed by one sample user corresponding to the set and sample menopause state data for identifying a menopause symptom state of one sample user corresponding to the set; a generation step of generating an estimation model configured to output target menopause state data for identifying a menopause symptom state of the target user, the target menopause state data including at least one of target comprehensive state data for identifying a comprehensive state of a menopause symptom of the target user, target first state data for identifying a first symptom state of the target user, and target second state data for identifying a second symptom state of the target user by inputting the target behavior data for identifying a history of behavior executed by the target user when inputting the plurality of sets of teacher data into a learning model and learning the plurality of sets of teacher data".

According to a 48th aspect, in the method, "the acquisition step can include a step of acquiring a first plurality of sets of teacher data included in the plurality of sets of teacher data, each set of teacher data included in the first plurality of sets of teacher data includes at least sample comprehensive state data for identifying a score corresponding to a comprehensive state of a menopause symptom of one sample user corresponding to the set as the sample menopause state data, and the generation step can include a step of, regarding the each set of teacher data included in the acquired first plurality of sets of teacher data, executing preprocessing of converting the sample comprehensive state data for identifying a score corresponding to the comprehensive state of the menopause symptom of one sample user corresponding to the set into sample comprehensive state data for identifying a category corresponding to the comprehensive state of the menopause symptom of one sample user corresponding to the set, and a step of generating the estimation model by inputting the first plurality of sets of teacher data after preprocessing, each set of teacher data including the sample behavior data of one sample user corresponding to the set and the sample comprehensive state data of one sample user corresponding to the set, to the learning model" in the 47th aspect.

According to a 49th aspect, in the method, "the acquisition step can include a step of acquiring a second plurality of sets of teacher data included in the plurality of sets of teacher data, each set of teacher data included in the second plurality of sets of teacher data includes at least sample first state data for identifying a score corresponding to a first symptom state of one sample user corresponding to the set as the sample menopause state data, and the generation step can include a step of, regarding the each set of teacher data included in the acquired second plurality of sets of teacher data, executing preprocessing of converting the sample first state data for identifying a score corresponding to a first symptom state of one sample user corresponding to the set into sample first state data for identifying a category corresponding to the first symptom state of one sample user corresponding to the set, and a step of generating the estimation model by inputting the second plurality of sets of teacher data after preprocessing, each set of teacher data including the sample behavior data of one sample user corresponding to the set and the sample first state data of one sample user corresponding to the set, to the learning model" in the 47th aspect.

According to a 50th aspect, in the method, "the acquisition step can include a step of acquiring a third plurality of sets of teacher data included in the plurality of sets of teacher data, each set of teacher data included in the third plurality of sets of teacher data includes at least sample second state data for identifying a score corresponding to a second symptom state of one sample user corresponding to the set as the sample menopause state data, and the generation step can include a step of, regarding the each set of teacher data included in the acquired third plurality of sets of teacher data, executing preprocessing of converting the sample second state data for identifying a score corresponding to a second symptom state of one sample user corresponding to the set into sample second state data for identifying a category corresponding to the second symptom state of one sample user corresponding to the set, and a step of generating the estimation model by inputting the third plurality of sets of teacher data after preprocessing, each set of teacher data including the sample behavior data of one sample user corresponding to the set and the sample second state data of one sample user corresponding to the set, to the learning model" in the 47th aspect.

According to a 51st aspect, in the method, "the at least one processor can include a central processing unit (CPU), a microprocessor, and/or a graphics processing unit (GPU)" in the 47th or 50th aspect.

According to a 52nd aspect, the computer program can "cause, by being executed by at least one processor, the at least one processor to function to: acquire target behavior data for identifying a history of behavior executed by a target user; and output, from an estimation model generated by executing supervised learning, state data for identifying an exercise state, a state related to a lifestyle disease, or a state of physical activity insufficiency of the target user, the state data including at least one of data for identifying a body weight of the target user, data for identifying a body mass index (BMI) of the target user, data for identifying the number of steps of the target user, data for identifying whether the target user smokes, and data for identifying a metabolic syndrome diagnostic reference value of the target user, by inputting the target behavior data to the estimation model".

According to a 53rd aspect, the computer program can "cause, by being executed by at least one processor, the at least one processor to function to: acquire target behavior data for identifying a history of behavior executed by a target user; and output, from an estimation model generated by executing supervised learning, state data for identifying a hydration state of the target user, the state data including at least one of data for identifying a serum Na value of the target user, data for identifying a urine osmotic pressure of the target user, data for identifying urine specific gravity of the target user, data for identifying a urine color of the target user, data for identifying a BUN (urea nitrogen)/creatinine ratio of the target user, data for identifying a dehydration evaluation scale of the target user, and data for identifying a subjective symptom associated with moisture deficiency of the target user, by inputting the target behavior data to the estimation model".

According to a 54th aspect, the computer program can "cause, by being executed by at least one processor, the at least one processor to function to: acquire target behavior data for identifying a history of behavior executed by a target user; and output, from an estimation model generated by executing supervised learning, state data for identifying an immune state of the target user, the state data including at least one of data for identifying susceptibility of the target user to cold, data for identifying a frequency of causing cold of the target user, data for identifying SIgA concentration of the target user, data for identifying allergic symptoms of the target user, data for identifying oral environment and/or a state of immunity of the target user, data for identifying a sleep state of the target user, data for identifying an exercise state of the target user, and data for identifying a stress state of the target user, by inputting the target behavior data to the estimation model".

According to a 55th aspect, the computer program can "cause, by being executed by at least one processor, the at least one processor to function to: acquire target behavior data for identifying a history of behavior executed by a target user; and output, from an estimation model generated by executing supervised learning, state data for identifying a nutritional state of the target user, the state data including at least one of data for identifying the presence or absence of subjective symptoms associated with nutritional deficiencies of the target user, data for identifying a food intake diversity score (DVS) of the target user, data for identifying a Simplified Nutritional Appetite Questionnaire of the target user, and data for identifying BMI (Body Mass Index) of the target user, by inputting the target behavior data to the estimation model".

II. Chapter 2

The technology described in Chapter 1 and the technology described below in Chapter 2 can be used in combination.

For example, the estimation model for estimating the sleep state described in Chapter 1 can be used as a sleep state prediction model 110b in the technology described below. Similarly, the estimation model for estimating the menopause symptom state described in Chapter 1 can be used as a female health prediction model 100a in the technology described below. Furthermore, similarly, the estimation model for predicting the exercise state, the estimation model for predicting the immune state, the estimation model for predicting the hydration state, and the estimation model for predicting the nutritional state described in Chapter 1 can be used as a lifestyle disease prediction model 110c, an immune state prediction model 110e, a moisture state prediction model 110d, and a nutritional state prediction model 110f, respectively, in the technology described below.

Alternatively, at least one feature included in the technology described in Chapter 1 (or at least one feature included in the technology described in Chapter 2) can be used in the technology described in Chapter 2 (or the technology described in Chapter 1).

In recent years, living environments are changing significantly around the world, such as a decrease in population, an economic depression, occurrence of natural disasters, and spread of infectious diseases. On the other hand, opportunities for utilizing artificial intelligence (AI) have increased, and development and popularization of a 5th generation mobile communication system have progressed. Under the changing living environment, infrastructure that supports society starts to shift to an industrial frame based on data by utilizing technology, and social activity/behavior information starts to be visualized.

With the growing interest in health, it is also required to adapt to social movements as a response to health management. Until now, the focus has been on "passive healthcare" in which treatment is received after illness and symptoms begin to worsen. However, in the future, by acquiring data of a healthy person at home and collecting the data to convert the health state of each person into data, it is considered that "proactive medical care" for performing proactive health management and "preventive medical care" for performing morbidity prevention will also be performed. After that, "predictive medical care" for predicting a future disease and calling attention to health can also be assumed.

Patent Literature 1 discloses a method for constructing an estimation model for estimating a health state of a target user based on data describing a purchase history of the target user. It should be noted that this patent literature is incorporated herein by reference in its entirety.

There are various kinds of risks in health state, but the method disclosed in Patent Literature 1 does not consider that customers grasp various health risks.

Some aspects of the present invention have been made in view of any of the above problems, and an object thereof is to provide a program, an information processing device, and an information processing method capable of suitably providing information regarding health to a customer.

A program according to an exemplary embodiment of the present invention is a program for causing an information processing device to execute processing of acquiring first purchase data indicating a purchase history of a target customer, prediction processing of predicting degrees of a plurality of types of health symptoms from the first purchase data by using a health prediction model related to the plurality of types of health symptoms of a first type to an N-th type (N is an integer of 2 or more), and processing of providing health information indicating the predicted degrees of the plurality of types of health symptoms to the target customer, in which the health prediction model related to a k-th type ($1 \leq k \leq N$) of health symptoms calculates a numerical value indicating the degrees of the k-th type of health symptoms from the first purchase data related to a product category.

An information processing device according to an exemplary embodiment of the present invention is an information processing device including: an input unit that acquires first purchase data indicating a purchase history of a target customer; a prediction unit that predicts degrees of a plurality of types of health symptoms from the first purchase data by using a health prediction model related to the plurality of types of health symptoms of a first type to an N-th type (N is an integer of 2 or more); and an output unit that provides health information indicating the predicted degrees of the plurality of types of health symptoms to the target customer, in which the health prediction model related to a k-th type ($1 \leq k \leq N$) of health symptoms calculates a numerical value indicating the degrees of the k-th type of health symptoms from the first purchase data related to a product category.

An information processing method according to an exemplary embodiment of the present invention is an information processing method in which an information processing device performs processing of acquiring first purchase data indicating a purchase history of a target customer, processing of predicting degrees of a plurality of types of health symptoms from the first purchase data by using a health prediction model related to the plurality of types of health symptoms of a first type to an N-th type (N is an integer of 2 or more), and processing of providing health information indicating the predicted degrees of the plurality of types of health symptoms to the target customer, and the health prediction model related to a k-th type ($1 \leq k \leq N$) of health symptoms calculates a numerical value indicating the degrees of the k-th type of health symptoms from the first purchase data related to a product category.

Note that, in the present invention, "unit", "means", "device", and "system" do not simply mean physical means, and also include a case where functions of the "unit", "means", "device", and "system" are implemented by software. In addition, the functions of one "unit", "means", "device", or "system" may be implemented by two or more physical means or devices, or the functions of two or more "unit", "means", "device", or "system" may be implemented by one physical means or device.

1. Overview

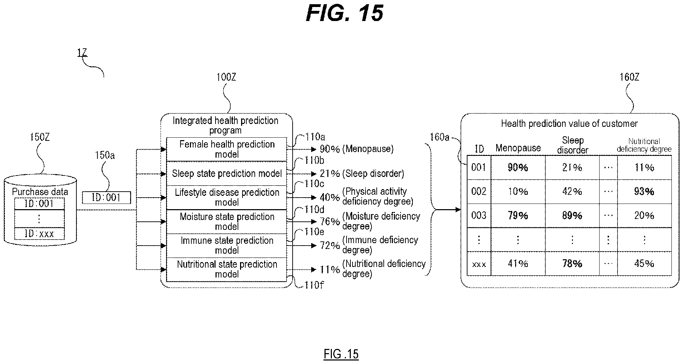

Figs. 15 to 28 are diagrams for describing embodiments. Hereinafter, an outline, a functional configuration, a flow of processing, and the like will be sequentially described while being roughly divided into a health prediction system that predicts a health state of a customer (also referred to as a health symptom) and a learning system that generates a prediction model for predicting the health state of the customer used in the health prediction system.

2 Health prediction system

2.1 Overview of processing of health prediction system

First, an outline of rough processing of a health prediction system 1Z according to the embodiment will be described with reference to Fig. 15. Fig. 15 is a diagram schematically illustrating a flow of data for performing health prediction of a customer using the health prediction system 1Z according to the embodiment.

The health prediction system 1Z is for estimating a health state of an individual from a purchase behavior of the individual. By using the health prediction system 1Z, the health state of an individual customer can be estimated based on purchase data that is a customer's purchase behavior without data regarding the health of the person himself/herself such as medical examination data. The estimated health state can be useful, for example, for improving the health of the customer.

Here, an integrated health prediction program 100Z for predicting the health state of an individual from the purchase behavior of the individual includes a plurality of types of learned prediction models for predicting the health state from the purchase behavior of the individual. In the example of Fig. 15, the integrated health prediction program 100Z is configured by integrating six prediction models, but is not limited to six, and may be five or less or seven or more.

The integrated health prediction program 100Z according to the present embodiment includes six prediction models (hereinafter, collectively referred to as a "prediction model 110Z") including a female health prediction model 110a that predicts female health, a sleep state prediction model 110b that predicts a sleep state, a lifestyle disease prediction model 110c that predicts a lifestyle disease, a moisture state prediction model 110d that predicts a moisture state, an immune state prediction model 110e that predicts an immune state, and a nutritional state prediction model 110f that predicts a nutritional state. Each prediction model included in the integrated health prediction program 100Z calculates a value (a value indicating the possibility and/or degree of suffering from a specific disease) indicating a female health state (SMI or the like related to menopause symptoms)/sleep state/lifestyle disease/moisture state (hydration status) /immune state/nutritional state from purchase data 150Z indicating a purchase history of a customer as a health prediction value 160Z of the customer. In the example of Fig. 15, values indicating six health states are indicated in %. Depending on the type of disease, only males, only females, or both males and females may be targeted. These prediction models 110Z may be described as functions. The prediction model is constructed by machine learning using teacher data including a product purchase history and various states related to health. A method for constructing the prediction model will be described later.

More specifically, the integrated health prediction program 100Z receives the purchase data 150Z of the customer. The purchase data 150Z is data related to a purchase history of a customer whose health is desired to be predicted, and is data corresponding to an explanatory variable of teacher data at the time of constructing each prediction model 110Z included in the integrated health prediction program 100Z. The integrated health prediction program 100Z inputs the purchase data 150Z to each prediction model 110Z in parallel to calculate the health prediction value 160Z of the customer. In the present embodiment, only the purchase data 150Z is used as an input to the prediction model 110Z, but for example, attribute information of the customer such as the gender and the age of the customer may also be input.

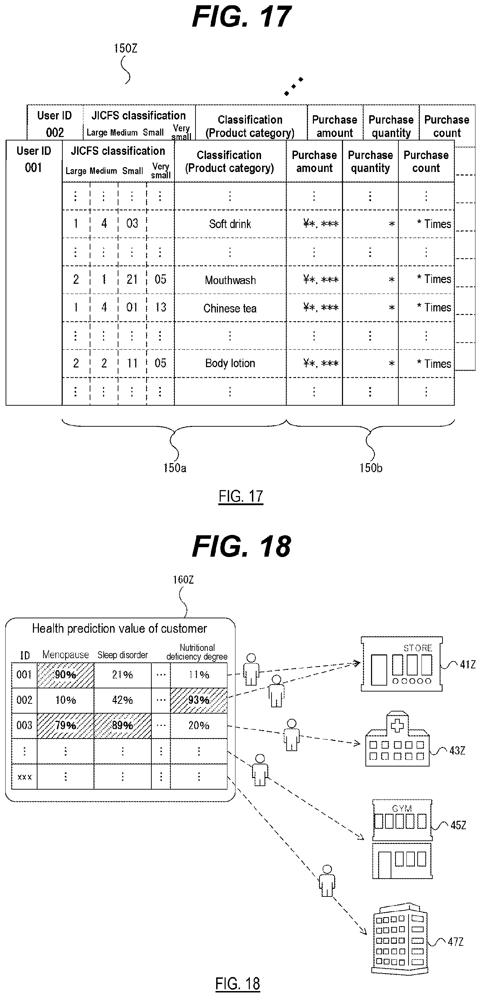

Furthermore, the integrated health prediction program 100Z may make various proposals regarding health improvement and the like to the customer according to the calculated health prediction value 160Z. More specifically, for example, it is conceivable to point out a category having a high health prediction value to the customer, and propose a measure that can be taken while the customer is not aware of (potential) symptoms or symptoms that become apparent are mild. The proposal method will be described later with reference to Fig. 18 and the like.

2.2 Configuration of integrated health prediction program

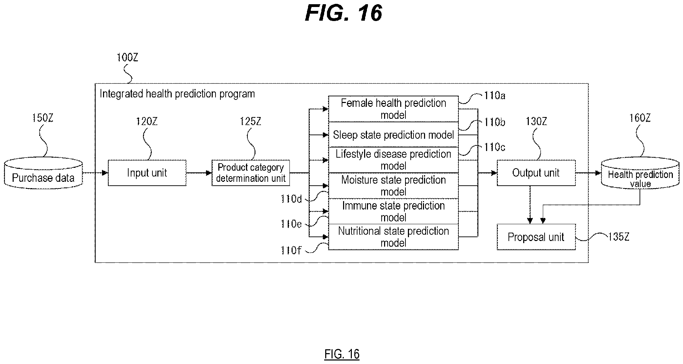

Hereinafter, a functional configuration of the integrated health prediction program 100Z will be described with reference to Fig. 16. Fig. 16 is a diagram illustrating the functional configuration of the integrated health prediction program 100Z. The integrated health prediction program 100Z includes an input unit 120Z, a product category determination unit 125Z, a prediction model 110Z, an output unit 130Z, and a proposal unit 135Z. Note that the integrated health prediction program 100Z may be implemented as a single program, or may be implemented as a plurality of programs that can operate in cooperation by input and output of data.

The input unit 120Z receives (acquires) an input of the purchase data 150Z. The input unit 120Z may read the purchase data 150Z from a storage medium such as an HDD (hard disk drive) or an SSD (solid state drive) built in an information processing device (computer) in which the integrated health prediction program 100Z is executed, or may receive an input of the purchase data 150Z from an external information processing device or the like connected via a LAN (local area network), the Internet, or the like.

Here, a specific example of the purchase data 150Z input to the input unit 120Z will be described with reference to Fig. 17. Fig. 17 is a diagram illustrating a specific example of the purchase data 150Z. Here, as the purchase data 150Z, panel data in which a purchase history of a customer provided by a research company is accumulated can be adopted. The panel data is data acquired by scanning a product by the customer who is a purchaser when the product is purchased. When the customer scans a product using his/her smartphone or a lent barcode reader, the product is accumulated in association with preset attributes of the customer (company employee/student/housewife, or the like), information on a purchase destination, and the like. This allows information to be gathered about what kind of people purchased what, how much, and at what store.

An example of the purchase data 150Z in Fig. 17 indicates a purchase history of food and/or daily necessities purchased by a customer over a certain period (for example, one year). Each product purchased by the customer is classified into a product category in a JICFS (JAN Item Code File Service) classification. In other words, at least one product belongs to each product category. In the purchase data 150Z, for each ID of the customer, each item 150a of about 500 items (examples: soft drinks, mouthwash, or the like) of the small categories included in the large category "food" and "daily necessities" in the JICFS classification related to the product category to which the product purchased by the customer belongs, and performance data 150b which is numerical data such as the purchase amount, the purchase quantity, and the purchase count during the period are input in association with each other.

In the present embodiment, what is included in the purchase data 150Z is the purchase history of food and/or daily necessities, but the present invention is not limited thereto as long as the relationship with the health state of the customer is estimated. For example, it is also conceivable that the purchase data 150Z includes purchase histories of other products such as medicines and various services such as massage and acupuncture. In addition, it is conceivable that the purchase data 150Z is specified by payment information managed by a retail store, or is specified by using, for example, payment information of a credit card, payment information to a financial institution, or the like.

Upon receiving the purchase data 150Z from the input unit 120Z, the product category determination unit 125Z determines a classification code (JICFS classification) and a product category to be input to each of the prediction models 110Z from the purchase data 150Z input to each of the prediction models 110Z, and inputs the performance data 150b (only a part thereof may be used) that is numerical data such as a purchase amount, a purchase quantity, and a purchase count regarding the obtained classification code and product category to each of the prediction models 110Z in parallel.

Note that the classification code (product category) of the purchase data 150Z input to the prediction model 110Z can be determined for each prediction model 110Z. A method of determining the product category to be an input of the prediction model 110Z will be described later in 3.4 related to creation of the prediction model 110Z.

Note that it is also conceivable to input the performance data 15-b directly to the prediction model 110Z without narrowing the purchase data 150Z input from the input unit 120Z particularly by product category. In this case, the product category determination unit 125Z is unnecessary.

The prediction model 110Z receives an input of the performance data 150b which is numerical data such as the purchase amount, the purchase quantity, and the purchase count regarding the classification code and the product category selected by the product category determination unit 125Z, and calculates health prediction values of a female health state (menopause symptom), a sleep state (sleep disorder), a lifestyle disease (physical activity deficiency), a moisture state (moisture deficiency degree/hydration status), an immune state (immune deficiency degree), and a nutritional state (nutritional deficiency degree). In the example of Fig. 15, for the customer with user ID001, each prediction model 110Z calculates values of menopause symptom 90%, sleep disorder 21%, physical activity deficiency degree 40%, moisture deficiency degree 76%, immune deficiency degree 72%, and nutritional deficiency degree 11%.

As described above, the prediction model 110Z can be constructed by machine learning using teacher data in which the purchase data 150Z that is the purchase history of each customer is associated with the health state of these customers, with the former as an explanatory variable and the latter as an objective variable. A method for constructing the prediction model 110Z will be described below in 3.

The output unit 130Z outputs the value calculated by each prediction model 110Z as the health prediction value 160Z related to the customer to the proposal unit 135Z, a built-in storage medium, another information processing device connected via a network, or the like. Alternatively, the output unit 130Z may display the calculated health prediction value 160Z on the display device. In the example of Fig. 15, the output unit 140Z outputs the health prediction value 160Z for a plurality of customers by sequentially arranging values such as the customer ID, menopause, and sleep disorder in the row direction and repeating the arrangement for each ID of the customer data. Note that the health prediction value 160Z can include not only an actualized health state of the customer but also a health state that can be expected to occur in the future (potential health state).

The proposal unit 135Z proposes food, supplies, services, and the like according to the predicted health prediction value 160Z for each customer. Various proposal methods can be considered. For example, the proposal may be displayed on various terminals used by a store clerk of a retail store, a proposal content may be directly sent to a customer by messenger service or the like, or the proposal content may be displayed on a web page for the customer.

2.3 Specific examples and processing of various proposals to customers

Hereinafter, various proposals made by the integrated health prediction program 100Z to customers will be described with reference to Figs. 18 to 20.

Fig. 18 is a diagram schematically illustrating food, supplies, services, and the like proposed by the proposal unit 135Z according to the health prediction value 160Z of the customer. For example, the proposal unit 135Z proposes a product for improving the menopause symptom sold in a drug store 41Z to a woman (customer ID = 001) whose menopause symptom is predicted to be 90%. In addition, the proposal unit 135Z proposes a high-functional nutritional food sold in the drug store 41Z to a male (customer ID = 002) predicted to have a nutritional deficiency degree of 93%. As another example, the proposal unit 135Z proposes a visit to the medical institution 43Z for a woman (customer ID = 003) whose prediction values of menopause and sleep disorder are 79% and 89%, respectively. In addition, the proposal unit 135Z proposes to go to the sports gym 45Z for a customer having a relatively high physical activity deficiency degree, and proposes a medical insurance product corresponding to a lifestyle disease provided by the insurance company 47Z for a customer having a physical activity insufficiency degree lower than a predetermined value.

As described above, the proposal unit 135Z of the integrated health prediction program 100Z obtains a prediction result from each of the plurality of prediction models 110Z from the purchase data 150Z, thereby being able to propose a food/supplies/service suitable for the customer. In order to realize such a proposal, a list of foods, supplies, and/or services associated with each health symptom that is an output value of the prediction model may be prepared in advance.

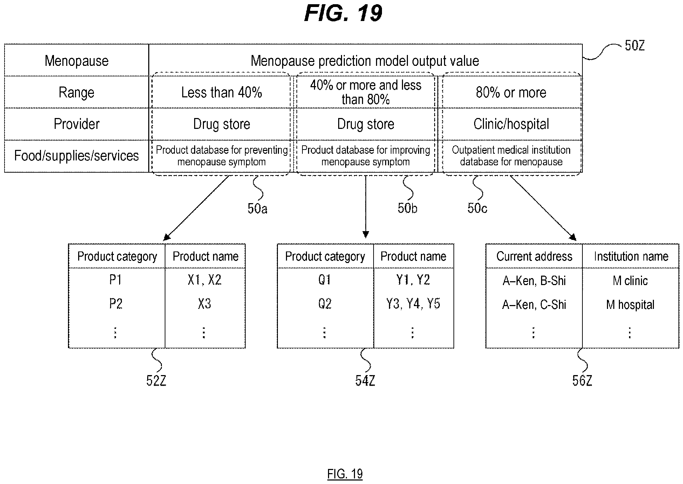

Fig. 19 illustrates a table 50Z for realizing proposals regarding menopause symptoms and databases 52Z, 54Z, and 56Z to be referred to.

In the table 50Z, three classes of a class 50a in which the output range of the menopause prediction model (the female health prediction model 110a in Fig. 16) is less than 40%, a class 50b in which the output range is 40% or more and less than 80%, and a class 50c in which the output range is 80% or more are provided. In the class 50a, a product database 52Z for preventing menopause symptoms in a drug store is referred to. In the product database 52Z, products X1 and X2 of a product category P1 and a product X3 of a product category P2 are described. The proposal unit 135Z can propose some or all of the categories and/or the product names to the corresponding customer with reference to the product database 52Z. In a case where the output value of the prediction model corresponds to the class 50b, the proposal unit 135Z refers to the product database 54Z for improving the menopause symptom and proposes the products Y1 and Y2 of the product category Q1. In a case where the output value of the prediction model corresponds to 50c, the proposal unit 135Z refers to a menopause outpatient medical institution database 56Z by using information of the current location and/or the residence of the customer registered in advance, and proposes a medical institution. That is, this corresponds to introduction to a customer of a medical institution that performs preventive medical care and/or treatment according to a health prediction result of the customer.

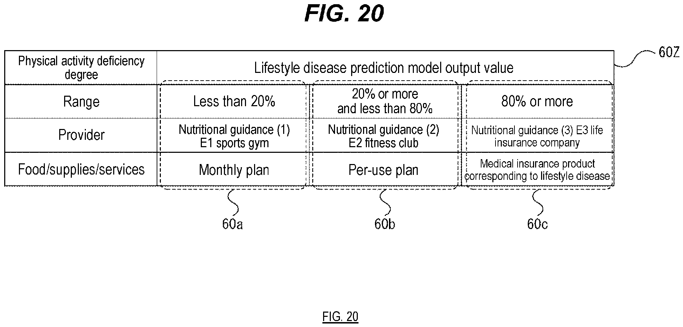

Another example will be described with reference to Fig. 20. Fig. 20 illustrates a table 60Z for realizing the proposal according to the output value of the lifestyle disease prediction model 110c.

In the table 60Z, three classes including a class 60a in which the output value of the lifestyle disease prediction model 110c, in other words, the range of physical activity insufficiency degree is less than 20%, a class 60b in which the range of the physical activity insufficiency degree is 20% or more and less than 80%, and a class 60c in which the range of the physical activity insufficiency degree is 80% or more are provided. In the class 60a, it is considered that the physical activity is satisfactory, and in order to continue the physical activity, nutritional guidance (1) and a monthly fee plan of an E1 sports gym are proposed. The nutritional guidance (1) is, for example, a proposal for a meal for taking nutritional components of less than 10 grams of lipid and 30 grams or more of protein per meal. In the class 60b, it is considered that the physical activity is not insufficient, and a subscription plan for an E2 fitness club is proposed in order to perform nutritional guidance (2) and the physical activity periodically. The nutritional guidance (2) is, for example, a proposal of a meal for taking nutritional components of less than 20 grams of lipid and 20 grams or more of protein per meal. In the class 60c, based on the fact that physical activity is insufficient and there is a possibility of suffering from a lifestyle disease in the future, nutritional guidance (3) and a medical insurance product proposed by an E3 life insurance company are proposed. The nutritional guidance (3) is, for example, a proposal of a meal for taking nutritional components of less than 20 grams of lipid, 20 grams or more of protein, and less than 20 grams of carbohydrate per meal. Proposal of nutritional guidance, sports gym, fitness club, and insurance products falls within the category of product or service provision. With respect to the output value of the lifestyle disease prediction model 110c, the proposal unit 135Z can make a proposal according to the output value to the customer by referring to the table 60Z.

In addition to the examples of Figs. 19 and 20, for each of the sleep state, the immune state, the moisture state, and the nutritional state, by providing a similar table and further a database as necessary, the proposal unit 135Z can make a proposal to the customer according to the health prediction value 160Z which is an output value of each prediction model 110Z for each customer.

Note that the integrated health prediction program 100Z itself does not necessarily include the proposal unit 135Z. For example, it is conceivable that the output unit 130Z of the integrated health prediction program 100Z transmits the health prediction value 160Z to a business person different from the business operator operating the integrated health prediction program 100Z under the permission of the customer, and the program of the other business person implements the program related to the proposal unit 135Z to make the above proposal to the customer. For example, the integrated health prediction program 100Z may transmit the health prediction value 160Z, which is an output value of the prediction model 110Z, to an insurance company so that the insurance company proposes an insurance product to the customer.

2.4 Flow of processing

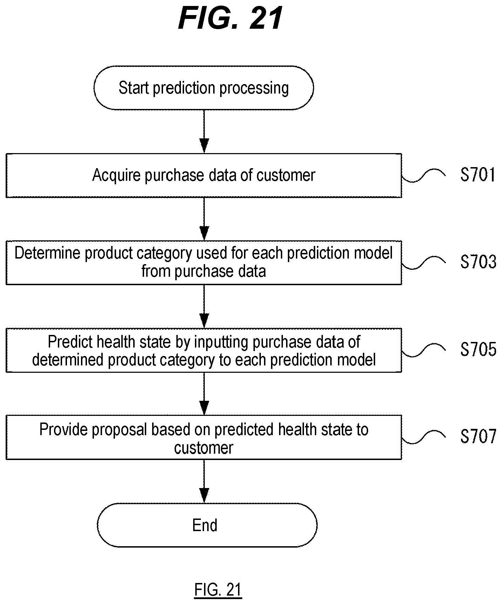

Hereinafter, a flow of processing of the integrated health prediction program 100Z will be described with reference to Fig. 21. Fig. 21 is a flowchart showing a procedure of health prediction processing for a customer executed by the integrated health prediction program 100Z.

The input unit 120Z of the integrated health prediction program 100Z acquires purchase data 150Z indicating a purchase history of a customer whose health state is desired to be predicted (S701). For example, when a product is purchased at a retail store, the input unit 120Z reads a membership card of the customer from a point of sale (POS) system, thereby constructing a database in which the customer and a purchase history are associated with each other. By extracting the purchase data 150Z from such a database with the acceptance of the customer, the input unit 120Z can input the purchase data 150Z to the integrated health prediction program 100Z. As described above, such purchase data 150Z includes, for example, the JICFS classification and the product category.

The product category determination unit 125Z determines the JICFS classification (classification code) and the product category as inputs of each prediction model 110Z in the purchase data 150Z (S703). Note that, as described above, when the prediction model 110Z does not particularly narrow down the product category to be used for prediction and uses the entire purchase data 150Z, the processing of S703 is unnecessary.

Next, each prediction model 110Z predicts the health state using the purchase data 150Z of the classification code and the product category determined by the product category determination unit 125Z (S705). The output unit 130Z outputs a health prediction value 160Z indicating the health state predicted by the prediction model 110Z, and the proposal unit 135Z provides a proposal based on the health prediction value 160Z to the customer (S707). Specifically, it is conceivable that the health prediction value 160Z or the proposal unit 135Z proposes health improvement to the customer in the form of printing on a receipt, display on a display, notification to a smartphone, or the like.

2.5 Hardware configuration

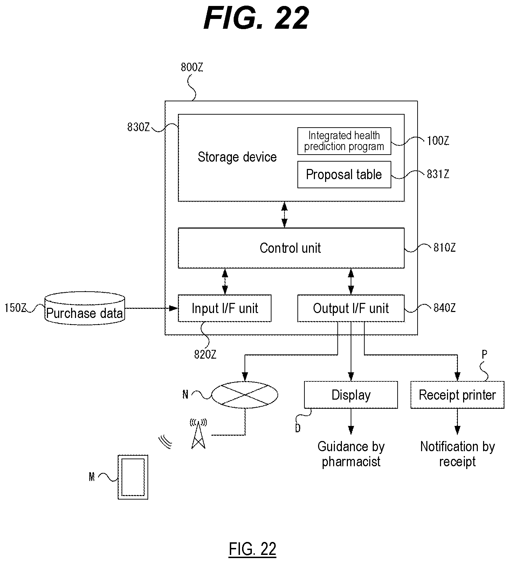

A specific example of a hardware configuration of the information processing device 800Z on which the integrated health prediction program 100Z is executed will be described with reference to Fig. 22. Fig. 22 is a hardware configuration diagram of the information processing device 800Z on which the integrated health prediction program 100Z is executed. The information processing device 800Z uses the integrated health prediction program 100Z to calculate a prediction result regarding the customer's menopause symptom, sleep state, or the like. The information processing device 800Z may be a generally available computer system, for example, a desktop personal computer (PC), a notebook PC, a tablet PC, a server computer, or the like. The computer system may be installed in a business place of a business operator who operates the health prediction system 1Z, or may be provided as a cloud service. In the latter case, the business place may be provided with a PC or the like that can communicate with the cloud service.

The information processing device 800Z includes a control unit 810Z, an input interface (I/F) unit 820Z, a storage device 830Z, and an output I/F unit 840Z.

The control unit 810Z may include a CPU (central processing unit, not illustrated), ROM (Read Only Memory, not illustrated), a RAM (random access memory), and the like. The control unit 810Z can execute the integrated health prediction program 100Z stored in the storage device 830Z. As a result, the information processing device 800Z can execute various processes related to the health prediction described above in addition to the function as a general computer. Note that an arithmetic circuit included in the control unit 810Z may not be a CPU, may be realized by various processors such as an MPU and a GPU, and may be realized by a plurality of processors instead of one processor.

The input I/F unit 820Z receives the purchase data 150Z regarding the customer whose health state is desired to be predicted. As described above, the input I/F unit 820Z can receive the purchase data 150Z from an information processing device such as an external server connected via a network such as the Internet or a LAN. The input I/F unit 820Z can be realized by, for example, a communication circuit that performs communication in accordance with a standard such as an Ethernet (registered trademark) communication terminal, a USB (registered trademark) terminal, IEEE802.11, 4G, or 5G.

The storage device 830Z is a storage medium that stores computer programs and data necessary for operating the information processing device 800Z. The storage device 830Z can be, for example, an HDD or an SSD that is a semiconductor storage device. The storage device 830Z may include a temporary storage element configured by a RAM such as a DRAM or an SRAM, for example, and may function as a work area of the control unit 810Z. The storage device 830Z stores the integrated health prediction program 100Z, a proposal table 831Z showing specific examples as the table 50Z in Fig. 19 or the table 60Z in Fig. 20.

Note that the prediction model 110Z may be incorporated as a part of the integrated health prediction program 100Z, or may be provided as data separate from the integrated health prediction program 100Z. Furthermore, the prediction model 110Z may be stored in the storage device 830Z as a table or a parameter group.

Furthermore, the proposal table 831Z can associate products/services to be proposed according to each degree with a prediction result indicating the degree of health state, for example, H layer/M layer/L layer or 90%/50%/20%.

The output I/F unit 840Z is a communication circuit and/or a communication terminal connected to various output devices provided outside the information processing device 800Z. For example, the output I/F unit 840Z can be a USB terminal that outputs print data indicating contents to be printed on a receipt printer P. The integrated health prediction program 100Z can calculate a prediction result related to the health state of the customer, transmit the result or a proposal according to the result to the customer to the receipt printer P, and cause the receipt printer P to print the proposal.

The output I/F unit 840Z may be a video output terminal connected to a display D. The integrated health prediction program 100Z calculates a prediction result regarding the health state of the customer, and causes the display D to display the result or a proposal to the customer according to the result. The pharmacist checks the contents displayed on the display D, for example, so that the pharmacist can propose one or a plurality of products according to the symptom of the customer.

As another form, the output I/F unit 840Z may be a communication terminal or a communication circuit that is connected to the communication network N or the like and can perform data communication. In a case where the output I/F unit 840Z is a communication terminal or a communication circuit, hardware of the input I/F unit 820Z and the output I/F unit 840Z may be the same. The output I/F unit 840Z can transmit a prediction result regarding the health state of the customer or a proposal according to the result to the customer to a smartphone M of the customer via, for example, a mobile phone line.

3 Learning system 9 for learning prediction model 110Z

3.1 Overview

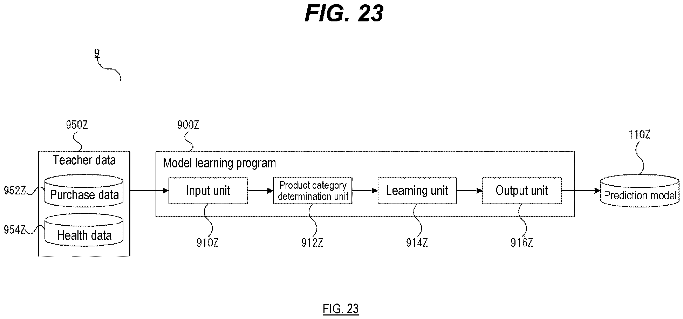

Next, a method for generating the prediction model 110Z included in the integrated health prediction program 100Z by machine learning will be described with reference to Fig. 23. Here, it is assumed that a model learning program 900Z generates the prediction model 110Z. Note that the model learning program 900Z may be implemented as a single program, or may be implemented as a plurality of programs that can operate in cooperation by input and output of data.

Note that the integrated health prediction program 100Z illustrated in Fig. 15 includes six types of prediction models 110Z, but these prediction models can be similarly generated only by a difference in the health data 954Z among the teacher data 950Z to be used. Hereinafter, a case of generating the menopause prediction model 110a regarding female health will be mainly described.

3.2 Teacher data 950Z

The model learning program 900Z performs machine learning using teacher data 950Z including various data collected from a large number of test subjects to construct a prediction model 110Z. The teacher data 950Z includes purchase data 952Z related to purchase behavior of each of the test subjects and health data 954Z which is data related to health of the test subjects.

The purchase data 952Z relates to information similar to that illustrated in the specific example with reference to Fig. 17. That is, in the present embodiment, for each ID of the test subject, the item 150a related to the category in the JICFS classification and the performance data 150b which is numerical data such as the purchase amount, the purchase quantity, and the purchase count during the period can be included in the purchase data 952Z. The purchase data 952Z preferably includes a purchase history of the food and/or beverage since the food and/or beverage is suitable as a product associated with the health state of the test subject and the customer.

The health data 954Z describes the health state of the test subject. The health state may be a state related to subjective symptoms or a state recognized as a result of measurement. That is, at least one of health consciousness, mental health, cognitive function, and a medical examination result can be used for the health data 954Z. In the health data 954Z in the present embodiment, a determination value for each state segment of the test subject is described as the health state of the test subject. The state segment is defined for each combination of the type and level (in other words, size) of the health state. The type and level of the health state in the present embodiment are the type and level of the mental and physical disorder possessed by the corresponding individual. The determination value can represent an index indicating a health state by two classes, three classes, or the like. Note that the "type and level of health state" referred to herein does not involve strict medical standards, and can be understood as the type and level of health concerns possessed by an individual.

Here, as an example, a case where the model learning program 900Z generates the menopause prediction model 110a and a case where the symptom of the simplified menopause index SMI is used to determine the level of menopause will be considered. In this case, the health data 954Z can include a label indicating a symptom of the simplified menopause index SMI to which the test subject belongs, in the health data 954Z. For example, the simplified menopause index SMI can be expressed as two classes. Specifically, "H" indicating that the menopause symptom is relatively large, and "other than H" are provided. It is also possible to subdivide (that is, symptoms are expressed as a total of three classes) "other than H" into "M" indicating that the symptom is relatively moderate and "L" indicating that the symptom is relatively small. Hereinafter, the test subject layer corresponding to "H" is referred to as "H layer".

In the labeling indicating the degree of symptom in the health data 954Z, not two or three classes but four or more classes may be further provided by subdivision, or a numerical value indicating the possibility of corresponding to the menopause symptom may be used instead of these classes.

That is, the teacher data 950Z is obtained by associating the purchase data 952Z including the information regarding each product category of each test subject and the information regarding the purchase amount, the purchase quantity, the purchase count, and the like during the predetermined period with the health data 954Z including at least the information of the label indicating the health state. Here, if the model learning program 900Z generates six prediction models 110Z of, for example, a female health state (menopause symptom), a sleep state (sleep disorder), a lifestyle disease (physical activity deficiency), a moisture state (moisture deficiency degree), an immune state (immune deficiency degree), and a nutritional state (nutritional deficiency degree), information corresponding to these prediction models 110Z may be included in the health data 954Z. In machine learning in the supervised learning, teacher data 950Z is roughly classified into an explanatory variable and an objective variable. In the present embodiment, the purchase data 952Z corresponds to an explanatory variable, and the health data 954Z corresponds to an objective variable. In the present embodiment, only the purchase data 952Z is used as the explanatory variable, but the present invention is not limited thereto. For example, it is conceivable to add sex and age, which are attributes of the test subject, to the explanatory variables.

Hereinafter, if the appropriate health data 954Z is prepared, the model learning program 900Z can generate the prediction model 110Z related to various symptoms such as a sleep state, a lifestyle disease, an immune state, a moisture state, a nutritional state, dementia, oxygen utilization, vascular health, hair health, an intestinal environment, promotion of an exercise effect, appetite promotion, eye health, skin health, oral health, cardiac health, depression, stress, headache, stiff shoulder, ear health, joint health, body temperature homeostasis, fatigue, bone health, male menopause disorder, hypertension, and weather pain. Hereinafter, in particular, indices that can be used as indices indicating a female health state (menopause symptom), a sleep state (sleep disorder), a lifestyle disease (physical activity deficiency), a moisture state (moisture deficiency degree), an immune state (immune deficiency degree), and a nutritional state (nutritional deficiency degree) will be exemplified.

3.2.1 Female health state (menopause symptom)

As an index indicating the degree of the menopause symptom, for example, as described above, the simplified menopause index SMI can be used. The simplified menopause index SMI is acquired by asking each test subject to prepare corresponding items according to the degrees of various listed symptoms in advance and scoring the results. The simplified menopause index SMI is considered to be an index reflecting symptoms peculiar to Japanese menopause women. Therefore, it is also conceivable to appropriately use, depending on the country, region, or the like, any one or more of other indices related to menopause symptoms, for example, the Menopause Rating Scale, The Kupperman index, a Greene Climacteric Scale, a PSST (The premenstrual symptoms screening tool), a MRS (Menopause rating Scale), a WHQ (The Women's Health Questionnaire), a VAS (Visual analogue scale), a HFRDI (Hot Flash Related Daily Interference Scale), a HFCS (Hot Flash Composite Score), and a MENQOL (Menopause-Specific Quality of life), and a menopause symptoms assessment table for Japanese women.

Regarding the female health state, in addition to the menopause symptom, a symptom of premenstrual syndrome (PMS, PMDD) is also considered. That is, in order to predict premenstrual syndrome, the health prediction model 110Z may be constructed by performing machine learning with the index indicating the degree of symptoms of premenstrual syndrome as the objective variable and the purchase data 952 as the explanatory variable.

In addition, the health data 954Z may include numerical data of these indices, or may include only a label indicating the degree of symptoms from the numerical values of these indices. In this regard, the same applies to the following indices such as the sleep state and the lifestyle disease.

3.2.2 Sleep state (sleep disorder)

It is conceivable that the index indicating sleep is an index indicating any of deterioration of a sleep state, sleep rhythm, and inappropriate sleep time. It may be a subjective symptom of each control acquired by a questionnaire or the like, or may be a measured value recognized as a result of measuring each control. As the sleep state, at least one of the Athens Sleep Rating Scale (AIS), the Pittsburgh Sleep Quality Index (PSQI), the 3 Dimensional Sleep Scale (3DSS), or the Insomnia Severity Index (ISI) can be used. The degree of symptoms is analyzed singly and in combination for each of the items, and the health state of the living person is classified into categories. The sleep rhythm can be grasped by using the Munich Chronotype Questionnaire ($\mu$MCTQ), determining the median time of sleep on each of weekdays and holidays, and calculating a social jet lag (SJL) from the difference.

3.2.3 Lifestyle disease (physical activity deficiency)

The index indicating the exercise state may include about one or more selected from lack of exercise, obesity, and a change in body weight in a certain period of time. More specifically, it is conceivable to use any one or more of the body weight, the body mass index (BMI), the number of steps, and/or the metabolic syndrome diagnostic reference value of each test subject. The metabolic syndrome diagnostic reference value is any one or more of a waist circumference diameter, a threshold determined for diagnosis of hyperglyceridemia and/or hypo-HDL cholesterolemia, a maximum value and/or a minimum value of blood pressure, and a threshold determined for diagnosis of fasting hyperglycemia.

Note that, at the time of machine learning for generating a prediction model regarding a lifestyle disease or an exercise state, machine learning may be performed by including, in addition to the purchase data 952Z, one or more pieces of information regarding whether the test subject smokes and the sitting position behavior of the test subject as explanatory variables.

3.2.4 Moisture state (moisture deficiency degree)

As an index indicating the moisture state, a serum Na value, a BUN/creatinine ratio, and/or a subjective symptom associated with urine osmotic pressure, urine specific gravity, urine color, dehydration evaluation scale, or moisture deficiency are considered. These pieces of information may be acquired by a method similar to the previous example of a menopause symptom. For example, a change in the body experienced by each of a plurality of controls for a certain period of time, for example, one year is obtained by a clinical test value obtained by a medical examination or a complete medical checkup or a health questionnaire conducted separately. On the other hand, the subjective symptom associated with the decrease in moisture can be acquired by a questionnaire or the like. The degree of symptom can be grasped from at least one or more of a serum Na value, urine osmotic pressure, urine specific gravity, a BUN/creatinine ratio, an amount of water consumed, an amount of physical activity, and a urine color.

Based on these indices, it is possible to find a state in which a deteriorated health state is experienced or a state in which the health state is likely to be experienced in the future in the literature from the epidemiological information. For example, it has been found that a living person having a serum Na value of more than 142 mEq/L has a higher risk of onset of cognitive impairment and hypertension than a living person having a serum Na value of less than 142 mEq/L, and it is possible to evaluate that the health risk is high.

Note that, at the time of machine learning for generating the prediction model regarding the moisture state, in addition to the purchase data 952Z, machine learning may be performed by including one or more pieces of information of the amount of water consumed, the amount of alcohol consumed, the amount of physical activity, the urine color, the urine osmotic pressure, the urine specific gravity, the serum Na value, and the BUN/creatinine ratio of the test subject as explanatory variables.

3.2.5 Immune state (immune deficiency degree)

As an index indicating the immune state, for example, susceptibility to a cold and/or its frequency, and cold-like symptoms can be used. The susceptibility or frequency of catching a cold may be subjective symptoms of each control acquired by a questionnaire or the like, or may be acquired from a medical visit history of a medical institution. The degrees of symptoms are tabulated in three or more categories, and the degrees of symptoms may be susceptibility to cold, SIgA concentration, an allergic symptom, an oral environment/state of immunity, an exercise state, a stress state, a severe sleep symptom, a moderate sleep symptom, a mild sleep symptom, and a cold-like symptom.

3.2.6 Nutritional state (nutritional deficiency degree)

The index indicating the nutritional state may be a subjective symptom of each control acquired by a questionnaire or the like, or may be a measured value recognized as a result of measuring each control. The degree of the symptom may be at least one of the presence or absence of subjective symptoms associated with nutritional deterioration, Diversity Score (DVS) of Food Intake, Simplified nutritional appetite questionnaire (SNAQ), and BMI (Body Mass Index).

3.3 Method of machine learning

The model learning program 900Z according to the present embodiment uses logistic regression as a method of machine learning. However, the logistic regression is an example, and it is also conceivable to adopt other methods, for example, random forest, decision tree, gradient boosting, support vector regression, linear regression, partial least squares (PLS) regression, Gaussian process regression, neural network, and the like. Also, when the logistic regression is used, the accuracy may be further improved by using an L1 normalization method, or the logistic regression (ridge regression) using an L2 normalization method may be adopted instead of the L1 normalization method. An elastic net adopting both the L1 normalization method and the L2 normalization method can also be used.

The logistic regression is a method for determining whether or not an input corresponds to a certain event by receiving the input and calculating a probability that the input corresponds to the certain event (or a probability that the input does not correspond to the certain event). As an example, the logistic regression model is described as the following Equation (1) called logit function. In Equation (1), the left side is a natural logarithm. Further, i indicates an i-th target person, p indicates the probability that the event of an objective variable will occur, $b_1$, $b_2$ ... $b_n$ indicates a partial regression coefficient, $b_0$ indicates a constant term, and $x_1$, $x_2$ ... $x_n$ indicates an explanatory variable.

[Equation 1]

$$\log \frac{p_i}{1 - p_i} = b_0 + b_1 x_1 + b_2 x_2 + \cdots + b_n x_n \quad \cdots (1)$$

When the right side of Equation (1) is z, Equation (1) can be transformed into Equation (2) representing the probability p.
[Equation 2]

$$p_i = \frac{1}{1 + e^{-z}} \quad \cdots (2)$$

Equation (2) is called a sigmoid function and is an activation function in the logistic regression model. The partial regression coefficients $b_1$, $b_2$ ... $b_n$ in the definition of z are called weights, and $b_0$ is a bias term. When z is input to the sigmoid function, a value of 0 to 1, that is, a probability value is calculated.

Here, the sigmoid function obtained by Equation (2) is placed as "$\varphi(z)$", and classified into an output y indicating one of binary values according to the output value. Equation (3) means that classification into a class of 1 is performed when $\varphi(z)$ is 0.5 or more, and classification into a class of 0 is performed when $\varphi(z)$ is less than 0.5. Note that 0.5 is an example, and a value other than 0.5 may be used as the threshold. In other words, Equation (3) means that classification into a class of 1 is performed when z in Equation (2) is 0 or more, and classification into a class of 0 is performed when z in Equation (2) is less than 0.
[Equation 3]

$$y = \begin{cases} 1 & (\varphi(z) \geq 0.5 \quad \leftrightarrow \quad z \geq 0) \\ 0 & (\varphi(z) < 0.5 \quad \leftrightarrow \quad z < 0) \end{cases} \quad \cdots (3)$$

Next, "likelihood" used for learning in the logistic regression is introduced. The likelihood means the likelihood of the condition seen from the result. A likelihood function L representing the likelihood is expressed by the following Equation (4). Note that P represents a probability value.
[Equation 4]

$$L(\omega) = \prod_{i=1}^{n} P\left(y^{(i)} \big| x^{(i)}; \omega\right) = \prod_{i=1}^{n} \left(\varphi\left(z^{(i)}\right)\right)^{y^{(i)}} \left(1 - \varphi\left(z^{(i)}\right)\right)^{1-y^{(i)}} \quad \cdots (4)$$

The likelihood function L indicates the probability of correctly determining all events. By obtaining the weight that maximizes the likelihood, the probability of the event desired to be predicted can be output more accurately. Specifically, the likelihood function L is multiplied by (-1) to reverse the positive and negative of the likelihood function. The likelihood function in which the positive and negative signs are reversed is an error function in the logistic regression. In order to obtain the weight at which the error function has the minimum value, that is, the partial regression coefficients $b_1$, $b_2$ ... $b_n$, a partial differential method and a gradient descent method are applied to each of the error functions $b_1$, $b_2$ ... $b_n$. As a result, learning of logistic regression is performed.

Note that a log likelihood function using the natural logarithm of Equation (4) may be used as the likelihood function. The optimum weight can be found by minimizing a function obtained by inverting positive and negative by multiplying the log likelihood function by (-1).

Arithmetic algorithms related to the logistic regression described above are well known, and software applications for performing machine learning by such algorithms are easily available. In principle, as described above, by using available software, even a person who does not know details of a specific analysis method using a mathematical expression can realize machine learning using the logistic regression according to the present embodiment.

3.4 Method for determining product category used for health prediction

Numerical data such as a purchase amount, a purchase quantity, and a purchase count regarding each product or service category in the purchase data 952Z included in the teacher data 950Z corresponds to the explanatory variable x in the above-described Equation (1) and the like.

As described above, in the health data 954Z included in the teacher data 950Z in the present embodiment, a label "H" or "L" (or "H", "M", or "L") indicating whether each symptom such as the menopause symptom or the sleep disorder is classified into the H layer or allocated to the L layer is allocated to each test subject. In the above Equation (3), the likelihood function can be obtained by performing classification such that "1" is output in the case of the H layer and "0" is output in the case other than the H layer. The element constituting the explanatory variable x is not limited to the purchase data 952Z described above. For example, it is also conceivable to include data indicating the age group and data indicating the gender as explanatory variables.

It is possible to discriminate a product group (category) which is often purchased by customers in the H layer by performing machine learning using the teacher data 950Z for a large number of test subjects and constructing a prediction model.

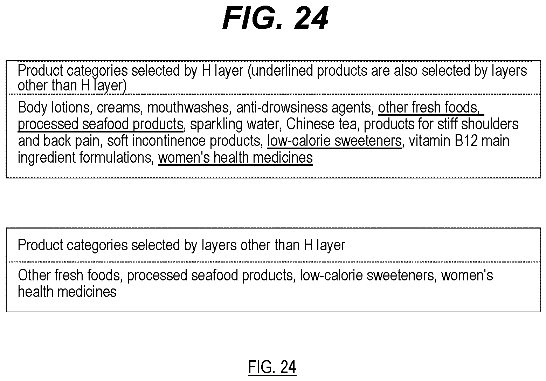

Fig. 24 illustrates product categories selected (frequently purchased) by many of test subjects in the H layer and layers other than the H layer regarding menopause symptoms. The upper part of Fig. 24 illustrates a product category selected by many test subjects in the H layer, and the lower part illustrates a product category selected by test subjects other than the H layer.

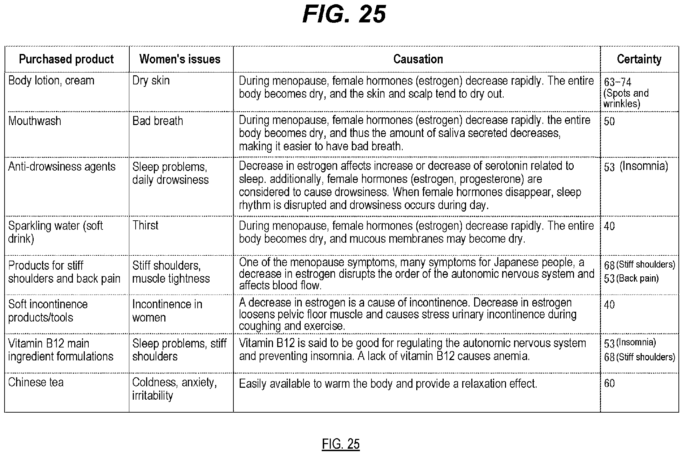

Fig. 25 illustrates a relationship between a menopause symptom found from a purchased item by a test subject in a H layer related to the menopause symptom and a health problem. The numerical value in the rightmost column of Fig. 25 indicates the degree (deviation value) indicating the ease of selection by female having menopause symptoms. This numerical value is cited from "deviation value by symptom for matters desired to be dealt with even if spending money - Research project report on understanding of female health and beauty lifestyle - Result of behavior observation survey".

From these results, it can be seen that the test subjects in the H layer regarding menopause symptoms actively select (purchase) a specific product. The test subjects in the H layer purchase products corresponding to dry skin or body and unidentified complaints.

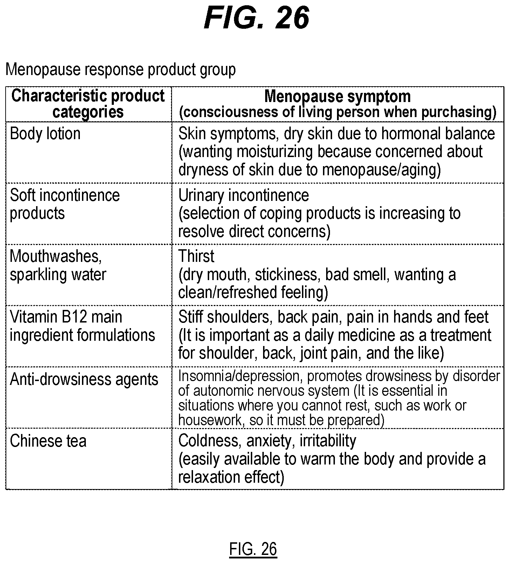

Fig. 26 illustrates an example of a product group (product category) for female having menopause symptoms belonging to the H layer. As described above, since it is possible to determine a product category of the product frequently purchased by the test subject in the H layer compared with the test subject in a layer other than the H layer, it is possible to estimate that the test subject who frequently purchases (quantity, amount, number of times) such a product is highly likely to belong to the H layer in the menopause symptom. Similarly, since it is also possible to determine a product category of the product frequently purchased by a test subject who does not belong to the H layer as compared with the test subject in the H layer, it can be estimated that a subject who frequently purchases from such a product category is highly likely not to belong to the H layer in the menopause symptom.

Therefore, the model learning program 900Z in the present embodiment constructs the prediction model 110Z using the data of the product category that can contribute to the estimation as to belonging to the H layer or not belonging to the H layer in the purchase data 952Z, so that the customer belonging to the H layer can be accurately estimated.

3.5 Configuration of model learning program 900Z

Hereinafter, a functional configuration of the model learning program 900Z will be described with reference to Fig. 23. Fig. 23 is a diagram illustrating a functional configuration of the model learning program 900Z. The model learning program 900Z includes an input unit 910Z, a product category determination unit 912Z, a learning unit 914Z, and an output unit 916Z. Note that the model learning program 900Z may be realized as a single program or may be realized as a plurality of programs that cooperate with each other.

The input unit 910Z receives the input of the teacher data 950Z. The input unit 910Z may read the teacher data 950Z from a storage medium such as an HDD or an SSD built in an information processing device (computer) in which the model learning program 900Z is executed, or may receive the input of the teacher data 950Z from an external information processing device or the like connected via a network such as a LAN or the Internet.

The product category determination unit 912Z selects a product category of the purchase data 952Z used for machine learning of the prediction model 110Z to be learned. Since the method for selecting the product category has been described in the above 3.4, the detailed description thereof will be omitted here. Note that, in a case of generating a plurality of prediction models 110Z respectively corresponding to a plurality of symptoms, the product category determination unit 912Z can select different product categories for the respective prediction models 110Z.

Note that, as described above, it is also conceivable to create the prediction model 110Z using the entire purchase data 952Z without narrowing down the product category at the time of machine learning of the prediction model 110Z. In this case, the product category determination unit 912Z is unnecessary.

The learning unit 914Z performs machine learning using the purchase data 952Z of the product category determined by the product category determination unit 912Z to generate the prediction model 110Z. Since the method for generating the prediction model 110Z has been described in the above 3.3, detailed description thereof is omitted here.

The output unit 916Z outputs the prediction model 110Z generated by the learning unit 914Z to a built-in storage medium, another information processing device (for example, the information processing device 800Z described with reference to Fig. 22) or the like connected via a network or the like. Note that, as an output method, it is also conceivable to output as a function itself constituting the prediction model 110Z, or it is also conceivable to output as a parameter of the function constituting the prediction model 110Z.

3.6 Flow of processing

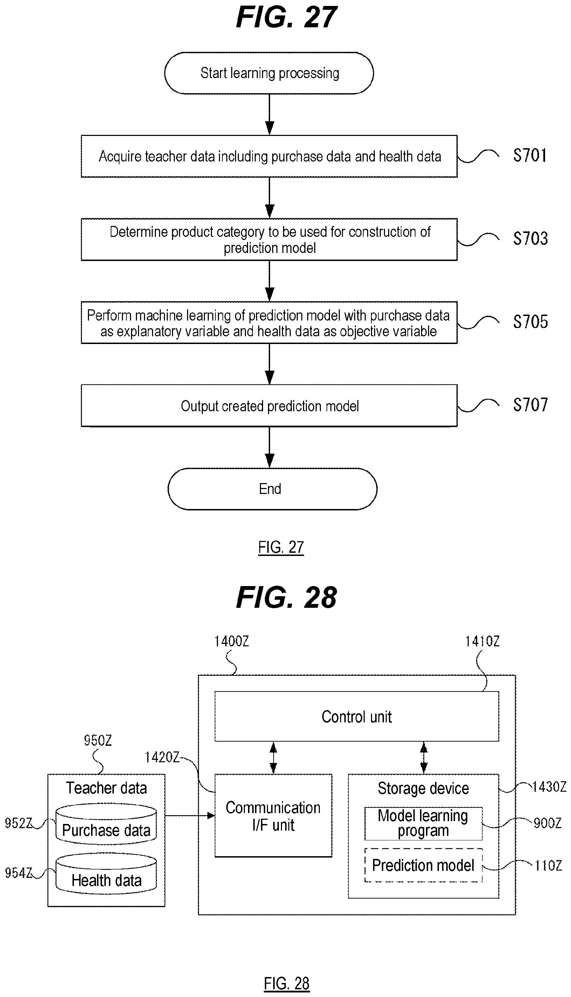

Hereinafter, a flow of processing of the model learning program 900Z will be described with reference to Fig. 27.

Fig. 27 is a flowchart illustrating a procedure for creating the prediction model 110Z, which is executed by the model learning program 900Z.

The input unit 910Z of the model learning program 900Z acquires the teacher data 950Z regarding a large number of test subjects used for learning (S1301). The teacher data 950Z includes purchase data 952Z indicating the purchase history of the test subject and health data 954Z indicating the health state (the degree of symptoms). As described above, the purchase data 952Z can include, for example, JICFS classification (classification code) and product categories, and numerical data such as the purchase count, the purchase amount, and the purchase quantity corresponding thereto.

Next, the product category determination unit 912Z determines the JICFS classification and the product category to be used for generation of the prediction model 110Z (S1303). The determination method is as described in the above 3.4, but the product category determination unit 912Z may select the JICFS classification and the product category that have a large influence on the estimation of the health state obtained as the health data 954Z. When the prediction model 110Z is created using the entire purchase data 952Z without particularly narrowing down the product category, the process of S1303 is unnecessary.

The learning unit 914Z performs machine learning with the purchase data 952Z as an explanatory variable and the health data 954Z as an objective function (S1305). Since the method of machine learning has been described with a specific example in the above 3.3, the description thereof will be omitted here.

The output unit 916Z outputs the prediction model 110Z generated by the learning unit 914Z in S1305 to a built-in storage medium, another information processing device connected via a network, or the like. Note that the prediction model 110Z may be output as a function itself constituting the prediction model 110Z or may be output in the form of a parameter or the like as described above.

3.7 Hardware configuration

A specific example of a hardware configuration of the information processing device 1400Z on which the model learning program 900Z is executed will be described with reference to Fig. 28. Fig. 28 is a hardware configuration diagram of the information processing device 1400Z on which the model learning program 900Z is executed. The information processing device 1400Z uses the model learning program 900Z to generate the prediction model 110Z that performs prediction regarding the menopause symptom, the sleep state, or the like of the customer.

The information processing device 1400Z is similar to the information processing device 800Z described above in that the information processing device can be realized as a generally available computer system, that the information processing device may be installed at a business place of a business operator who operates the health prediction system 1Z or may be provided as a cloud service, and the like. Note that, in the present embodiment, a case where the information processing device 800Z that performs health prediction and the information processing device 1400Z that generates the prediction model 110Z are different devices will be mainly described, but both may be realized as the same device.

The information processing device 1400Z includes a control unit 1410Z, a communication I/F unit 1420Z, and a storage device 1430Z.

The control unit 1410Z can include a CPU, a ROM, a RAM, and the like. The control unit 1410Z can execute the model learning program 900Z stored in the storage device 1430Z. As a result, the information processing device 1400Z can execute various processes related to the construction of the prediction model 110Z described above in addition to the function as a general computer. Note that the arithmetic circuit included in the control unit 1410Z may not be a CPU, and may be realized by various processors such as an MPU and a GPU. Furthermore, the arithmetic circuit may be realized by a plurality of processors instead of one processor.

The communication I/F unit 1420Z (the input I/F unit 820Z) can be realized by, for example, a communication circuit that performs communication in accordance with a standard such as an Ethernet (registered trademark) communication terminal, a USB (registered trademark) terminal, IEEE802.11, 4G, or 5G. The communication I/F unit 1420Z is connectable to a communication network such as an intranet and the Internet, and receives the teacher data 950Z prepared by the business operator operating the learning system 9. Furthermore, the information processing device 1400Z may directly communicate with another device via the communication I/F unit 1420Z, or may communicate via an access point or the like.

The storage device 1430Z is a storage medium that stores computer programs and data necessary for operating the information processing device 1400Z. The storage device 1430Z can be, for example, an HDD or an SSD. The storage device 1430Z may include a temporary storage element configured by a RAM such as a DRAM or an SRAM, for example, and may function as a work area of the control unit 1410Z. The storage device 1430Z stores the model learning program 900Z and the prediction model 110Z (including a table and/or a parameter group defining the prediction model 110Z) generated by the model learning program 900Z.

4 Effects of the present embodiment

In the health prediction system 1Z according to the present embodiment, the integrated health prediction program 100Z can predict a plurality of health risks of a customer from purchase data 150Z of the customer. Based on the individual prediction result, a proposal for improving and/or maintaining the current or future health state can be made finely for each customer.

5. Appendix

Note that the configurations of the above-described embodiments may be combined or some components may be interchanged. Further, the configuration of the present invention is not limited to the above-described embodiment, and various modifications may be made without departing from the gist of the present invention.

For example, in the above-described embodiment, the integrated health prediction program 100Z includes six types of health prediction models, but including six types is merely an example. Two or more types, less than six types, or seven or more types of health prediction models may be included. Generally described, the integrated health prediction program 100Z may include prediction models 110Z related to respective health symptoms of the first type to the N-th type (N is an integer of 2 or more). The health prediction model 110Z related to the k-th type ($1 \leq k \leq N$) of health symptom may be constructed as a prediction model in which the purchase data 150Z related to the product category to which at least one product belongs is input and the degree of the k-th type of health symptom is output. The health prediction model 110Z related to k-th type ($1 \leq k \leq N$) of health symptoms can be constructed by machine learning with the purchase data 952Z related to the purchase history of each of a plurality of test subjects as an explanatory variable and the health data 954Z indicating the degree of health state collected from each of the test subjects as an objective variable.

In addition, when the degree of health symptoms is represented by a numerical value, the numerical values of the upper limit and the lower limit of the numerical range defining the class are an example, and can be appropriately changed. The number of defined classes may also be optionally determined by a person skilled in the art.

As will be readily understood by a person skilled in the art having the benefit of this disclosure, the various examples described above may be used in appropriate combination in various patterns relative to one another as long as no contradiction arises.

In view of the many possible embodiments to which the principles of the invention disclosed herein may be applied, it is to be understood that the various illustrated embodiments are only various preferred examples and should not be considered as limiting the scope of the invention according to the claims to these various preferred examples. Actually, the technical scope of the invention according to the claims is defined by the appended claims. Accordingly, a patent is claimed as the invention of the present inventors for all the inventions belonging to the technical scope of the invention described in the claims.

The present application is based on and claims the benefit of priority from the following Japanese patent applications. The entire contents of these Japanese patent applications are incorporated herein by reference.

(1) Japanese Patent Application No. 2022-046894 filed on March 23, 2022 with a title "COMPUTER PROGRAM, INFORMATION PROCESSING DEVICE, AND METHOD"

(2) Japanese Patent Application No. 2022-046895 filed on March 23, 2022 with a title "PROGRAM, INFORMATION PROCESSING DEVICE, AND INFORMATION PROCESSING METHOD"

Reference Signs List

1 Communication system
2 Communication network (communication line)
10, 10A, 10B Server device
20, 20A, 20B Terminal device
11, 21 Central processing unit (CPU)

**Claims**

**1.** A computer program causing, by being executed at least one processor, the at least one processor to function to:

acquire target behavior data for identifying a history of behavior executed by a target user; and

output, from an estimation model generated by executing supervised learning, state data for identifying a hydration state of the target user, the state data including at least one of data for identifying a serum Na value of the target user, data for identifying a urine osmotic pressure of the target user, data for identifying urine specific gravity of the target user, data for identifying a urine color of the target user, data for identifying a BUN (urea nitrogen)/creatinine ratio of the target user, data for identifying a dehydration evaluation scale of the target user, and data for identifying a subjective symptom associated with moisture deficiency of the target user, by inputting the target behavior data to the estimation model.

2. A computer program causing, by being executed at least one processor, the at least one processor to function to:

acquire target behavior data for identifying a history of behavior executed by a target user; and
output, from an estimation model generated by executing supervised learning, state data for identifying an immune state of the target user, the state data including at least one of data for identifying susceptibility of the target user to cold, data for identifying a frequency of causing cold of the target user, data for identifying SIgA concentration of the target user, data for identifying allergic symptoms of the target user, data for identifying oral environment and/or a state of immunity of the target user, data for identifying a sleep state of the target user, data for identifying an exercise state of the target user, and data for identifying a stress state of the target user, by inputting the target behavior data to the estimation model.

3. A computer program causing, by being executed at least one processor, the at least one processor to function to:

acquire target behavior data for identifying a history of behavior executed by a target user; and
output, from an estimation model generated by executing supervised learning, state data for identifying a nutritional state of the target user, the state data including at least one of data for identifying the presence or absence of subjective symptoms associated with nutritional deficiencies of the target user, data for identifying a food intake diversity score (DVS) of the target user, data for identifying a Simplified Nutritional Appetite Questionnaire of the target user, and data for identifying BMI (Body Mass Index) of the target user, by inputting the target behavior data to the estimation model.

# *FIG. 1*

1

Server
device

10 (10A)

Server
device

10 (10B)

Communication
network

2

20 (20A)

Terminal
device

20 (20B)

Terminal
device

FIG. 1

# *FIG. 2*

10 (20)

15 (25)

Auxiliary
storage device

Communication
network

2

Input/output interface device

13 (23)

Input
device

14 (24)

Main storage
device

12 (22)

Output
device

16 (26)

Central
processing unit

11 (21)

FIG. 2

# FIG. 3

Start

1000

Information processing device acquires teacher data

1002

Information processing device generates estimation model by using teacher data

1004

Information processing device acquires target behavior data for identifying history of behavior executed by target user

1006

Information processing device acquires target sleep state data for identifying sleep state of target user by using target behavior data and estimation model

1008

Information processing device determines target product/target service corresponding to target sleep state data

1010

Information processing device provides proposal data for identifying target product/target service

End

FIG. 3

# *FIG. 4*

## Athens insomnia scale (AIS) self-assessment of insomnia

Please select that you have experienced at least 3 times a week in the past month.

| | | | |
|---|---|---|---|
| 1 | Does it take you a while to get into bed and actually sleep? | 0 | Sleeping is easier than usual |
| | | 1 | It took a little longer than usual |
| | | 2 | It took a lot longer than usual |
| | | 3 | It took a lot more time than usual, or couldn't sleep at all |
| 2 | Did you wake up during sleep at night? | 0 | It wasn't a problem |
| | | 1 | There are times like that a little bit |
| | | 2 | There are quite often |
| | | 3 | Serious condition, or couldn't sleep at all |
| 3 | Have you ever woken up earlier than you wanted to wake up and been unable to fall asleep since? | 0 | Never been like that |
| | | 1 | Woke up a little early |
| | | 2 | Woke up pretty early |
| | | 3 | Woke up much earlier, or couldn't sleep at all |
| 4 | Do you have enough sleep, including sleeping at night and taking naps? | 0 | Enough |
| | | 1 | A little lacking |
| | | 2 | Quite lacking |
| | | 3 | Not enough, or couldn't sleep at all |
| 5 | How do you feel about your sleep quality overall? | 0 | Satisfied |
| | | 1 | A little dissatisfied |
| | | 2 | Quite dissatisfied |
| | | 3 | Very dissatisfied, or couldn't sleep at all |
| 6 | How is your day feeling? | 0 | Always the same |
| | | 1 | A little depressed |
| | | 2 | Pretty depressed |
| | | 3 | Quite depressed |
| 7 | How is your physical and mental activity throughout the day? | 0 | Always the same |
| | | 1 | A little deteriorated |
| | | 2 | Pretty deteriorated |
| | | 3 | Quite deteriorated |
| 8 | Are there times during the day when you feel sleepy? | 0 | Not at all |
| | | 1 | Sometimes |
| | | 2 | Often |
| | | 3 | Very often |
| | Total | | 1 to 3 points: sleep well<br>4 to 5 points: slightly suspect insomnia<br>6 points or more: high likelihood of insomnia |

FIG. 4

# FIG. 5A

You will be asked about your usual sleeping habits over the past month. Please answer all of the following questions as accurately as possible, considering the days and nights of most days in the past month.

**Q.1**
**In the past month, what time did you usually go to bed?**
Bedtime
(1.AM 2.PM) About __:__

**Q.2**
**In the past month, how long did it take you to fall asleep in bed?**
About minutes

**Q.3**
**What time did you usually wake up in the past month?**
Waking time
(1.AM 2.PM) About __:__

**Q.4**
**How many hours did you actually sleep during the past month? This may be different from the amount of time you spend in bed.**
Sleeping time
Daily average:
About __ hours __ minutes

**During the past month, how often have you had trouble sleeping for the following reasons?**
**Circle the one that suits you best.**

**Q.5a**
**Never fell asleep within 30 minutes of getting into bed**
0. None
1. Less than once in a week
2. Once or twice in a week
3. Three times or more in a week

**Q.5b**
**Woke up during the night or early in the morning**
0. None
1. Less than once in a week
2. Once or twice in a week
3. Three times or more in a week

**Q.5c**
**Woke up go to the bathroom**
0. None
1. Less than once in a week
2. Once or twice in a week
3. Three times or more in a week

**Q.5d**
**Difficulty breathing**
0. None
1. Less than once in a week
2. Once or twice in a week
3. Three times or more in a week

**Q.5e**
**Coughing or snoring a lot**
0. None
1. Less than once in a week
2. Once or twice in a week
3. Three times or more in a week

**Q.5f**
**Felt very cold**
0. None
1. Less than once in a week
2. Once or twice in a week
3. Three times or more in a week

FIG. 5A

# FIG. 5B

**Q.5g**
**Felt very hot**
   0. None
   1. Less than once in a week
   2. Once or twice in a week
   3. Three times or more in a week

**Q.5h**
**Nightmare dream**
   0. None
   1. Less than once in a week
   2. Once or twice in a week
   3. Three times or more in a week

**Q.5i**
**Felt pain**
   0. None
   1. Less than once in a week
   2. Once or twice in a week
   3. Three times or more in a week

**Q.5j**
   **If there is a reason other than the above, please write in the space below.**

**Reason**
**For this reason, how often have you had trouble sleeping in the past month?**
   0. None
   1. Less than once in a week
   2. Once or twice in a week
   3. Three times or more in a week

**Q.6**
**How would you rate your sleep quality overall over the past month?**
   1: Very good
   2: Pretty good
   3: Pretty bad
   4: Very bad

**Q.7**
**During the past month, how often have you taken medication to help you sleep (Medication prescribed by a doctor or medication purchased from a pharmacy)?**
   0. None
   1. Less than once in a week
   2. Once or twice in a week
   3. Three times or more in a week

**Q.8**
**In the past month, how often have you had trouble staying awake in situations where you should not fall asleep, such as while driving, eating, or engaging in social activities?**
   0. None
   1. Less than once in a week
   2. Once or twice in a week
   3. Three times or more in a week

**Q.9**
**In the past month, to what extent have you had trouble maintaining the motivation needed to perform certain tasks?**
   0: No problem
   1: Very little problem
   2: A little problem
   3: Serious problem

Total score

FIG. 5B

# FIG. 5C

Nippon Rinsho Vol 66, Suppl 2, 2008

Table 2 Pittsburgh sleep quality index

Quality of sleep (C1)
Q.6 Subjective evaluation of sleep quality over the past month
Quite good,    0 points
Pretty good,    1 point
Pretty bad,    2 point
Quite bad,    3 point     Score of C1   points
Time to sleep (C2)
(1) Q.2 Time taken from going to bed to falling asleep in the past month
Less than 16 minutes,    0 points
More than 16 minutes but less than 31 minutes,    1 point
More than 31 minutes and less than 61 minutes,    2 points
Exceeded 61 minutes,    3 points
Score of C2(1)   points
(2) Q.5A For the past month, you have had difficulty sleeping because you were
unable to fall asleep within 30 minutes after getting to bed.
None,    0 points
Less than once a week,    1 point
One or twice a week,    2 points
More than 3 times a week,    3 points
Score of C2(2)   points
Score of C2(1) + C2 (2)   points
Score of C2(1) + C2 (2)
0 points,    0 points
1 to 2 points,    1 point
3 to 4 points,    2 points
5 to 6 points,    3 points
Score of C2   points
Sleep time (C3)
Q.4 Actual sleep time in the past month
Exceeded 7 hours,    0 points
More than 6 hours but equal to or less than 7 hours,   1 point
Equal to or more than 5 hours but equal to
or less than 6 hours,    2 points
Less than 5 hours,    3 points
Score of C3   points
Sleep efficiency (C4)
(1) Q.4 In the past month, actual sleep time   hour
(2) Q.3 In the past month, time in bed (bed time - wake up time)   hour
(3) Calculate sleep efficiency (%), actual sleep time (1)/time in bed (2) x 100(%)
85% or more,    0 points
75% or more but less than 85%,    1 point
65% or more but less than 75%,    2 points
Less than 65%,    3 points
Score of C4   points

Difficulty sleeping (C5)
The reasons for difficulty sleeping for one month (Q. 5b to 5j) are scored as follows:
None,    0 points
Less than once a week,    1 point
One or twice a week,    2 points
More than 3 times a week,    3 points
Score of Q.5b    points
Score of Q.5c    points
Score of Q.5d    points
Score of Q.5e    points
Score of Q.5f    points
Score of Q.5g    points
Score of Q.5h    points
Score of Q.5i    points
Score of Q.5j    points
Total score from Q. 5b to 5j   points
Total score from Q. 5b to 5j
0 points,    0 points
1 to 9 points,    1 point
10 to 18 points,    2 points
19 to 27 points,    3 points    Score of C5   points
Use of sleeping pills (C6)
Q.7 Frequency of use of sleeping pills in the past month
None,    0 points
Less than once a week,    1 point
One or twice a week,    2 points
More than 3 times a week,    3 points    Score of C6   points
Difficulty in waking up during the day (C7)
(1) Q.8: Excessive daytime sleepiness in the past month
None,    0 points
Less than once a week,    1 point
One or twice a week,    2 points
More than 3 times a week,    3 points    Score of C7(1)   points
(2) Q. 9 Sustained motivation in the past month
No problems at all,    0 points
Only a few problems,    1 point
Some problems,    2 points
Very big problems,    3 points    Score of C7(2)   points
Score of C7(1)+C7(2)   points
Score of C7(1)+C7(2)
0 points,    0 points
1 to 2 points,    1 point
3 to 4 points,    2 points
5 to 6 points,    3 points    Score OF C7   points

Pittsburgh sleep quality index overall score (PSQIG): 0 to 21 points
Total points from C1 to C7 (C1 + C2 + C3 + C4 + C5 + C6 + C7)
PSQIG point

(Yuriko DOI: Sleep Disorders Treatment Guidelines, P.228-229, 2002.)

FIG. 5C

# FIG. 6A

**Sleep type diagnosis by 3DSS (three-dimensional sleep scale) check sheet**

For the following questions, please select the answer that best applies within the past month. Please answer about your average daily life without thinking about special cases. Please click on the appropriate answer

FIG. 6A

# FIG. 6B

### 3DSS check sheet

3DSS (Three Dimensional Sleep Scale) is a scale that grasps an individual's sleep habits from three aspects: "phase (rhythm)", "quality", and "quantity". In today's 24-hour society, since irregular sleep and nocturnal sleep patterns have become problems, it is necessary to evaluate problems related to phase (rhythm). The unique feature of 3DSS is that it scores these three aspects simultaneously and individually, making it possible to immediately determine which area has a problem.

**For the following questions, please select the answer that best applies within the past month. Please answer about your average daily life without thinking about special cases. Please circle the number that applies to you.**

| | Very applicable | Somewhat applicable | Not very applicable | Not applicable at all | Phase | Quality | Quantity |
|---|---|---|---|---|---|---|---|
| Weekday sleep time is less than 6 hours | | | | | | | |
| Actually, you want to sleep more, but you don't sleep as much as you want to | | | | | | | |
| Bedtime rarely changes regardless of weekdays and holidays | | | | | | | |
| Wake-up time rarely changes regardless of weekdays and holidays | | | | | | | |
| Eat a proper breakfast every day | | | | | | | |
| When lie down to sleep, cannot fall asleep for more than 30 minutes | | | | | | | |
| Wake up more than twice in the late night | | | | | | | |
| If wake-up more than 2 hours earlier than scheduled wake-up time, cannot fall asleep after that time | | | | | | | |
| Can't feel like got a good night's sleep | | | | | | | |
| Feel anxious because you can't sleep | | | | | | | |
| Immediately after waking up, strong drowsiness and fatigue remain | | | | | | | |
| Feel sleepy not only during the day but also in the morning or evening | | | | | | | |
| Take a nap or a short nap | | | | | | | |
| In terms of "morning type" and "evening type," you are "morning type" | | | | | | | |

Wake up time on weekdays is...

[3] → Around 6 A.M. or earlier    [2] → Around 6:30 A.M. than 6 A.M.

[1] → Around 7 A.M.           [0] → Later than 7 A.M.

Phase

Just add up the numbers you circled. → **Total score**

**Please circle the total score.** ↓

| | Warning | Caution | Good |
|---|---|---|---|
| Phase | | | |
| Quality | | | |
| Quantity | | | |

Quantity       Quality

**Explanations and advices are on the back**

FIG. 6B

EP 4 498 376 A1

# *FIG. 7*

1. Please rate the current (i.e., last 2 weeks) **SEVERITY** of your insomnia problem(s).

| | None | Mild | Moderate | Severe | Very |
|---|---|---|---|---|---|
| Difficulty falling asleep: | 0 | 1 | 2 | 3 | 4 |
| Difficulty staying asleep: | 0 | 1 | 2 | 3 | 4 |
| Problem waking up too early: | 0 | 1 | 2 | 3 | 4 |

2. How **SATISFIED**/dissatisfied are you with your current sleep pattern?

| Very Satisfied | | | | Very Dissatisfied |
|---|---|---|---|---|
| 0 | 1 | 2 | 3 | 4 |

3. To what extent do you consider your sleep problem to **INTERFERE** with your daily functioning (e.g. daytime fatigue, ability to function at work/daily chores, concentration, memory, mood, etc.).

| Not at all Interfering | A Little | Somewhat | Much | Very Much Interfering |
|---|---|---|---|---|
| 0 | 1 | 2 | 3 | 4 |

4. How **NOTICEABLE** to others do you think your sleeping problem is in terms of impairing the quality of your life?

| Not at all Noticeable | Barely | Somewhat | Much | Very Much Noticeable |
|---|---|---|---|---|
| 0 | 1 | 2 | 3 | 4 |

5. How **WORRIED**/distressed are you about your current sleep problem?

| Not at all | A Little | Somewhat | Much | Very Much |
|---|---|---|---|---|
| 0 | 1 | 2 | 3 | 4 |

**Guidelines for Scoring/Interpretation:**

Add scores for all seven items (1a+1b+1c+ 2+3+4+5)   = _____
Total score ranges from 0-28
  0-7      = No clinically significant insomnia
  8-14     = Subthreshold insomnia
  15-21    = Clinical insomnia (moderate severity)
  22-28    = Clinical insomnia (severe)

FIG. 7

59

# FIG. 8A

Munich ChronoType Questionnaire (MCTQ)

I work (go to school) regularly. This includes, for example, students or housewives.
Yes ☐       I work for (1 ☐, 2 ☐, 3 ☐, 4 ☐, 5 ☐, 6 ☐, 7 ☐) days a week
No ☐

If your answer is "yes, for 7 days" or "no", compare the normal "days with work" and "days without work" to see if your sleep schedule will not change anyway. Think about it. On "work (school) days," your behavior, especially your sleep schedule, is greatly influenced by social constraints rather than your internal desires. Please fill out the MCTQ questionnaire with this in mind.

**Please use 24-hour units (23:00, not 11:00 PM)**

## Work (school) days

Fig. 1: I go to bed at ____.
Fig. 2: Please be careful as some people may stay up for a while after going to bed.
Fig. 3: I actually get ready to get to sleep (lights out) at ____.
Fig. 4: I need __ minutes until I sleep.
Fig. 5: I wake up at ____.
Fig. 6: I get up in __ minutes after waking up.
I use an alarm clock on days when I have work (school) to do.      Yes ☐ No ☐
The only person who answered "yes": I usually wake up before the alarm clock rings.
Yes ☐ No ☐

## A day without work (school)

Fig. 1: I go to bed at ____.
Fig. 2: Please be careful as some people may stay up for a while after going to bed.
Fig. 3: I actually get ready to get to sleep (lights out) at ____.
Fig. 4: I need __ minutes until I sleep.
Fig. 5: I wake up at ____.
Fig. 6: I get up in __ minutes after waking up.
The reason I wake up (fig. 5) is because I use the alarm clock.      Yes ☐ No ☐
There are certain reasons why you **can't choose** your own sleep schedule on days when you don't have work.
Yes ☐      (If you answered "yes": Children/pets ☐ Hobby ☐ Etc. ☐ Example: _____)
No ☐

FIG. 8A

# *FIG. 8B*

**Time spent outdoors**

On average, I spend the following time outdoors (without a roof over my head) per day.
Day with work (school) __ hours __ minutes
Day without work (school) __ hours __ minutes

**Regarding work (school)**

For the past 3 months I worked as a shift worker.
No ☐        Yes ☐

My work (school) schedule usually
Starts at __:__
Ends at __:__

My work (school) schedule is
    Very flexible ☐
    Quite flexible ☐
    Not flexible ☐
    Not flexible at all ☐

When traveling to work (school)
    I am in a closed vehicle (for example, car, bus, train, subway) ☐
    I am not in a closed vehicle (for example, walking, bicycle) ☐
    Work at home ☐

When **going to** work (school), I need __ hour(s) __ minutes to commute (to school).
When **coming back from** work (school), I need __ hour(s) __ minutes to commute (to school).

**Stimulant**

Please pay attention to the following points:

Please answer some questions with the amount "per day," and other questions with the amount "per week" or "per month."

Please write down the **approximate or average amount**

|  | Per day/week/month |
|---|---|
| I smoke __ cigarettes. | Per ☐ ☐ ☐ |
| I drink __ bottles of beer. | Per ☐ ☐ ☐ |
| I drink __ glasses of wine. | Per ☐ ☐ ☐ |
| I drink __ glasses of distilled alcohol/whiskey/gin. | Per ☐ ☐ ☐ |
| I drink __ cup(s) of coffee. | Per ☐ ☐ ☐ |
| I drink __ cup(s) of black tea. | Per ☐ ☐ ☐ |
| I drink __ cup(s) of caffeinated beverage (soft drink). | Per ☐ ☐ ☐ |
| I take sleeping pills __. | Per ☐ ☐ ☐ |

FIG. 8B

# *FIG. 9*

|  |  | Score identified by sample property data | Score identified by sample property data |
|---|---|---|---|
|  |  | THRESHOLD A1 (6 points) or more | Less than threshold A1 (6 points) |
| Value identified by sample rhythm data | Threshold B1 (1 hour) or more | Sleep category 10 | Sleep category 20 |
| Value identified by sample rhythm data | Less than threshold B1 (1 hour) | Sleep category 11 | Sleep category 21 |

FIG. 9

# FIG. 10

|  |  | Score identified by sample property data | Score identified by sample property data | Score identified by sample property data |
|---|---|---|---|---|
|  |  | Threshold A2 (8 POINTS) or more | Threshold A1 (6 POINTS) OR more, less than threshold A2 (8 Points) | Less than threshold A1 (6 points) |
| Value identified by sample rhythm data | Threshold B2 (3 hours) or more | Sleep category 300 | Sleep category 100 | Sleep category 200 |
| Value identified by sample rhythm data | Threshold B1 (1 hour) or more, less than threshold B2 (3 HOURS) | Sleep category 301 | Sleep category 101 | Sleep category 201 |
| Value identified by sample rhythm data | Less than threshold B1 (1 HOUR) | Sleep category 302 | Sleep category 102 | Sleep category 202 |

FIG. 10

# FIG. 11

| Target sleep state data | Target product/target service | | |
|---|---|---|---|
| Target property data | Class V1 Product P1 | Class V2 Product P2, product P3 | Class V3 Product P4, service S1 |
| Target rhythm data | Class W1 Product P5, product P6 | Class W2 Product P7, service S2 | Class W3 Product P8, service S1 |
| Target time data | Class X1 Product P2 | Class X2 Service S3, service S4 | Class X3 Product P9, Product P10 |
| Target sleep category data | Class Y1 Service S5 | Class Y2 Product P11, service S6 | Class Y3 Service S7, Service S8 |

FIG. 11

# FIG. 12

Start

1100
Information processing device acquires teacher data

1102
Information processing device generates estimation model by using teacher data

1104
Information processing device acquires target behavior data for identifying history of behavior executed by target user

1106
Information processing device acquires target menopause state data for identifying menopause symptom state of target user by using target behavior data and estimation model

1108
Information processing device determines target product/target service corresponding to target menopause state data

1110
Information processing device provides proposal data for identifying target product/ target service

End

FIG. 12

# FIG. 13

Depending on the degree of the symptom, circle it, enter a score, and check based on the total score. If any of the symptoms is strong, please circle it.

| Symptoms | Strong | Medium | Weak | None | Score |
|---|---|---|---|---|---|
| (1) Face is hot | 10 | 6 | 3 | 0 | |
| (2) Easily sweat | 10 | 6 | 3 | 0 | |
| (3) Waist and hands and feet are likely to cool | 14 | 9 | 5 | 0 | |
| (4) Breathlessness, palpitations | 12 | 8 | 4 | 0 | |
| (5) Trouble falling asleep or having light sleep | 14 | 9 | 5 | 0 | |
| (6) Easily angered, easily annoyed | 12 | 8 | 4 | 0 | |
| (7) Sometimes worry or feel depressed | 7 | 5 | 3 | 0 | |
| (8) Frequent headaches, dizziness, and nausea | 7 | 5 | 3 | 0 | |
| (9) Easily tired | 7 | 4 | 2 | 0 | |
| (10) Stiff shoulders, back pain, pain in hands and feet | 7 | 5 | 3 | 0 | |
| **Total score** | | | | | |

Menopausal self-scoring assessment method

| | |
|---|---|
| 0 to 25 points: | you are going through menopause well. Continue your current lifestyle |
| 26 to 50 points: | Pay attention to your diet and exercise, and do not overdo your lifestyle. |
| 51 to 65 points: | Recommended to see a doctor and receive life guidance, counseling, and drug treatment. |
| 66 to 80 points: | Long-term (more than half a year) planned treatment is required. |
| 81 to 100 points: | It is necessary to undergo detailed examination by each department, and in case of menopausal disorders only, long-term response from a specialist is required. |

FIG. 13

# FIG. 14

| Target menopause state data | Target product/target service | | |
|---|---|---|---|
| Target comprehensive state data | Class V1 | Class V2 | Class V3 |
| | Product P10 | Product P20, product P30 | Product P40, service S10 |
| Target first state data | Class W1 | Class W2 | Class W3 |
| | Product P50, product P60 | Product P70, service S20 | Product P80, service S10 |
| Target second state data | Class X1 | Class X2 | Class X3 |
| | Product P20 | Service S30, service S40 | Product P90, Product P100 |

FIG. 14

## FIG. 15

| | Health prediction value of customer | | | |
|---|---|---|---|---|
| ID | Menopause | Sleep disorder | | Nutritional deficiency degree |
| 001 | **90%** | 21% | ··· | 11% |
| 002 | 10% | 42% | ··· | **93%** |
| 003 | **79%** | **89%** | ··· | 20% |
| ⋮ | ⋮ | ⋮ | ⋮ | ⋮ |
| xxx | 41% | **78%** | ··· | 45% |

FIG.15

## FIG. 16

FIG. 16

# FIG. 17

150Z

| User ID 002 | JICFS classification | | | | Classification (Product category) | Purchase amount | Purchase quantity | Purchase count |
|---|---|---|---|---|---|---|---|---|
| | Large | Medium | Small | Very small | | | | |

| User ID 001 | JICFS classification | | | | Classification (Product category) | Purchase amount | Purchase quantity | Purchase count | |
|---|---|---|---|---|---|---|---|---|---|
| | Large | Medium | Small | Very small | | | | | |
| | ⋮ | ⋮ | ⋮ | ⋮ | ⋮ | ⋮ | ⋮ | ⋮ | |
| | 1 | 4 | 03 | | Soft drink | ¥*,*** | * | * Times | |
| | ⋮ | ⋮ | ⋮ | ⋮ | ⋮ | ⋮ | ⋮ | ⋮ | |
| | 2 | 1 | 21 | 05 | Mouthwash | ¥*,*** | * | * Times | |
| | 1 | 4 | 01 | 13 | Chinese tea | ¥*,*** | * | * Times | |
| | ⋮ | ⋮ | ⋮ | ⋮ | ⋮ | ⋮ | ⋮ | ⋮ | |
| | 2 | 2 | 11 | 05 | Body lotion | ¥*,*** | * | * Times | |
| | ⋮ | ⋮ | ⋮ | ⋮ | ⋮ | ⋮ | ⋮ | ⋮ | |

150a            150b

FIG. 17

# FIG. 18

160Z

**Health prediction value of customer**

| ID | Menopause | Sleep disorder | | Nutritional deficiency degree |
|---|---|---|---|---|
| 001 | 90% | 21% | ⋯ | 11% |
| 002 | 10% | 42% | ⋯ | 93% |
| 003 | 79% | 89% | ⋯ | 20% |
| ⋮ | ⋮ | ⋮ | ⋮ | ⋮ |
| xxx | ⋮ | ⋮ | ⋮ | ⋮ |

STORE ~41Z

~43Z

GYM ~45Z

~47Z

FIG. 18

## FIG. 19

| Menopause | Menopause prediction model output value | | |
|---|---|---|---|
| Range | Less than 40% | 40% or more and less than 80% | 80% or more |
| Provider | Drug store | Drug store | Clinic/hospital |
| Food/supplies/services | Product database for preventing menopause symptom | Product database for improving menopause symptom | Outpatient medical institution database for menopause |

50Z

50a     50b     50c

| Product category | Product name |
|---|---|
| P1 | X1, X2 |
| P2 | X3 |
| ⋮ | ⋮ |

52Z

| Product category | Product name |
|---|---|
| Q1 | Y1, Y2 |
| Q2 | Y3, Y4, Y5 |
| ⋮ | ⋮ |

54Z

| Current address | Institution name |
|---|---|
| A–Ken, B-Shi | M clinic |
| A–Ken, C-Shi | M hospital |
| ⋮ | ⋮ |

56Z

FIG. 19

## FIG. 20

| Physical activity deficiency degree | Lifestyle disease prediction model output value | | |
|---|---|---|---|
| Range | Less than 20% | 20% or more and less than 80% | 80% or more |
| Provider | Nutritional guidance (1) E1 sports gym | Nutritional guidance (2) E2 fitness club | Nutritional guidance (3) E3 life insurance company |
| Food/supplies/services | Monthly plan | Per-use plan | Medical insurance product corresponding to lifestyle disease |

60Z

60a     60b     60c

FIG. 20

# FIG. 21

```
┌─────────────────────────────┐
│   Start prediction processing  │
└─────────────────────────────┘
              │
              ▼
┌──────────────────────────────────────────┐
│      Acquire purchase data of customer       │  ∿  S701
└──────────────────────────────────────────┘
              │
              ▼
┌──────────────────────────────────────────┐
│ Determine product category used for each prediction model │  ∿  S703
│              from purchase data              │
└──────────────────────────────────────────┘
              │
              ▼
┌──────────────────────────────────────────┐
│    Predict health state by inputting purchase data of    │  ∿  S705
│  determined product category to each prediction model   │
└──────────────────────────────────────────┘
              │
              ▼
┌──────────────────────────────────────────┐
│   Provide proposal based on predicted health state to    │  ∿  S707
│                    customer                    │
└──────────────────────────────────────────┘
              │
              ▼
┌─────────────────────────────┐
│             End              │
└─────────────────────────────┘
```

FIG. 21

# FIG. 22

FIG. 22

# FIG. 23

9

FIG. 23

# FIG. 24

| Product categories selected by H layer (underlined products are also selected by layers other than H layer) |
| --- |
| Body lotions, creams, mouthwashes, anti-drowsiness agents, <u>other fresh foods, processed seafood products</u>, sparkling water, Chinese tea, products for stiff shoulders and back pain, soft incontinence products, <u>low-calorie sweeteners</u>, vitamin B12 main ingredient formulations, <u>women's health medicines</u> |

| Product categories selected by layers other than H layer |
| --- |
| Other fresh foods, processed seafood products, low-calorie sweeteners, women's health medicines |

FIG. 24

## FIG. 25

| Purchased product | Women's issues | Causation | Certainty |
|---|---|---|---|
| Body lotion, cream | Dry skin | During menopause, female hormones (estrogen) decrease rapidly. The entire body becomes dry, and the skin and scalp tend to dry out. | 63-74 (Spots and wrinkles) |
| Mouthwash | Bad breath | During menopause, female hormones (estrogen) decrease rapidly. the entire body becomes dry, and thus the amount of saliva secreted decreases, making it easier to have bad breath. | 50 |
| Anti-drowsiness agents | Sleep problems, daily drowsiness | Decrease in estrogen affects increase or decrease of serotonin related to sleep. additionally, female hormones (estrogen, progesterone) are considered to cause drowsiness. When female hormones disappear, sleep rhythm is disrupted and drowsiness occurs during day. | 53 (Insomnia) |
| Sparkling water (soft drink) | Thirst | During menopause, female hormones (estrogen) decrease rapidly. The entire body becomes dry, and mucous membranes may become dry. | 40 |
| Products for stiff shoulders and back pain | Stiff shoulders, muscle tightness | One of the menopause symptoms, many symptoms for Japanese people, a decrease in estrogen disrupts the order of the autonomic nervous system and affects blood flow. | 68 (Stiff shoulders) 53 (Back pain) |
| Soft incontinence products/tools | Incontinence in women | A decrease in estrogen is a cause of incontinence. Decrease in estrogen loosens pelvic floor muscle and causes stress urinary incontinence during coughing and exercise. | 40 |
| Vitamin B12 main ingredient formulations | Sleep problems, stiff shoulders | Vitamin B12 is said to be good for regulating the autonomic nervous system and preventing insomnia. A lack of vitamin B12 causes anemia. | 53 (Insomnia) 68 (Stiff shoulders) |
| Chinese tea | Coldness, anxiety, irritability | Easily available to warm the body and provide a relaxation effect. | 60 |

FIG. 25

# FIG. 26

Menopause response product group

| Characteristic product categories | Menopause symptom (consciousness of living person when purchasing) |
|---|---|
| Body lotion | Skin symptoms, dry skin due to hormonal balance (wanting moisturizing because concerned about dryness of skin due to menopause/aging) |
| Soft incontinence products | Urinary incontinence (selection of coping products is increasing to resolve direct concerns) |
| Mouthwashes, sparkling water | Thirst (dry mouth, stickiness, bad smell, wanting a clean/refreshed feeling) |
| Vitamin B12 main ingredient formulations | Stiff shoulders, back pain, pain in hands and feet (It is important as a daily medicine as a treatment for shoulder, back, joint pain, and the like) |
| Anti-drowsiness agents | Insomnia/depression, promotes drowsiness by disorder of autonomic nervous system (It is essential in situations where you cannot rest, such as work or housework, so it must be prepared) |
| Chinese tea | Coldness, anxiety, irritability (easily available to warm the body and provide a relaxation effect) |

FIG. 26

# FIG. 27

Start learning processing

Acquire teacher data including purchase data and health data — S701

Determine product category to be used for construction of prediction model — S703

Perform machine learning of prediction model with purchase data as explanatory variable and health data as objective variable — S705

Output created prediction model — S707

End

FIG. 27

# FIG. 28

1400Z    1410Z

Control unit

Storage device — 1430Z

Model learning program — 900Z

Prediction model — 110Z

950Z
1420Z

Teacher data

952Z — Purchase data

954Z — Health data

Communication I/F unit

FIG. 28

## INTERNATIONAL SEARCH REPORT

| International application No. |
| --- |
| **PCT/JP2023/010383** |

**A.    CLASSIFICATION OF SUBJECT MATTER**

*G16H 10/00*(2018.01)i; *G16H 50/30*(2018.01)i
FI:    G16H50/30; G16H10/00

According to International Patent Classification (IPC) or to both national classification and IPC

**B.    FIELDS SEARCHED**

Minimum documentation searched (classification system followed by classification symbols)

G16H10/00; G16H50/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2022-42721 A (TOKYU CONSTRUCTION CO., LTD.) 15 March 2022 (2022-03-15) paragraphs [0015]-[0017], [0032], [0036]-[0040], [0044]-[0069] | 1 |
| A | JP 2021-135971 A (SUWA UNIV. OF SCIENCE) 13 September 2021 (2021-09-13) paragraphs [0056]-[0060] | 1 |
| A | JP 2018-116584 A (SUYAMA, Shunsuke) 26 July 2018 (2018-07-26) paragraphs [0025]-[0032] | 1 |
| X | JP 6949277 B1 (MITSUBISHI ELECTRIC CORP.) 13 October 2021 (2021-10-13) paragraphs [0022], [0023], [0028], [0031] | 2 |
| X | JP 2020-201851 A (AMI CORP.) 17 December 2020 (2020-12-17) paragraphs [0049]-[0055], [0123], [0124], [0131]-[0139] | 3 |
| A | WO 2021/044696 A1 (NEC SOLUTION INNOVATORS, LTD.) 11 March 2021 (2021-03-11) entire text, all drawings | 3 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **09 May 2023** | **16 May 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2023/010383**

| Box No. III    Observations where unity of invention is lacking (Continuation of item 3 of first sheet) |
|---|

This International Searching Authority found multiple inventions in this international application, as follows:

Document 1: JP 2022-42721 A (TOKYU CONSTRUCTION CO., LTD.) 15 March 2022 (2022-03-15),
paragraphs [0015]-[0017], [0032], [0036]-[0040], [0044]-[0069]
(Family: none)

The claims are classified into the three inventions below.

(Invention 1) Claim 1
Document 1 (in particular, refer to paragraphs [0015]-[0017], [0032], [0036]-[0040], and [0044]-[0069])
describes an inference processing unit 6 of a dehydrated state management system 1, wherein the dehydrated state
of a subject H1 to be managed is inferred on the basis of blood flow rate data, the amount of water supply, and an
environment state by using an inference model constructed on the basis of a database constructed by acquiring, as
data on the amount of water supply to the subject H1 to be managed, the number of detections of information of
water supply by a water supply device 4. In light of document 1, claim 1 lacks novelty, and thus does not have a
special technical feature. Accordingly claim 1 is classified as invention 1.

(Invention 2) Claim 2
Claim 2 cannot be said to have the same or corresponding special technical features between this claim and claim
1 classified as invention 1.
Furthermore, claim 2 does not depend from claim 1. In addition, claim 2 is not substantially identical to or
similarly closely related to the claim classified as invention 1.
Accordingly claim 2 cannot be identified as invention 1.
Meanwhile, claim 2 has the special technical feature of "causing the inference model to output state data that
identifies the immune state of the subject user, and that includes at least one of data for identifying how susceptible
the subject user is to colds, data for identifying how frequently the subject user has a cold, data for identifying
the SIgA concentration of the subject user, data for identifying allergic symptoms of the subject user, data for
identifying the oral environment and/or the immune strength state of the subject user, data for identifying the state
of sleep of the subject user, data for identifying the state of exercise of the subject user, and data for identifying the
state of stress of the subject user"; thus this claim is classified as invention 2.

(Invention 3) Claims 3
Claim 3 cannot be said to have the same or corresponding special technical features between this claim and claim
1 classified as invention 1 or claim 2 classified as invention 2.
Furthermore, claim 3 does not depend from claim 1 or 2. In addition, claim 3 is not substantially identical to or
similarly closely related to either of the claims classified as invention 1 or 2.
Accordingly claim 3 cannot be identified as either of inventions 1 and 2.
Meanwhile, claim 3 has the special technical feature of "causing the inference model to output state data that
identifies the nutritional state of the subject user, and that includes at least one of data for identifying whether the
subject user has subjective symptoms associated with low nutrition, data for identifying a diversity score (DVS) on
food intake of the subject user, data for identifying the simplified nutritional appetite questionnaire (SNAQ) of the
subject user, and data for identifying the body mass index (BMI) of the subject user"; thus this claim is classified as
invention 3.

Form PCT/ISA/210 (continuation of first sheet) (January 2015)

**INTERNATIONAL SEARCH REPORT**

International application No.

**PCT/JP2023/010383**

| Box No. III | Observations where unity of invention is lacking (Continuation of item 3 of first sheet) |
|---|---|

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☑ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2023/010383**

| Patent document cited in search report | | | Publication date (day/month/year) | Patent family member(s) | | | Publication date (day/month/year) |
|---|---|---|---|---|---|---|---|
| JP | 2022-42721 | A | 15 March 2022 | (Family: none) | | | |
| JP | 2021-135971 | A | 13 September 2021 | (Family: none) | | | |
| JP | 2018-116584 | A | 26 July 2018 | (Family: none) | | | |
| JP | 6949277 | B1 | 13 October 2021 | WO 2022/153469 A1 paragraphs [0022], [0023], [0028], [0031] | | | |
| JP | 2020-201851 | A | 17 December 2020 | JP 6671609 B1 | | | |
| WO | 2021/044696 | A1 | 11 March 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6916367 B
- JP 2022046894 A
- JP 2022046895 A